# EUROPÄISCHE PATENTSCHRIFT

(11) **EP 1 918 379 B1**
(45) Veröffentlichungstag und Bekanntmachung des Hinweises auf die Patenterteilung: **26.10.2011**
(21) Anmeldenummer: 06022970.5
(22) Anmeldetag: 03.11.2006
(51) Int. Cl.: C12N 15/81

(54) **Expressionsvektoren zur multiplen Gen-Integration und Überexpression von homologen und heterologen Proteinen in Hefen der Gattung Arxula**
Expression vectors for multiple gene integration and Overexpression of homologous and heterologous proteins in yeasts of the species Arxula
Vecteurs d'expression pour l'intégration multiple de gène et l'expression des protéines homologues et hétérologues en levures de l'espèce Arxula

(43) Veröffentlichungstag der Anmeldung: 07.05.2008
(73) Patentinhaber: Leibniz-Institut für Pflanzengenetik und Kulturpflanzenforschung (IPK), 06466 Gatersleben (DE)
(72) Erfinder: Kunze, Gotthard, Prof. Dr., 06466 Gatersleben (DE); Steinborn, Gerhard, Dr., 06466 Gatersleben (DE)
(74) Vertreter: Grünecker, Kinkeldey, Stockmair & Schwanhäusser

(56) Entgegenhaltungen:
- WO-A-01/85925
- DE-A1- 10 060 133
- TERENTIEV YAROSLAV ET AL: "A wide-range integrative yeast expression vector system based on Arxula adeninivorans-derived elements" JOURNAL OF INDUSTRIAL MICROBIOLOGY & BIOTECHNOLOGY, Bd. 31, Nr. 5, Juni 2004 (2004-06), Seiten 223-228, XP002428711 ISSN: 1367-5435
- WARTMANN T ET AL: "The constitutive AHSB4 promoter: A novel component of the Arxula adeninivorans-based expression platform." APPLIED MICROBIOLOGY AND BIOTECHNOLOGY, Bd. 62, Nr. 5-6, Oktober 2003 (2003-10), Seiten 528-535, XP002428712 ISSN: 0175-7598
- WARTMANN THOMAS ET AL: "The ALEU2 gene: A new component for an Arxula adeninivorans-based expression platform." FEMS YEAST RESEARCH, Bd. 3, Nr. 2, März 2003 (2003-03), Seiten 223-232, XP002428713 ISSN: 1567-1356
- WARTMANN THOMAS ET AL: "High-level production and secretion of recombinant proteins by the dimorphic yeast Arxula adeninivorans" FEMS YEAST RESEARCH, Bd. 2, Nr. 3, August 2002 (2002-08), Seiten 363-369, XP002428714 ISSN: 1567-1356

## Beschreibung

### Gebiet der Erfindung

Die vorliegende Erfindung betrifft Expressionsvektoren, die in Hefen der Gattung *Arxula,* insbesondere in der Art *Arxula adeninivorans,* zur multiplen, chromosomalen Gen-Integration und dadurch bedingt zu einer hohen Produktion von homologen und vorzugsweise heterologen Proteinen genutzt werden können. Zudem betrifft die Erfindung Verfahren zur Expression von Proteinen, Verfahren zur Herstellung von Wirtszellen der Gattung *Arxula,* sowie Kits, Wirtszellen und pharmazeutische Zusammensetzungen.

### Hintergrund der Erfindung

Die Anwendung rekombinanter Hefezellen und -stämme einschließlich derer der Gattung *Arxula* zur Produktion von heterologen Proteinen ist allgemein bekannt. Bislang wurden bei der Hefe *Saccharomyces cerevisiae* episomale (YEp), d. h. extrachromosomal replizierende Plasmide als Expressionsvektoren genutzt, die bis zu 200 Kopien pro haploides Genom aufweisen können. Bei Langzeitkultivierungen erfordern diese jedoch die Anwendung eines Selektionsdruckes zur Plasmidstabilisierung. Allerdings kann dieser Selektionsdruck bei der industriellen Fermentation aus ökonomischen Gründen und wegen Bedenken zur biologischen Sicherheit nicht durch Anwendung von Antibiotika vermittelt werden. Deshalb werden zunehmend, besonders bei sogenannten nicht-konventionellen Hefen, Integrationsplasmide (Ylp) als Expressionsvektoren genutzt, die durch homologe Rekombination in das Hefegenom integriert und mit dem Hefegenom mitotisch stabil repliziert werden. Für ein hinreichend starkes Expressionsniveau ist es von Vorteil, wenn mehrere Kopien des für das heterologe Protein kodierenden Gens integriert werden, die dispers verteilt oder als direktes DNA-Repeat vorliegen. Eine solche multiple genomische Integration mit ökonomisch bedeutender Steigerung der Gen-Expression konnte für *Saccharomyces cerevisiae* und *Aspergillus oryzae* mit Hilfe des rDNA-Integrationssystems erreicht werden. Bei diesem Verfahren wird primär eine Gen-Kopie gerichtet in die chromosomale rDNA integriert und nachfolgend das integrierte Gen aufgrund der Clusterstruktur der rDNA von bis zu 200 direkten Repeats amplifiziert. Die Gen-Amplifikation mit dem Ergebnis von multiplen Integranten erfolgt also sekundär bei nachfolgender Kultivierung der nach Transformation erhaltenen primären, singulären Integranten unter Selektionsdruck durch ein Antibiotikum oder bei industriellen Anwendungen unter Nutzung eines sehr gering exprimierten Auxotrophie-Selektionsmarkers. Dazu wird die Promotoraktivität des Auxotrophie-Selektionsmarkers auf ein geeignetes Niveau reduziert, so dass ein hinreichend starker Selektionsdruck erzeugt wird, der eine Anreicherung und Isolierung von Integranten ermöglichen soll, die eine multiple Integration des Expressionsvektors aufweisen. Zur Einstellung des geeigneten Selektionsdrucks werden die Promotoren in unterschiedlichem Ausmaß deletiert; teilweise muss sogar zusätzlich das Struktur-Gen des Selektionsmarkers mutiert werden. Nachteilig hat sich bei den bislang bekannten Hefe-basierten Expressionssystemen und den darauf basierenden Protein-Expressionsverfahren erwiesen, dass die multiple Integration der Expressionsvektoren in das Genom experimentell sehr aufwändig und nicht reproduzierbar ist.

Es ist allgemein bekannt, dass die Wrts-Vektor-Systeme generell nicht gattungsübergreifend eingesetzt werden können, so dass etwa ein in der Gattung *Saccharomyces* erfolgreich verwendbares Expressionsvektorsystem nicht einfach in einer anderen Gattung, wie etwa den Hefen der Gattung *Arxula,* eingesetzt werden kann.

Lediglich das auf *A. adeninivorans* Elementen basierende sog. "wide range system" kann auch in andere Hefearten transformiert werden. Voraussetzung hierfür ist jedoch, das ein starker konstitutiver Promotor für die Expression der entsprechenden Expressionskassette genutzt wird, dass die als Wirt genutzte Hefe den allgemeinen genetischen Code benutzt und dass der verwendete Selektionsmarker (z.B. *hph*-Gene - verursacht Hygromycin B Resistenz; *ALEU2*-Gen) überhaupt in der entsprechenden Hefe selektierbar ist. Nachteil dieses System ist, dass in den Integranten, speziell in den nicht-*A*. *adeninivorans* Integranten geringe Expressionsspiegel erreicht werden, da die Promotoren im heterologen System niemals volle Aktivitäten erreichen und ihre Regulation verändert ist. Dieses System ist deshalb nur für Testzwecke einsetzbar (Detektion von artspezifischen posttranskriptionalen bzw. post-translationalen Modifikationen an den rekombinanten Proteinen), um den für das jeweilige heterologe Gen optimalen Wirt zu ermitteln. Das entsprechende heterologe Gen muss dann mit wirtseigenen Expressionsplasmiden in die entsprechende Hefe transformiert und dort hoch exprimiert werden. Aus diesem Grund bedarf es zur Entwicklung von *Arxula*-kompatiblen Expressionsvektoren und darauf basierenden Verfahren zur Herstellung von Proteinen in Hefen der Gattung *Arxula* aufwendiger Forschungsarbeiten.

Die Hefe *A. adeninivorans* ist eine nicht-konventionelle, nicht-pathogene, dimorphe, haploide Hefe die auf Grund ihrer außergewöhnlichen Wachstumseigenschaften (Schnellwüchsigkeit, sehr hohe Zelldichten, sehr umfangreiches Substratspektrum, Osmo- und Thermoresistenz) für biotechnologische Anwendungen besonders gut geeignet ist. Zur Transformation und Integration in *A. adeninivorans* liegen bisher folgende Ergebnisse vor.

Mit einer modifizierten Transformationsmethode (Dohmen, R.J.et al. 1991, Yeast 7, 691-692) lässt sich diese Hefe mit zirkulären Plasmiden transformieren. Bei Einsatz dieses Verfahrens integrieren die Plasmide jedoch reproduzierbar in nur einer Kopie in das Genom der Hefe. Außerdem ist die Konzentrationen der hergestellten heterologen Proteine sehr gering und die Integranten sind mitotisch instabil (DD-Patentschrift 298 821 A5, Kunze, G. & Kunze, I. 1996: Arxula adeninivorans. In: Nonconventional yeasts in biotechnology (ed. Wolf, K.) Springer Verlag, Berlin-Heidelberg-New York, pp. 389-409).

Ferner ist ein sog. integratives Transformationsverfahren für die Herstellung von mitotisch stabilen Hefe-Integranten beschrieben worden. Hierbei wurden Plasmide genutzt, die Teile der 25S rDNA enthalten und die sich nach Linearisierung durch homologe Rekombination in die 25S rDNA integrieren lassen. Als Selektionsmarker wurden das aus *E. coli* stammende *hph*-Gen (kodiert Resistenz gegenüber Hygromycin B) genutzt, wobei die Kassette den *TEF1*-Promotor (*A. adeninivorans),* das *hph*-Gen sowie den PH05-Terminator (*S. cerevisiae)* enthielt. Als Alternative zu dem *hph*-Gen wurde die aus *Arxula* stammenden Gene *ALEU2, ATRP1* und *AlLV1* zur Komplementation entsprechender Auxotrophien genutzt.

Der Nachteil dieser Methoden ist, dass die Plasmide auf Grund ihres hohen Molekulargewichtes nur sehr schwer zu handhaben sind und reproduzierbar nur in einer Kopie pro Kopie des Genoms in die chromosomale DNA integriert werden. Das dabei erreichte quantitative Niveau der heterologen Protein-Expression ist außerdem gering.

Zusammenfassend ist es bis dato daher nicht möglich, in Hefen der Gattung *Arxula* eine multiple genomische und vorzugsweise mitotisch stabile Integration von Expressionsvektoren zu bewerkstelligen, um so ein hohes quantitatives Niveau der Produktion heterologer Proteine zu ermöglichen.

Es ist eine Aufgabe der vorliegenden Erfindung, Expressionsvektoren, Wirtszellen, Kits und Verfahren zur Expression heterologer Proteine bereitzustellen, die die Nachteile im Stand der Technik überwinden.

### Beschreibung der Erfindung

Die Aufgabe wird durch Bereitstellen der in den Ansprüchen definierten Gegenstände gelöst.

In einem ersten Aspekt bezieht sich die Erfindung auf einen Expressionsvektor zur multiplen genomischen Integration in eine Wirtszelle der Hefegattung Arxula, umfassend:
a) eine rDNA Sequenz AA, deren Sequenz mindestens 80% Identität mit einer genomischen ribosomalen DNA Sequenz A der Wirtszelle der Hefegattung Arxula aufweist;
b) eine Protein-Expressionskassette, enthaltend einen ersten konstitutiven oder induzierbaren Promotor an oder hinter dessen 3'-Ende eine für ein heterologes Protein kodierende Nukleinsäure insertiert werden kann, so dass die insertierte Nukleinsäure unter der Transkriptionskontrolle des Promotors steht;
c) eine Selektionsmarker-Expressionskassette, enthaltend einen zweiten konstitutiven oder induzierbaren Promotor sowie ein Selektionsmarkergen, das unter der Transkriptionskontrolle des zweiten Promotors steht;
d) eine rDNA Sequenz BB, deren Sequenz mindestens 80% Identität mit einer genomischen ribosomalen DNA Sequenz B der Wirtszelle der Hefegattung Arxula aufweist;
e) eine Plasmid-Amplifikationskassette zur Amplifikation des Expressionsvektors in einem Wirt;
wobei der zweite Promotor der Selektionsmarker-Expressionskassette ein modifizierter ALEU2-Promotor ist, dessen Promotoraktivität höchstens etwa 20% der Aktivität des nativen ALEU2-Promotor beträgt, wobei sich die genomische ribosomale DNA Sequenz B im Genom des Hefestammes der Gattung *Arxula* im wesentlichen unmittelbar an das 5' Ende der genomischen ribosomalen DNA Sequenz A anschließt; wobei die rDNA Sequenzen AA und BB jeweils eine Länge von mindestens etwa 40 Nukleotiden und höchstens etwa 2.000 Nukleotide aufweisen; wobei die rDNA Sequenzen AA und BB den von den Vektorabschnitten b) und c) gebildeten Sequenzabschnitt des Expressionsvektors flankieren und wobei die Gesamtlänge des von den Abschnitten a) bis d) eingeschlossenen Abschnitts höchstens etwa 7.6kb beträgt,

und wobei der modifizierte *ALEU2*-Promotor eine der folgenden Nukleinsäuresequenzen enthält oder daraus besteht:
a) eine Nukleinsäuresequenz der SEQ ID Nr. 2, wobei vom 5' Ende der Sequenz ausgehend etwa 0 bis etwa 100 Nukleotide deletiert wurden;
b) eine Nukleinsäuresequenz der SEQ ID Nr. 3, wobei vom 5' Ende der Sequenz ausgehend etwa 0 bis etwa 92 Nukleotide deletiert wurden;
c) eine Nukleinsäuresequenz der SEQ ID Nr. 4, wobei vom 5' Ende der Sequenz ausgehend etwa 0 bis etwa 83 Nukleotide deletiert wurden;
d) eine Nukleinsäuresequenz der SEQ ID Nr. 5, wobei vom 5' Ende der Sequenz ausgehend etwa 0 bis etwa 74 Nukleotide deletiert wurden;
e) eine Nukleinsäuresequenz der SEQ ID Nr. 6, wobei vom 5' Ende der Sequenz ausgehend etwa 0 bis etwa 64 Nukleotide deletiert wurden;
f) eine Nukleinsäuresequenz der SEQ ID Nr. 7, wobei vom 5' Ende der Sequenz ausgehend etwa 0 bis etwa 55 Nukleotide deletiert wurden;
g) eine Nukleinsäuresequenz der SEQ ID Nr. 8, wobei vom 5' Ende der Sequenz ausgehend etwa 0 bis etwa 48 Nukleotide deletiert wurden;
h) eine Nukleinsäuresequenz der SEQ ID Nr. 9, wobei vom 5' Ende der Sequenz ausgehend etwa 0 bis etwa 39 Nukleotide deletiert wurden;
i) eine Nukleinsäuresequenz der SEQ ID Nr. 10, wobei vom 5' Ende der Sequenz ausgehend etwa 0 bis etwa 25 Nukleotide deletiert wurden;
j) eine Nukleinsäuresequenz der SEQ ID Nr. 11, wobei vom 5' Ende der Sequenz ausgehend etwa 0 bis etwa 12 Nukleotide deletiert wurden;
k) eine Nukleinsäuresequenz der SEQ ID Nr. 12, wobei vom 5' Ende der Sequenz ausgehend etwa 0 bis etwa 10 Nukleotide deletiert wurden;
l) eine Nukleinsäuresequenz, die mindestens 50% Sequenzidentität mit einer der Nukleinsäuresequenzen gemäß a) bis k) aufweist und höchstens etwa 20% der Promotoraktivität des nativen ALEU2-Promotors aufweist;
m) eine Nukleinsäuresequenz, die mit einem Gegenstrang einer der Nukleinsäuresequenzen gemäß a) bis 1) unter stringenten Bedingungen hybridisiert und höchstens etwa 20% der Promotoraktivität des nativen ALEU2-Promotors aufweist; und
n) eine Nukleinsäuresequenz, die durch Substitution, Addition und/oder Deletion eines oder mehrerer, vorzugsweise von höchstens etwa 10% der Nukleotide der Nukleinsäuresequenzen gemäß a) bis 1) erzeugt wird und höchstens etwa 20% der Promotoraktivität des nativen ALEU2-Promotors aufweist.

Der von den Abschnitten a) bis d) eingeschlossene Abschnitt des erfindungsgemäßen Expressionsvektors kann überraschenderweise reproduzierbar, multiple und vorzugsweise mitotisch stabil in das Genom von Wirtszellen der Hefegattung *Arxula* integriert werden, wie dies in den Beispielen gezeigt wurde. Dies ermöglicht ein hohes quantitatives Niveau der Produktion heterologer Proteine, wie dies in den Beispielen anhand der Expression des homologen ALIP1-Gens, kodierend für extrazelluläre Lipase von *A. adeninivorans* und des für extrazelluläre alpha-Amylase von *Bacillus amyloliquefaciens* kodierenden *amyA*-Gens gezeigt wird, das dort als Reporter-Gen sowie vorzugsweise auch als für ein heterologes Protein im Sinne der Erfindung kodierendes Gen fungiert.

Unter "multipler genomischer Integration" im Sinne der Erfindung wird verstanden, dass pro Kopie des Hefegenoms der Wirtszelle zwei oder mehr als zwei Kopien des Expressionsvektors durch homologe Rekombination in das Genom der Hefezellen insertiert werden, wobei dabei nur der Teil des Expressionsvektors genomisch integriert wird, der von den Abschnitten a) bis d) eingeschlossen wird (=Hefe-Integrations-Expressions-Kassette, "HK"), wobei gegebenenfalls noch eine oder mehrere Gen-Expressionskassetten, vorzugsweise eine oder mehrere Protein-Expressionskassetten und/oder eine oder mehrere Reportergen-Expressionskassetten hinzutreten können. Die Plasmid-Amplifikationskassette wird dabei bevorzugterweise nicht mit in das Genom der Wirtszelle integriert. Aufgrund der multiplen genomischen Integration kann der Gen-Dosis Effekt für die Expression des heterologen Proteins ausgenutzt werden, d.h. mit steigender Zahl der pro Kopie des Genoms in die Wirtszelle integrierten HK der Expressionsvektoren kann bei Kultivierung der Wirtszellen unter Bedingungen, die eine Expression des heterologen Proteins erlauben, eine hohe Ausbeute des produzierten heterologen Proteins erzielt werden. Dabei sollte bevorzugterweise die Zahl der pro Kopie des Hefegenoms genomisch integrierten Kopien der HK des Expressionsvektors (Amplifikationsgrad) die Grösse von etwa 10 nicht überschreiten. Vorzugsweise liegt der Amplifikationsgrad zwischen etwa 3 und etwa 10, idealerweise bei 4, 5, 6, 7, 8, oder 9, noch bevorzugter zwischen etwa 5 und etwa 10, weiter bevorzugt zwischen etwa 8 und etwa 10. Der Amplifikationsgrad kann nach im Stand der Technik allgemein bekannten Verfahren wie z.B. Southemblot-Hybridisierung bestimmt werden.

Bei den angegebenen Amplifikationsgraden ist die biologische Funktion des rDNA-Clusters des Rezipientenstammes gewahrt, während deutlich höhere Amplifikationsgrade zu einer verminderten mitotischen Stabilität der Hefe-Integrantenstämme führen. Da der Amplifikationsgrad sich auch in der Ausbeute des herstellbaren Proteins niederschlägt, sollte der Amplifikationsgrad so gewählt werden, dass bei hoher Ausbeute der Proteinproduktion eine hinreichende mitotische Stabilität der Integrantenstämme erzielt wird. Die eben genannten Amplifikationsgrade erfüllen diese Bedingung. Vorzugsweise sollte der Amplifikationsgrad daher den Wert von etwa 3 nicht weit unterschreiten (geringe Ausbeute) und idealerweise nicht wesentlich größer als etwa 10 (reduzierte Stabilität) sein.

Wie in den Beispielen gezeigt, eignen sich die erfindungsgemäßen Expressionsvektoren sehr gut zur Produktion heterologer Proteine, vorzugsweise auch zu ihrer Herstellung im großtechnischen Maßstab. Die multiple Integration der HK der Expressionsvektoren wird dadurch ermöglicht, dass pro Kopie des Hefegenoms eine Vielzahl von Kopien der ribosomalen DNA vorhanden sind und beispielsweise bei einem erfindungsgemäßen Rezipientenstamm *A. adeninivorans* eine Kopienzahl von vorzugsweise ca. 50 bis ca. 100 vorliegt.

Die Expressionsvektoren der vorliegenden Erfindung zeichnen sich vorzugsweise dadurch aus, dass sie bei erfindungsgemäßem Einsatz eine mitotisch stabile genomische Integration der HK der Expressionsvektoren in das Genom der Wirtszelle der Hefegattung *Arxula* erlauben, d.h. die nach dem hier beschriebenen Verfahren erzeugten und isolierbaren stabilen Integrantenstämme zeigen über mehr als etwa 10, vorzugsweise mehr als etwa 20, noch bevorzugter über etwa 40, noch bevorzugter über etwa 50, noch bevorzugter über etwa 70, noch bevorzugter über etwa 100 Generationen hinweg keine signifikante Reduktion der multiplen Kopienzahl der in das Genom integrierten HK der Expressionsvektoren. Dies kann durch Bestimmen der Konzentration des produzierten Proteins bei einer gegebenen Zahl von Wirtszellen von Generation zu Generation bestimmt werden, wie dies dem Fachmann allgemein bekannt ist und in den Beispielen illustriert wird. Alternativ kann die multiple Integration auch auf der genomischen DNA Ebene etwa mittels allgemein bekannter Southemblot-Hybridisierungs-Techniken bestimmt werden.

Weiterhin zeichnen sich die erfindungsgemäßen Expressionsvektoren durch ihre biologische Sicherheit aus, da nur die HK mit in das Genom des Rezipienten integriert werden, nicht aber die für industrielle Fermentationen unerwünschte Plasmid-Amplifikationskassette des Expressionsvektors, insbesondere nicht ihre Antibiotikaresistenz-Gene.

Der Begriff "rDNA" im Sinne der Erfindung bezeichnet eine ribosomale DNA, d.h. eine DNA-Sequenz, die zu dem weiter unten spezifizierten Grad der Sequenzidentität mit genomischen ribosomalen DNA Sequenzen der betreffenden Hefe der Gattung *Arxula* übereinstimmt. Der Begriff der "genomischen ribosomalen DNA" ist dabei weit zu fassen und schließt die für ribosomale RNA kodierenden DNA Sequenzen, flankierende Bereiche davon sowie DNA Sequenzen ein, die nur abschnittsweise für ribosomale RNA kodierende DNA-Sequenzen enthalten. Vorzugsweise ist die genomische ribosomale DNA ausgewählt aus einer DNA, die für 5,8S RNA kodiert, einer DNA, die für 18S RNA kodiert, einer DNA, die für 25S RNA kodiert, einer DNA, die für 45S RNA kodiert und einer DNA, die aus einem flankierenden Bereich der vorgenannten RNA kodierenden Bereiche stammt.

Es ist weiterhin bevorzugt, dass die die Bereiche b) und c) des Vektors flankierenden rDNA Sequenzen AA und BB in ihrer relativen Lage und Orientierung zueinander im Vektor, der Lage und Orientierung der entsprechenden sequenzidentischen genomischen ribosomalen DNA Sequenzen A und B im Genom der Wirtszelle entsprechen. In anderen Worten, wenn z.B. das 5' Ende der rDNA Sequenz BB mit dem 3' Ende der rDNA Sequenzen AA gemäß ihrer relativen Orientierung im Expressionsvektor zueinander miteinander verbunden werden, entspricht diese aneinandergefügte Sequenz in ihrer Lage und Orientierung der entsprechenden bei dem spezifizierten Sequenzidentitätsgrad der sequenzidentischen genomischen ribosomalen DNA Sequenz im Genom der betroffenen Wirtszelle, wobei die genomische ribosomale DNA Sequenz für den Zweck des Vergleichs durch Aneinanderreihen der genomischen ribosomalen DNA Sequenzen A und B gebildet werden und dabei das 5' Ende der genomischen ribosomalen DNA Sequenz B an das 3' Ende der genomischen ribosomalen DNA Sequenz A angefügt wird.

Vorzugsweise ist die rDNA-Sequenz AA zu mindestens etwa 80% sequenzidentisch mit der entsprechenden genomischen ribosomalen DNA Sequenz A, noch bevorzugter zu mindestens etwa 90%, noch bevorzugter zu mindestens etwa 95%, noch bevorzugter zu mindestens etwa 98%, noch bevorzugter zu mindestens etwa 99%, am bevorzugtesten zu 100% sequenzidentisch. Vorzugsweise ist die rDNA-Sequenz BB zu mindestens etwa 80% sequenzidentisch mit der entsprechenden genomischen ribosomalen DNA Sequenz B, noch bevorzugter zu mindestens etwa 90%, noch bevorzugter zu mindestens etwa 95%, noch bevorzugter zu mindestens etwa 98%, noch bevorzugter zu mindestens etwa 99%, am bevorzugtesten zu 100% sequenzidentisch. Der Begriff der Sequenzidentität ist dem Fachmann allgemein. Eine bevorzugte Definition ist weiter unten beschrieben.

Da es sich gezeigt hat, dass die Stabilität von Amplifikationsderivaten durch die Größe des in das Genom der Arxula-Hefe integrierten DNA-Fragments beeinträchtigt werden kann, sollte die Gesamtgröße des Expressionsvektors, wie er in die Wirtszelle zum Zwecke der Rekombination eingebracht wird, d.h. der HK, unter Einbeziehung der Länge der für das heterologe Protein kodierenden, in den Vektor zu insertierenden / insertierten Nukleinsäure die Länge von etwa 7.6 kb im wesentlichen nicht überschreiten, wobei eine optional vorhandene Reportergen-Expressionskassette ggf. mit in die Berechnung einbezogen wird. Dieser Wert entspricht der Länge einer rDNA-Kopie eines erfindungsgemäßen Rezipientenstammes *A. adeninivorans.* Vorzugsweise wird die Plasmid-Amplifikationskassette e) nicht mit in die Berechnung der Gesamtgröße des Expressionsvektors einbezogen, da diese vorzugsweise nicht mit in die Wirtszelle integriert wird. Es ist bevorzugt, dass die Gesamtlänge der HK, höchstens etwa 7,6 kb, vorzugsweise höchstens etwa 7 kb, vorzugsweise höchstens etwa 6 kb, noch bevorzugter höchstens etwa 5 kb, noch bevorzugter höchstens etwa 4 kb beträgt.

Um die Stabilität der Wirtszellen zu erhöhen, die die erfindungsgemäßen Expressionsvektoren in ihr Genom integrieren, beträgt in einer bevorzugten Ausführungsform der Erfindung die Länge der rDNA Sequenzen AA und/oder BB mindestens etwa 40, vorzugsweise mindestens etwa 50, noch bevorzugter mindestens etwa 100; jedoch höchstens etwa 4.000 Nukleotide, vorzugsweise höchstens etwa 3.000 Nukleotide, noch bevorzugter höchstens etwa 2.000 Nukleotide, noch bevorzugter höchstens etwa 1.000 Nukleotide, noch bevorzugter höchstens etwa 500 Nukleotide, noch bevorzugter höchstens etwa 400 Nukleotide, noch bevorzugter höchstens etwa 300 Nukleotide, am meisten bevorzugt höchstens etwa 200 Nukleotide. Durch Verkürzung der rDNA Sequenzen des Expressionsvektors wird überraschenderweise auch die mögliche maximale Länge der für das heterologe Protein kodierenden Nukleinsäure vergrößert, die in den Expressionsvektor inseriert werden kann, ohne dabei die Stabilität der genomischen Integration der HK des Expressionsvektors in das Genom der Wirtszelle zu beeinträchtigen. Dadurch ergibt sich auch die Möglichkeit, dass ein für ein zweites und ggf. drittes Protein-kodierendes Gen, vorzugsweise als Teil einer oder weiterer Protein-Expressionskassetten, zusätzlich in den Expressionsvektor inseriert und anschließend stabil integriert werden kann.

Durch die Reduzierung der Größe des genomisch integrierenden Fragments konnte überraschenderweise zudem eine mehrfache Steigerung der genomischen Integrantenhäufigkeit erreicht werden. Die so bedingte Steigerung der Integrantenhäufigkeit ist besonders in Hinsicht auf die multiple Integration vorteilhaft, wie es in den Beispielen gezeigt wurde.

Im Sinne der Erfindung bezieht sich der Ausdruck "% Identität" zweier Sequenzen auf die Identität auf DNA Ebene, die gemäß bekannter Verfahren, z. B. der computergestützten Sequenzvergleiche (Altschul et al. 1990) bestimmt werden kann. Der dem Fachmann bekannte Ausdruck "Identität" bezeichnet dabei den Grad der Verwandtschaft zwischen zwei oder mehr als zwei DNA-Molekülen, der durch die Übereinstimmung zwischen den Sequenzen bestimmt wird. Der Prozentsatz der "Identität" ergibt sich aus dem Prozentsatz identischer Bereiche in zwei oder mehr Sequenzen unter Berücksichtigung von Lücken oder anderen Sequenzbesonderheiten.

Die Identität miteinander verwandter DNA-Moleküle kann mit Hilfe bekannter Verfahren bestimmt werden. Computerprogramme zur Bestimmung der Identität zwischen zwei Sequenzen umfassen das GCG-Programmpaket, einschließlich GAP (Devereux et al. 1984); Genetics Computer Group University of Wisconsin, Madison, USA), BLASTP, BLASTN und FASTA (Altschul et al. 1990). Das BLAST X Programm kann vom National Center for Biotechnology Information (NCBI) oder aus anderen Quellen bezogen werden (BLAST Handbuch, Altschul S. et al., NCB NLM NIH Bethesda MD 20894; Altschul et al. 1990). Auch der allgemein bekannte Smith Waterman-Algorithmus kann zur Bestimmung der Sequenzidentität verwendet werden.

Ein bevorzugter Algorithmus nebst Parametern für den Sequenz-Vergleich ist
- Algorithmus : Needleman und Wunsch (1970)
- Vergleichsmatrix : Übereinstimmung (matches)+ 10,
   Nichtübereinstimmung (mismatch)= 0
- Lücken-Wert (Gap Penalty) : 15
- Lückentängen-Wert : (Gap Length Penalty) : 1 .

Das GAP-Programm ist auch zur Verwendung mit den vorstehenden Parametern geeignet. Die vorstehenden Parameter sind die Standardparameter für Nukleinsäuresequenz-Vergleiche.

Der Begriff "Nukleinsäure" ist dem Fachmann bekannt und bezieht sich unter anderem auf RNA sowie DNA, die einzel- oder doppeisträngig sein können. Ferner schließt der Begriff auch modifizierte DNA oder RNA Moleküle ein.

Der Begriff "Wirtszelle" bezieht sich vor allem auf Hefezellen und bevorzugterweise auch auf Hefestämme, vorzugsweise auf jene der Gattung Arxula. Der Begriff "Wirtszelle der Hefegattung *Arxula*", wie er erfindungsgemäß verstanden werden soll, ist dem Fachmann allgemein bekannt und schließt insbesondere folgende Hefen ein die aus *Arxula adeninivorans* und *Arxula terrestris* abgeleitet sind oder identisch sind mit diesen Hefen.

Der Begriff "Promotor" ist dem Fachmann ebenfalls geläufig und schließt nach dem Verständnis der Erfindung beliebige konstitutive oder induzierbare Promotoren ein, solange sie in einer Hefe der Gattung *Arxula* und insbesondere in der für die Expression des heterologen Proteins ausgewählten Hefezelle die Transkription eines Gens kontrollieren oder steuern können. Vorzugsweise stammen die Promotoren der vorliegenden Erfindung aus einer Hefe der Gattung *Arxula,* können jedoch, sofern ihre biologische Funktion in einer Hefe der Gattung *Arxula* aufrecht erhalten wird, auch aus anderen Spezies, wie anderen Hefen, Bakterien oder Nichtwirbeltieren oder Wirbeltieren stammen. Vorzugsweise enthält der Promotor zusätzlich eine TATA-Box.

"Konstitutive Promotoren" sind dem Fachmann allgemein bekannt und schließen im Sinne der Erfindung Promotoren ein, die die Transkription des unter ihrer Kontrolle stehenden Gens vorzugsweise unabhängig vom metabolischen Zustand der Wirtszelle aktivieren und kontrollieren und auch ohne aktivierende oder disinhibierende Mittel aktiv sind und bleiben, die etwa der Kultur von außen zugefügt werden. Die Aktivität der Promotoren kann nach dem Fachmann allgemein bekannten Verfahren bestimmt werden.

Bevorzugte konstitutive Promotoren sind *ALEU2, TEF1, ATRP1, AHSB4, AILV1* und *ARFC3,* deren Sequenzen und Eigenschaften in den nachfolgenden Publikationen eingehend beschrieben werden (Wartmann T. et al. 2003: The ALEU2 gene - a new component for an Arxula adeninivorans-based expression platform. FEMS Yeast Res. 3, Curr. Genet. 28: 360-366; Steinborn G. et al. 2006, J. Biotechnol. Doi:10.1016/j.jbiotec.2006.07.026; Wartmann T. et al. 2003, Appl. Microbiol. Biotechnol. 62: 528-535; Wartmann T. et al. 1998, Yeast 14: 1017-1025; Stoltenburg R. et al. 1999, Curr. Genet. 35: 8-13).

Der Begriff der "induzierbaren Promotoren" ist dem Fachmann ebenfalls geläufig und schließt im Sinne der Erfindung Promotoren ein, die zu ihrer Transkriptionsaktivität auf aktivierende oder disinhibierende Mittel, physikalische oder chemische Reize oder Signale angewiesen sind, deren An- oder Abwesenheit vorzugsweise durch den Experimentator, etwa durch Zugabe entsprechender Promotor-Aktivatoren zum Kulturmedium gesteuert werden kann und so eine gezielte Kontrolle der Transkription des Gens erlaubt, das unter der Kontrolle des betreffenden induzierbaren Promotors steht. Bevorzugte induzierbare Promotoren sind *GAA, AXDH, AINV, AHOG1, ALIP1, APHO1, AFET3, AEFG1* und *ATAN,* deren Sequenzen und Eigenschaften in den nachfolgenden Publikationen eingehend beschrieben werden (Bui D.M. et al. 1996, Appl. Microbiol. Biotechnol. 44: 610-619; Böer E. et al. 2005, Antonie van Leeuwenhoek 87: 233-243; Böer E. et al. 2004, Antonie van Leeuwenhoek 86: 121-134; Böer E. et al. 2004, Curr. Genet. 46: 269-276; Böer et al. 2005, Yeast 22: 523-535; Kaur P. et al. 2006, Antonie van Leeuwenhoek doi 10.1007/s10482-006-9094-6; Wartmann T. et al. 2002, Yeast 19: 849-862; Wartmann T. et al. 2001, Curr. Genet. 40: 172-178; DE 10 2004 032 216 A1).

Vorzugsweise sind die Promotoren der Erfindung keine der in der WO 01/85925 offenbarten Promotoren. Insbesondere stammen die Promotoren der Erfindung nicht von Promotoren ab, die von einem Gen stammen, das ausgewählt aus *ARFC3, AHSB4, GAA, AACP10, AACP10, APCR17, APRE4, AACE1, ATAL1, AGLC7, AFET3, AFTR1, AEFG1, ASTE6, APMD1* und *AXDH.*

Vorzugsweise sind die Promotoren der Erfindung solche der in der WO 01/85925 offenbarten Promotoren. So können die Promotoren der Erfindung von Promotoren abstammen, die von einem Gen stammen, das ausgewählt ist aus *ARFC3, AHSB4, GAA, AACP10, AACP10, APCR17, APRE4, AACE1, ATAL1, AGLC7, AFET3, AFTR1, AEFG1, ASTE6, APMD1* und *AXDH.*

Nach einer weiteren bevorzugten Ausführungsform der Erfindung ist der erste Promotor ein konstitutiver oder induzierbarer Promotor, vorzugsweise ein starker Promotor. Der Begriff des starken Promotors ist allgemein bekannt. Vorzugsweise ist ein getesteter Promotor ein starker Promotor im Sinne der Erfindung, wenn er bei Einsatz eines gegebenen Gens, wie eines Reportergens (z.B. *amyA, ALEU2, AILV1, ATRP1)* unter Kontrolle des getesteten Promotors eine Promotoraktivität zeigt, die mindestens etwa 70%, vorzugsweise mindestens etwa 80%, vorzugsweise mindestens etwa 90%, noch bevorzugterweise mindestens etwa 95%, am bevorzugtesten mindestens im wesentlichen die gleiche Promotoraktivität aufweist, die gleichen experimentellen Bedingungen unter Einsatz des *TEF1*-Promotors anstelle des zu testenden Promotors erzielt werden. Besonders bevorzugt ist bei der Bestimmung der Promotoraktivität der Einsatz des Vektors pGH69 oder des pGH72 Vektors, wobei zur Bestimmung der Promotoraktivität des Testpromotors der *TEF1*-Promotor durch den Testpromotor ausgetauscht wird und das *amy*A-Gen unter Transkriptionskontrolle des Testpromotors steht. Verfahren zur Bestimmung der Promotoraktivität sind dem Fachmann bekannt, in den Beispielen beschrieben und schließen zum Beispiel die Messung der β-Isopropylmalatdehydrogenase (Genprodukt von *ALEU2*), bei Einsatz des *ALEU2*-Gens als Reportergen; Treonindeaminase (Genprodukt von *AILV1*) bei Einsatz des A*ILV1*-Gens als Reportergen; bzw. Phosphoribosylanthranilat-isomerase (Genprodukt von *ATRP1*), bei Einsatz des *ATRP1*-Gens als Reportergen ein (Samsonova I. et al. 1996, Yeast 12, 1209-1217).

Vorzugsweise ist der erste Promotor ein Promotor ausgewählt aus *TEF1, GAA, ALIP1* und *ATAN*-Promotor.

Der Begriff "Expressionskassette" ist dem Fachmann allgemein bekannt und schließt nach dem Verständnis der vorliegenden Erfindung Sequenzen ein, die vorzugsweise in 5' nach 3' Richtung folgende Elemente enthalten: mindestens einen Promotor sowie mindestens eine für ein Protein kodierende Nukleinsäuresequenz, möglicherweise mit oder ohne Intronsequenzen, wobei die kodierende Nukleinsäuresequenz vorzugsweise relativ zu dem oder den Promotor(en) im gleichen Leserahmen angeordnet ist und bevorzugterweise mit dem Promotor funktionsfähig verknüpft ist, d.h. dass die kodierende Nukleinsäure und der Promotor so angeordnet sind, dass der Promotor die Transkription der kodierenden Nukleinsäure aktivieren und/oder kontrollieren kann. Dabei liegt dass 5' Ende der kodierenden Nukleinsäure vorzugsweise am oder hinter dem 3' Ende des Promotors. Am oder hinter dem 3' Ende der kodierenden Nukleinsäure enthält die Expressionskassette gegebenenfalls ein oder mehrere Terminatorsequenz(en), sowie weiterhin optional mindestens eine Restriktionsschnittsstelle am 5' und/oder am 3' Ende der Expressionskassette wobei die Restriktionsschnittsstellen gleich oder verschieden sein können. Vorzugsweise enthält die Expressionskassette weitere Promotoren und/oder Enhancerelemente, die in dem Fachmann bekannter Weise in der Expressionskassette angeordnet werden können.

Eine "für ein heterologes Protein kodierende Nukleinsäure", wie der Ausdruck hier verwendet wird, bezeichnet eine kodierende Nukleinsäuresequenz, die von einem anderen Gen als der zur Kontrolle ihrer Transkription eingesetzten Promotor stammt. Die kodierende Nukleinsäuresequenz kann aus Prokaryonten oder Eukaryonten stammen. Unter den Prokaryonten sind Bakterien wie etwa *E. coli* oder *Bacillus* zu nennen. Der Begriff "Eukaryont" schließt Wirbellose wie Hefen, Mollusken, Insekten und Nematoden ein, sowie Wirbeltiere, hierbei insbesondere Säugetiere und dabei Nutztiere, Ratten Mäuse, und Menschen. Beispiele für heterologe Proteine im Sinne der Erfindung sind unter anderem EPO, Insulin, Wachstumsfaktoren, Phytase, HGH, Hirudin, Lactoferrin oder G-CSF. Die "kodierende Nukleinsäure" kodiert vorzugsweise auch für funktionelle Varianten der heterologen Proteine, die durch Modifikation, d.h. durch Deletion, Insertion, Inversion, Substitution mindestens eines Nukleotids aus der nativen Gen-Sequenz erzeugt werden und eine im wesentlichen gleiche biologische Aktivität wie das native heterologe Protein zeigen. Ebenso eingeschlossen sind vorzugsweise für Vorläuferstufen des heterologen Proteins kodierende Nukleinsäuren, beispielsweise etwa Nukleinsäuren, die für ein Pro- oder Präpropolypeptid des heterologen Proteins kodieren. Dabei kann die kodierende Nukleinsäure auch mindestens ein Intron enthalten oder aber frei von Intronsequenzen sein. Vorzugsweise umfasst die kodierende Nukleinsäure den für das heterologe Protein kodierenden Bereich. Dieser Bereich beginnt bei Eukaryonten mit dem ersten in einer Kozak Sequenz (Kozak, 1987, Nucleic. Acids Res. 15: 8125-48) liegenden Start-Codon (ATG) und reicht bis zum nächsten Stop-Codon (TAG, TGA bzw. TAA), das im gleichen Leseraster zum ATG liegt. Bei Prokaryonten beginnt dieser Bereich mit dem ersten AUG (oder GUG) nach einer Shine-Dalgamo-Sequenz und endet mit dem nächsten Stop-Codon (TAG, TGA bzw. TAA), das im gleichen Leseraster zum ATG liegt.

Ferner kann die kodierende Nukleinsäure eine Poly-A Sequenz und/oder eine für ein Signalpeptid kodierende Sequenz enthalten. Auch für Spleißvarianten der für das heterologe Proteins kodierenden Nukleinsäure sind vorzugsweise eingeschlossen von dem Begriff der "für ein heterologes Protein kodierenden Nukleinsäure".

Es ist bekannt, dass Veränderungen in der Sequenz der erfindungsgemäßen Nukleinsäuren vorhanden sein können, zum Beispiel durch die Degenerierung des genetischen Codes, oder dass nicht translatierte Sequenzen am 5' und/oder 3'-Ende der Nukleinsäure angehängt sein können, ohne dass die Aktivität des kodierten Polypeptides dadurch wesentlich verändert wird. Die kodierenden Nukleinsäuren umfassen deshalb vorzugsweise auch derartige Varianten der vorangehend beschriebenen Nukleinsäuren.

Nach einer bevorzugten Ausführungsform der Expressionsvektoren handelt es sich bei den heterologen Proteinen um solche, die für die Zwecke des Screenings von Hefezellen eingesetzt werden können, die die HK der Expressionsvektoren rekombinant und multiple integriert haben. Diese bevorzugten heterologen Proteine weisen dabei einen einfach zu detektierenden Phänotyp auf, d.h. einen Phänotyp der nach Expression durch die transformierte Hefezelle mit geringem methodischen Aufwand qualitativ und/oder quantitativ ausgewertet werden kann. Darunter fallen etwa extrazelluläre Enzyme, die es erlauben, etwa durch Zugabe eines spezifischen Enzymsubstrats zu Agarplatten, die enzymatische Umsetzung qualitativ und/oder quantitativ anhand von Abbauhöfen um wachsende Kolonien einfach zu analysieren, da die enzymatische Umsetzung ein sichtbares Produkt erzeugt. Dem Fachmann sind derlei bevorzugte heterologe Proteine, z. B. Amylasen, DNasen, Glukanasen, Glukosidasen, Lipasen, RNasen, Phosphatase, Tannasen und Proteasen, allgemein bekannt.

Vorzugsweise enthält der Expressionsvektor, innerhalb des von den Abschnitten a) bis d) gebildeten Sequenzabschnitts, d.h. der HK, zusätzlich mindestens einen Polylinker oder aber mindestens eine Restriktionsschnittstelle. Der Polylinker enthält eine Sequenz, die mindestens zwei Restriktionsschnittstellen enthält. Vorzugsweise ist der Polylinker am oder hinter dem 3' Ende des ersten Promotors der Proteinexpressionskassette angeordnet und erlaubt eine funktionsfähige Insertion der für das heterologe Protein kodierenden Nukleinsäure, so dass die insertierte kodierende Nukleinsäure unter der Transkriptionskontrolle des ersten Promotors steht. Geeignete Polylinker und Restriktionsschnittstellen sowie Verfahren zur Klonierung der kodierenden Nukleinsäuren in diese Proteinexpressionskassetten sind dem Fachmann allgemein bekannt (z.b. Sambrook et al., Molecular cloning. A laboratory manual. Cold Spring Harbor Laboratory Press, Cold Spring Harbor, 2001) und können im Rahmen routinemäßiger Arbeiten ausgewählt und erzeugt werden.

Weiterhin kann der Expressionsvektor am oder hinter dem 5' Ende der rDNA Sequenz AA und am oder hinter dem 3' Ende der rDNA Sequenz BB mindestens eine Restriktionsschnittstelle enthalten. Vorzugsweise kommen diese Restriktionsschnittstellen nicht außerhalb der eben bezeichneten Stellen vor und können von den gleichen oder von verschiedenen Restriktionsendonukleasen geschnitten werden.

Dabei ist der Begriff "am oder hinter dem Ende der Sequenz" im Sinne der Erfindung weit zu verstehen und schließt auch Restriktionsschnittstellen ein, die ohne Abstand oder in einem Abstand von höchstens etwa 500 Nukleotiden, vorzugsweise von höchstens etwa 200 Nukleotiden, vorzugsweise von höchstens etwa 100 Nukleotiden, vorzugsweise von höchstens etwa 50 Nukleotiden, noch bevorzugter von höchstens etwa 10 Nukleotiden von dem letzten Nukleotid der betreffenden Sequenz liegen.

Die HK der erfindungsgemäßen Expressionsvektoren wird durch mindestens eine Restriktionsschnittstelle, vorzugsweise an oder hinter ihrem 5' Ende und/oder an oder hinter dem 3' Ende durch je eine gleiche Restriktionsschnittstelle, von der Plasmid-Amplifikationskassette abgegrenzt. Ein erfindungsgemäßer Expressionsvektor, enthält eine Plasmid-Amplifikationskassette zur Amplifikation des Expressionsvektors in einem Wirt, vorzugsweise einem bakteriellen Wirt, vorzugsweise in *E.coli* oder *Bacillus.* Vorzugsweise weist die Plasmid-Amplifikationskassette an oder hinter ihrem 3' Ende und/oder an oder hinter ihrem 5' Ende mindestens eine Restriktionsschnittstelle auf, die ein Abtrennen der Plasmid-Amplifikationskassette aus dem Expressionsvektors erlaubt, ohne dabei den übrigen Expressionsvektorteil zu zerschneiden, wobei die Restriktionsschnittstellen gleich oder verschieden sein können. Es ist bevorzugt, dass diese Restriktionsschnittstellen insgesamt nur je einmal, an den eben bezeichneten Stellen des Expressionsvektors vorkommen. Die Herstellung und Zusammenstellung der erforderlichen Komponenten der Plasmid-Amplifikationskassetten wird von dem zur Amplifikation des Expressionsvektors verwendeten Wirtsorganismus abhängen und ist dem Fachmann allgemein bekannt, etwa aus Sambrook et al., 2001, supra). Beispiele derartiger Plasmid-Amplifikationskassetten sind in den Beispielen und den Figuren beschrieben. Vorzugsweise enthält die Plasmid-Amplifikationskassette mindestens ein Replikationsorigin, wie ColE1 sowie ein Selektionsmarkergen, z.B. ein Antibiotika-Resistenzmarkergen wie ein Ampicillin-Markergen, gegebenenfalls und an oder hinter dem 5' Ende der Plasmid-Amplifikationskassette und/oder an oder hinter dem 3' Ende der Plasmid-Amplifikationskassette mindestens eine Restriktionsschnittstelle, wobei die Restriktionsschnittstelle vorzugsweise in dem Expressionsvektor nicht außerhalb der Plasmid-Amplifikationskassette vorkommt. Dabei sollten die Elemente der Plasmid-Amplifikationskassette auf den zur Klonierung eingesetzten Wirt angepasst werden, wie dies dem Fachmann allgemein geläufig ist.

Nach einer weiteren bevorzugten Ausführungsform ist die Plasmid-Amplifikationskassette in dem Bereich des Expressionsvektors angeordnet, der zwischen der rDNA Sequenz AA und der rDNA Sequenz BB liegt, wobei in diesem Bereich keiner der Sequenzabschnitte b) und c) angeordnet ist. In diesem Fall, liegen die eben vorgestellten Restriktionsschnittstellen zwischen der Plasmid-Amplifikationskassette und dem 5' Ende der rDNA Sequenz AA und/oder zwischen der Plasmid-Amplifikationskassette und dem 3' Ende der rDNA Sequenz BB. Eine bevorzugte Anordnung der Sequenzabschnitte a) bis d) relativ zu der Plasmid-Amplifikationskassette ist in dem Vektor pGH69 realisiert.

Zur Verbesserung der biologischen Sicherheit der unter Einsatz der erfindungsgemäßen Expressionsvektoren durchführbaren erfindungsgemäßen Verfahren zur Herstellung heterologer Proteine wird in einer bevorzugten Ausführungsform des Expressionsvektors die Plasmid-Amplifikationskassette des Expressionsvektors von der Integration in das Genom der Wirtszelle ausgeschlossen. Dies kann bewerkstelligt werden, indem die Plasmid-Amplifikationskassette vor der Transformation der Hefen mit dem Expressionsvektor mittels Restriktionsendonukleaseverdau aus dem Expressionsvektor entfernt wird.

Vorzugsweise enthält die Proteinexpressionskassette einen induzierbaren oder konstitutiv aktiven ersten Promotor. Vorzugsweise ist der erste Promotor ein wie oben definierter starker Promotor. Der Promotor ist vorzugsweise ausgewählt aus *TEF1-, GAA-, ALIP1-* und *ATAN-* Promotor. Die Insertion der kodierenden Nukleinsäure am 3' Ende oder hinter dem 3' Ende des ersten Promotors erfolgt vorzugsweise funktionsfähig und im gleichen Leserahmen des ersten Promotors, so dass die insertierte kodierende Nukleinsäure unter der Transkriptionskontrolle des ersten Promotors steht. Die Konstruktion der Proteinexpressionskassette kann vom Fachmann auf der Grundlage allgemein bekannter Techniken bewerkstelligt werden. Vorzugsweise enthält der Expressionsvektor mehr als eine Proteinexpressionskassette. Alternativ oder zusätzlich kann unter Einsatz von IRES (Inter-ribosomal entry site) Sequenzen, die in einer Proteinexpressionskassette zwischen zwei jeweils für ein heterologes Protein kodierenden Nukleinsäuren angeordnet werden, eine bicistronische Kassette erzeugt werden. Diese bicistronische Kassette gestattet es, die beiden hintereinandergeschalteten kodierenden Nukleinsäuren unter Kontrolle eines Promotors zu transkribieren, wie dies allgemein bekannt ist. Vorzugsweise sind die beiden kodierenden Nukleinsäuren dabei im gleichen Leserahmen angeordnet.

Der Begriff "Selektionsmarkergen" ist dem Fachmann ebenfalls allgemein bekannt. Im Sinne der Erfindung umfasst der Begriff dominante Marker wie etwa das *hph*-Gen (kodiert für Hygromycin B Resistenz) und vor allem Auxotrophiemarker-Gene. Dabei handelt es sich um Gene, deren Expressionsprodukte in auxotrophen Hefezellen der Gattung *Arxula* eine Auxotrophie komplementieren können. Bevorzugt ist ein Selektionsmarkergen aus *Arxula,* wie *ALEU2, ATRP1, ALYS2* und *AILV1* von *A. adeninivorans,* oder eine Variante der vorgenannten Gene, die die Funktion des jeweiligen Selektionsmarkergens im wesentlichen beibehält, d.h. die gegebene Auxotrophie komplementieren kann. Die Integranten sind dabei über die Hygromycin-Resistenz bzw. über die Komplementation des *aleu2, atrp1* bzw. *ailv1-*Markers durch das *ALEU2-, ATRP1-* bzw. *AILV1*-Gen identifizierbar. Die Sequenzen der vorgenannten dominanten Marker und Selektionsmarkergene sowie ihre Eigenschaften können den nachfolgenden Referenzen entnommen werden (Rösel, H. & Kunze, G. 1998, Curr. Genet. 33, 157-163; Wartmann, T. et al. 1998, Yeast 14, 1017-1025; Wartmann, T. et al. 2002, FEMS Yeast Res. 2, 363-369; Wartmann, T. et al. 2003, FEMS Yeast Res. 3, 223-232; Steinborn, G. 2005, FEMS Yeast Res. 5, 1047-1054/ Steinborn G. et al. 2006, J. Biotechnol. Doi:10.1016/j.jbiotec.2006.07.026. b).

Zum erfindungsgemäßen Einsatz der Expressionsvektoren müssen die in diesen Vektoren vorhandenen Selektionsmarkergene mit der Auxotrophie der einzusetzenden Wirtszellen abgestimmt werden, damit die Kandidaten-Integranten unter Einsatz der Selektionsmarker auf genomische Integration und/oder multiple genomische Integration gescreent werden können, wie dies in den Beispielen illustriert wurde. Der Einsatz eines Tryptophan-Auxotrophie komplementierenden Auxotrophiemarkergens wie *ATRP1* ist bevorzugt, da Tryptophan durch Autoklavieren inaktiviert wird und deshalb in sterilisierten Vollmedien ein selektiver Druck für Trp-auxotrophe Rezipientenhefezellen besteht. Geeignete auxotrophe Wirtszellen (Hefestämme) sind beschrieben und charakterisiert in Samsonova I. et al. 1996: A set of genetic markers for the chromosomes of the imperfect yeast Arxula adeninivorans; Yeast 12, 1209-1217; sowie in Steinborn G. et al. 2006, J. Biotechnol. Doi:10.1016/j.jbiotec.2006.07.026. Bevorzugte auxotrophe Wirtszellen (Hefestämme) sind *A. adeninivorans* G1211 [*aleu2-29*] ( Samsonova I. et al. 1996, Yeast 12, 1209-1217 ) und *A. adeninivorans* G1212 [*aleu2 atrp1::ALEU2*] (Steinborn G. et al. 2006, J. Biotechnol. Doi:10.1016/j.jbiotec.2006.07.026).

Der zweite Promotor der Selektionsmarker-Expressionskassette ist ein modifizierter ALEU2-Promotor.

Ein getesteter Promotor ist ein schwacher Promotor, wenn er bei Einsatz eines gegebenen Gens, wie eines Reportergens (z.B. *amyA, ALEU2, AILV1, ATRP1*) unter Kontrolle des getesteten Promotors eine Promotoraktivität zeigt, die im wesentlichen die gleiche Promotoraktivität aufweist, die bei gleichen experimentellen Bedingungen unter Einsatz von *ALEU2* anstelle des zu getesteten Promotors erzielt werden. Besonders bevorzugt ist bei der Bestimmung der Promotoraktivität der Einsatz des Vektors pGH69 oder pGH72, wobei zur Bestimmung der Promotoraktivität des Testpromotors der *ALEU2*-Promotor durch den Testpromotor ausgetauscht wird und das *amyA*-Gen unter Transkriptionskontrolle des Testpromotors steht. Verfahren zur Bestimmung der Promotoraktivität sind dem Fachmann bekannt, und wurden weiter oben und in den Beispielen beschrieben.

Gemäß der Erfindung ist der zweite Promotor ein gegenüber dem nativen Promotor modifizierter Promotor, dessen Promotoraktivität höchstens etwa 20%, vorzugsweise höchstens etwa 10%, vorzugsweise die höchstens etwa 5%, noch bevorzugter höchstens etwa 1 % der Promotoraktivität des *ALEU2*-Promotors beträgt, die bei gleichen experimentellen Bedingungen unter Einsatz des *ALEU2* anstelle des modifizierten Promotors erzielt werden, wobei der *ALEU2-*Promotor der native *ALEU2*-Promotor ist. Verfahren zur Bestimmung der Promotoraktivität sind dem Fachmann bekannt und wurden weiter oben beschrieben. Ein "modifizierter Promotor" ist dabei ein Promotor, der durch Mutation, Deletion, Insertion, Substitution von mindestens etwa einem Nukleotid und höchstens der Gesamtzahl der Nukleotide, vorzugsweise von etwa 100, vorzugsweise höchstens etwa 80, vorzugsweise höchstens etwa 60, vorzugsweise höchstens etwa 40, vorzugsweise höchstens etwa 20, noch vorzugsweise höchstens etwa 10, am meisten bevorzugt höchstens etwa 10 Nukleotiden aus dem nativen Promotor gewonnen werden kann.

Durch Modifikation der Promotoren in der Selektionsmarker-Expressionskassette kann der den Wirtszellen auferlegte Selektionsdruck genau eingestellt werden, wie es erforderlich ist, damit die Integranten nur bei Vorhandensein eines gewissen Amplifikationsgrades der genomisch integrierten Expressionsvektoren unter den gewählten Selektionsbedingungen wachsen zu können. Dies ist in den Beispielen beschrieben worden. Die geeignete Promotoraktivität wird z. B. von der Wahl des zweiten Promotors, des Selektionsmarkergens, der transformierten Wirtszelle und den Kultivierungsbedingungen abhängen und kann auf der Grundlage der vorliegenden Anleitung und der Beispiele im Rahmen routinemäßiger Versuche bestimmt werden. Bei Einsatz eines Auxotrophiemarkergens treten auch die Konzentration des Stoffes im Kulturmedium hinzu, für den die ausgewählte Hefezelle auxotroph ist und dessen Auxotrophie durch das ausgewählten Selektionsmarkergen komplementiert werden kann.

Die optimalen Parameter zur Erzeugung von multiplen genomischen Integranten mit mitotischer Stabilität und der dazu erforderlichen Auswahl der experimentellen Bedingungen und Promotoren kann der Fachmann in Kenntnis der vorliegenden Anleitung und der Beispiele im Rahmen routinemäßiger Versuche bestimmen.

Gemäß der Erfindung ist der zweite Promotor ein *ALEU2*-Promotor, der eine der folgenden Nukleinsäuresequenzen enthält oder daraus besteht:
a) eine Nukleinsäuresequenz der SEQ ID Nr. 2, wobei vom 5' Ende der Sequenz ausgehend etwa 0 bis etwa 100, vorzugsweise etwa 1 bis etwa 110 Nukleotide deletiert wurden;
b) eine Nukleinsäuresequenz der SEQ ID Nr. 3, wobei vom 5' Ende der Sequenz ausgehend etwa 0 bis etwa 92, vorzugsweise etwa 1 bis etwa 102 Nukleotide deletiert wurden;
c) eine Nukleinsäuresequenz der SEQ ID Nr. 4, wobei vom 5' Ende der Sequenz ausgehend etwa 0 bis etwa 83, vorzugsweise etwa 0 bis etwa 93 Nukleotide deletiert wurden;
d) eine Nukleinsäuresequenz der SEQ ID Nr. 5, wobei vom 5' Ende der Sequenz ausgehend etwa 0 bis etwa 74, vorzugsweise etwa 0 bis etwa 84 Nukleotide deletiert wurden;
e) eine Nukleinsäuresequenz der SEQ ID Nr. 6, wobei vom 5' Ende der Sequenz ausgehend etwa 0 bis etwa 64, vorzugsweise etwa 0 bis etwa 74 Nukleotide deletiert wurden;
f) eine Nukleinsäuresequenz der SEQ ID Nr. 7, wobei vom 5' Ende der Sequenz ausgehend etwa 0 bis etwa 55, vorzugsweise etwa 0 bis etwa 65 Nukleotide deletiert wurden;
g) eine Nukleinsäuresequenz der SEQ ID Nr. 8, wobei vom 5' Ende der Sequenz ausgehend etwa 0 bis etwa 48, vorzugsweise etwa 0 bis etwa 58 Nukleotide deletiert wurden;
h) eine Nukleinsäuresequenz der SEQ ID Nr. 9, wobei vom 5' Ende der Sequenz ausgehend etwa 0 bis etwa 39, vorzugsweise etwa 0 bis etwa 49 Nukleotide deletiert wurden;
i) eine Nukleinsäuresequenz der SEQ ID Nr. 10, wobei vom 5' Ende der Sequenz ausgehend etwa 0 bis etwa 25, vorzugsweise etwa 0 bis etwa 35 Nukleotide deletiert wurden;
j) eine Nukleinsäuresequenz der SEQ ID Nr. 11, wobei vom 5' Ende der Sequenz ausgehend etwa 0 bis etwa 12, vorzugsweise etwa 0 bis etwa 22 Nukleotide deletiert wurden;
k) eine Nukleinsäuresequenz der SEQ ID Nr. 12, wobei vom 5' Ende der Sequenz ausgehend etwa 0 bis etwa 10, vorzugsweise etwa 0 bis etwa 20 Nukleotide deletiert wurden;
l) eine Nukleinsäuresequenz, die mindestens 50% Sequenzidentität mit einer der Nukleinsäuresequenzen gemäß a) bis k) aufweist und höchstens etwa 20% der Promotoraktivität des nativen *ALEU2*-Promotors aufweist;
m) eine Nukleinsäuresequenz, die mit einem Gegenstrang einer der Nukleinsäuresequenzen gemäß a) bis 1) unter stringenten Bedingungen hybridisiert und höchstens etwa 20% der Promotoraktivität des nativen *ALEU2*-Promotors aufweist; und
n) eine Nukleinsäuresequenz, die durch Substitution, Addition und/oder Deletion eines oder mehrerer, vorzugsweise von höchstens etwa 10% der Nukleotide der Nukleinsäuresequenzen gemäß a) bis 1) erzeugt wird und höchstens etwa 20% der Promotoraktivität des nativen *ALEU2*-Promotors aufweist.

Verfahren zur Bestimmung von Sequenzen, die unter stringenten Hybridisierungsbedingungen mit dem Gegenstrang einer gegebenen zweiten Sequenz hybridisieren, sind dem Fachmann geläufig und etwa bei Sambrook et al., 2001 beschrieben. Bevorzugterweise sind stringente Hybridisierungsbedingungen beispielsweise Hybridisierung bei mindestens etwa 42° C in einer Hybridisierungslösung, die etwa 6xSSC enthält sowie ein Waschschritt bei mindestens etwa 42° C für 1 Stunde in einer Waschlösung, die etwa 0.5xSSC und etwa 0.5% SDS enthält. Es ist bevorzugt, die Hybridisierung und den Waschschritt jeweils bei mindestens etwa 50° C, noch bevorzugter bei mindestens etwa 55° C, am meisten bevorzugt bei mindestens etwa 60° C auszuführen.

Es ist ferner bevorzugt, dass die unter m) definierte Nukleinsäuresequenz mit einem Gegenstrang einer der Nukleinsäuresequenzen gemäß a) bis 1) unter hoch stringenten Bedingungen hybridisiert und höchstens 20% der Promotoraktivität des nativen *ALEU2*-Promotors aufweist. Hoch stringente Hybridierungsbedingungen schließen eine Hybridisierung bei mindestens etwa 65° C in einer Hybridisierungslösung ein, die etwa 6xSSC enthält sowie einen Waschschritt bei mindestens etwa 65° C für 1 Stunde in einer Waschlösung, die etwa 0.5xSSC und etwa 0.5% SDS enthält.

Es ist weiterhin bevorzugt, dass der unter I), m) und/oder n) definierte Promotor eine Promotoraktivität aufweist, die höchstens etwa 20%, insbesondere höchstens etwa 10%, vorzugsweise höchstens etwa 5%, noch bevorzugter höchstens etwa 1 % der Aktivität des nativen ALEU2-Promotors beträgt. Die Messung der *ALEU2*-Promotoraktivität erfolgt nach allgemein bekannten Verfahren zum Beispiel über die Messung der vom *ATRP1*-Gen kodierten Phosphoribosylanthranilat-Isomerase Aktivität.

Vorzugsweise enthält der Expressionsvektor ferner eine Reportergen-Expressionskassette, die einen dritten konstitutiven oder induzierbaren Promotor, vorzugsweise aus der Hefegattung *Arxula,* sowie ein Reportergen enthält, das unter der Transkriptionskontrolle des Promotors steht, wobei die Reportergen-Expressionskassette in dem Sequenzabschnitts angeordnet ist, der von den rDNA Sequenzen AA und BB flankiert wird und in dem auch die Abschnitte b) und c) liegen. Eine bevorzugte Anordnung der Sequenzabschnitte a) bis e) ist in dem Vektor pGH69 realisiert. Derlei Reportergen-Expressionskassetten erlauben vorzugsweise ein Screenen und Identifizieren von Hefezellen, die die HK des Expressionsvektors multiple und vorzugsweise mitotisch stabil in das Genom integriert haben, wie dies auch aus den Beispielen deutlich wird. Die Aufnahme einer Reportergen-Expressionskassette empfiehlt sich vor allem in den Fällen, in denen das heterologe Protein keinen für die eben genannten Zwecke ohne großen technischen, ökonomischen und/oder zeitlichen Aufwand zu screenenden Phänotyp aufweist, wie er weiter oben beschrieben wurde.

Geeignete Reportergene sind dem Fachmann allgemein bekannt, können etwa ausgewählt sein aus dem Green Fluorescent Protein-Gen, dem Yellow Fluorescent Protein-Gen und dem *amyA-*Gen kodierend für extrazelluläre α-Amylase, vorzugsweise das *amyA*-Gen für extrazelluläre α-Amylase von *B. amyloliquefaciens* (Steinborn, G., et al. 2006, J. Biotechnol, Doi:10.1016/j.jbiotec.2006.07.026). Die Vorteile des *amyA* Reportergens sind seine experimentell einfache Nachweisbarkeit aufgrund der deutlich sichtbaren Stärkeabbauhöfe sowie seine Verwendbarkeit für großtechnische Fermentationen, so dass hier ein anwendungsrelevantes, etabliertes Gen als Reporter verwendet werden kann. Bei Einsatz des *amyA*-Reportergens wurde ein relativ großer Stärkeabbauhof im Verhältnis zum Koloniedurchmesser als erster Hinweis auf eine multiple Integration gewertet, ein Verlust der Amylasebildung dagegen deutet auf mitotische Instabilität hin.

In einer bevorzugten Ausführungsform ist der dritte Promotor ein starker Promotor im Sinne der Erfindung. Der dritte Promotor kann ein induzierbarer oder konstitutiver Promotor sein, vorzugsweise ein aus *TEF1-, GAA-, ALIP-* und ATAN-Promotor ausgewählter Promotor. Grundsätzlich kann die Reportergen-Expressionskassette in dem Bereich des Expressionsvektors angeordnet sein, der von den rDNA Sequenzen AA und BB flankiert wird, wobei die Reportergen-Expressionskassette dabei vorzugsweise auf der Seite des von den Abschnitten a) und d) eingeschlossenen Teils angeordnet ist, in dem auch die Abschnitte b) und c) angeordnet sind und der vorzugsweise dem Abschnitt e) gegenüber liegt (siehe z.B. Anordnung im Vektor pGH69).

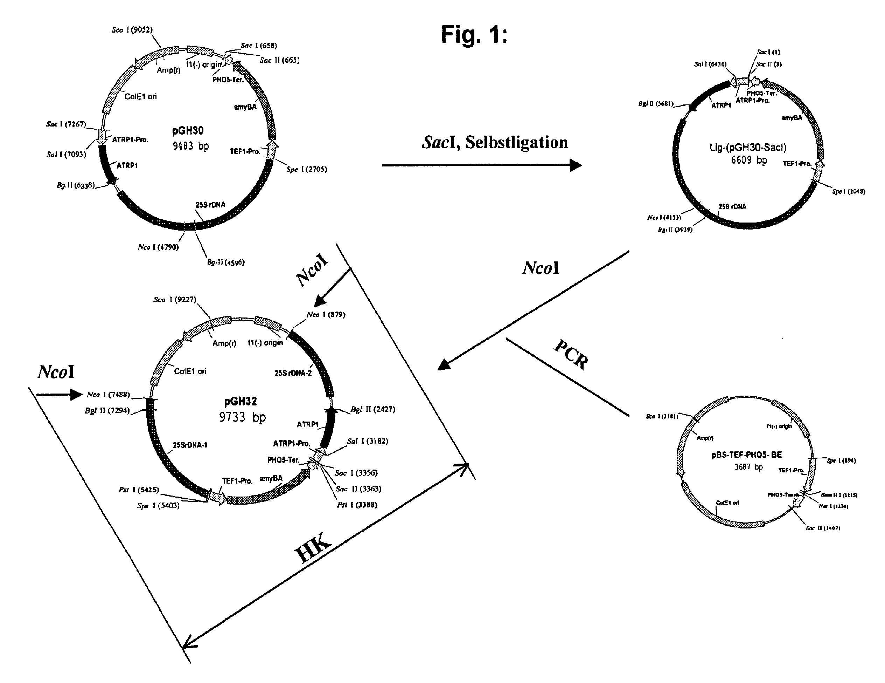

Die Reportergen-Expressionskassette kann dabei in gleicher oder entgegengesetzter Orientierung zu der Proteinexpressionskassette und/oder der Selektionsmarker-Expressionskassette liegen. Es ist bevorzugt, dass die Transkriptionskontrolle (i) der Nukleinsäure, die für das heterologe Protein kodiert und/oder (ii) des Selektionsmarkergens und/oder (iii) des Reportergens im wesentlichen unabhängig voneinander ist, d.h., dass die Frage, ob und auf welchem quantitativem Niveau die Transkription des jeweiligen Gens erfolgt, von Gen zu Gen im wesentlichen unabhängig reguliert wird. Dies kann etwa durch geeignete Wahl der Promotoren bewerkstelligt werden, z.B. indem in verschiedenen Expressionskassetten verschiedene induzierbare Promotoren eingesetzt werden, die wiederum durch unterschiedliche Signale steuerbar sind. Ebenso können unterschiedliche konstitutive oder konstitutive und induzierbare Promotoren in den verschiedenen Expressionskassetten eingesetzt werden, wie dies zum Beispiel in Figur 1 gezeigt wurde. Alternativ oder zusätzlich können am 3' Ende der Gensequenzen ein oder mehrere Terminatorsequenzen angeordnet werden, die ein "Durchlesen" des Promotors in die folgende Expressionskassette verhindern.

Vorzugsweise sollte die Promotoraktivität der Selektionsmarker-Expressionskassette (zweiter Promotor) keine Transkriptionskontrolle über die Transkription des Reportergens und/oder der für das heterologe Protein kodierenden Nukleinsäure haben. Gleiches gilt vorzugsweise für das Verhältnis des Promotors der Reportergen-Expressionskassette (dritter Promotor) vis-à-vis der Transkriptionskontrolle des Selektionsmarkergens und/oder der für das heterologe Protein. Es ist schließlich bevorzugt, dass die Promotoraktivität der Protein-Expressionskassette (erster Promotor) keine Transkriptionskontrolle über die Transkription des Selektionsmarkergens und/oder des Reportergens hat.

Besonders bevorzugte Expressionsvektoren im Sinne der Erfindung sind solche, die ausgewählt sind aus einem Vektor, enthaltend oder bestehend aus einer Nukleinsäuresequenz der SEQ ID Nr. 13; SEQ ID Nr. 14; SEQ ID Nr. 15; SEQ ID Nr. 16; SEQ ID Nr. 17 und SEQ ID Nr. 18; wobei in den Vektor eine Protein-Expressionskassette insertiert werden kann oder wurde, oder wobei die für das heterologe Protein kodierende Nukleinsäure anstelle des *amyA*-Gens insertiert werden kann oder wurde, sowie funktionale Varianten der vorgenannten Vektoren. Die Insertion der Protein-Expressionskassette kann z.B. vor oder hinter der *ATRP1*-Selektionsmarkerexpressionskassette oder alternativ, vor oder hinter der *amyA*-Expressionskassette erfolgen. Jedenfalls sollte die Protein-Expressionskassette innerhalb des von den rDNA-Sequenzen eingeschlossenen Abschnittes der HK insertiert werden, der auf der Seite des Vektors liegt, in dem auch die *ATRP1*-Selektionsmarker-Expressionskassette angeordnet ist. Erfolgt die Insertion der für das heterologe Protein kodierenden Nukleinsäure anstelle des *amyA*-Gens, so sollte die Insertion vorzugsweise funktionsfähig im gleichen Leserahmen mit dem Promotor erfolgen, der die Transkription des *amyA*-Gens kontrolliert, so dass die so insertierte kodierende Nukleinsäure unter der Transkriptionskontrolle des Promotors steht.

Überraschenderweise zeigten Wirtszellen, die den erfindungsgemäßen Expressionsvektor pGH32 (Fig. 1) enthielten, im Vergleich zu pGH30 ein weit besseres Wachstum. Als Ursache für das beschleunigte Wachstum von pGH32-Integranten kamen die unmittelbare Nachbarschaft und gleiche Transkriptionsrichtung des *ATRP1*-Selektionsmarkers zum *amyA*-Reporter-Gen in Betracht, so dass bei starker Aktivität des *TEF1*-Promotors und relativ geringerer Aktivität des *PHO5*-Terminators in den sonst schwach transkribierten *ATRP1*-Selektionmarker durchgelesen wird. Diese Annahme wurde dadurch bestätigt, dass der *ATRP1*-Selektionsmarker im Expressionsvektor pGH33 auch nach Deletion seines eigenen Promotors *ATRP1*-Pro. noch stark exprimiert wird, während die Umkehr der Transkriptionsrichtung von *amyA* in pGH32R wieder zu einer mit pGH30 vergleichbar geringen Expression des *ATRP1*-Selektionsmarkers führte. Für die Isolierung von multiplen Integranten ist bezüglich der Selektionsmarker-Expressionskassette ein Expressionsvektor mit starkem Promotor und eigenständiger Transkription bevorzugt, so dass die Promotoraktivität zur Einstellung des Selektionsdrucks in möglichst vielen Schritten graduell reduziert und so ein entsprechend breites Spektrum von Integrationsvektoren isoliert werden kann. In dem ebenfalls erfindungsgemäßen Expressionsvektor pGH32R wurde der *ATRP1-*Promotor gegen den *ALEU2*-Promotor ausgetauscht, und der so erzeugte Integrationsvektor pGH32RA ergab, wie pGH32, tatsächlich schnell wachsende Integranten mit hoher Häufigkeit und homogener Koloniegröße.

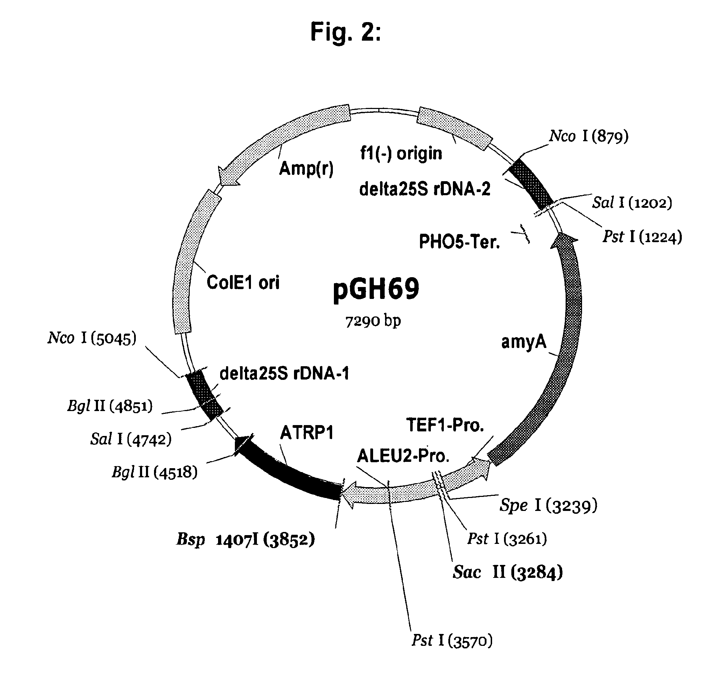

Außerdem ist es überraschenderweise vorteilhaft, die Länge des zu integrierenden DNA-Fragments weiter zu reduzieren, um dadurch die Kapazität der HK zugunsten von zusätzlichen oder anderen, größeren Ziel-Genen oder Gen-Gruppen zu erweitern. Deshalb wurden beide flankierende rDNA-Fragmente auf z.B. je ca. 300 bp reduziert und so ein weiterer bevorzugter erfindungsgemäßer Expressionsvektor pGH69 (Fig. 2) erhalten, der im Vergleich zum Ausgangsplasmid nach Transformation in *A. adeninivorans* G1212 [*aleu2 atrp1::ALEU2*] (Steinborn, G., et al. 2006: Construction of an Arxula adeninivorans host-vector system based on trp1 complementation; J. Biotechnol, Doi:10.1016/j.jbiotec.2006.07.026) eine vergleichbar hohe Integrantenhäufigkeit ergab.

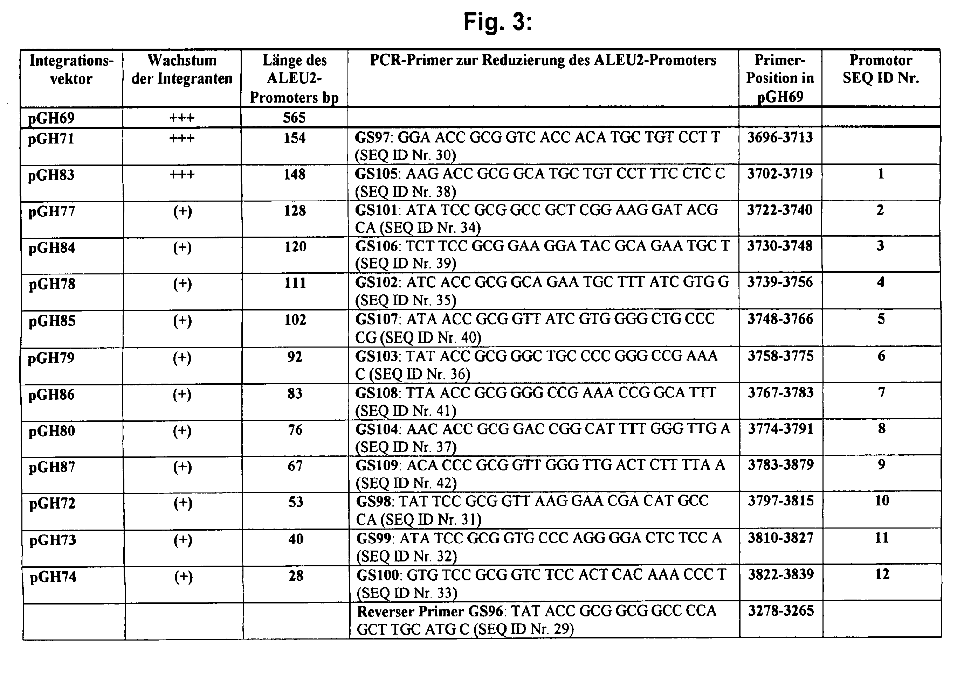

Die anschließende Konstruktion von bevorzugten Expressionsvektoren für multiple Integrationen in *A. adeninivorans* ging z.B. von pGH69 aus und sollte durch schrittweise Reduzierung der Länge (565 bp; Fig. 3) des *ALEU2*-Promotors eine geeignete Reduzierung der Promotoraktivität für den *ATRP1*-Selektionsmarker ergeben, so dass multiple Integranten von *A. adeninivorans* isolierbar sind. Vom 5'-Ende her wurde der Promotor zunächst in großen Schritten bis zu einer minimalen Länge deletiert, die noch eine starke Expression ermöglichte. Dann folgten kleinere Deletionsschritte, um das Limit der Promotorlänge einzugrenzen. Gemessen am Koloniewachstum von Integranten wurde die Expression des *ATRP1*-Selektionmarkers überraschenderweise nach Deletion auf 127 bp und auch nach weiteren Deletionen bis auf ca. 28 bp etwa in gleichem Grade drastisch reduziert, während bei Deletion auf 154 und 148 bp noch eine hohe Expression erfolgte (Fig. 3).

Tests auf ihre Eignung zur multiplen Gen-Integration erfolgten mit allen in Beispiel 3 genannten Integrationsvektoren, die aufgrund der starken Wachstumsreduktion der von ihnen erhaltenen Integranten auf eine starke Reduktion der *ALEU2*-Promotoraktivität schließen ließen. Dadurch wurde die Möglichkeit berücksichtigt, dass durch das Wachstumsverhalten der Integranten nicht messbare Unterschiede der *ALEU2-*Promotoraktivität und strukturelle Besonderheiten für die multiple Integration nutzbar sein könnten. Gemessen an der 2- bis 5-fachen Steigerung der alpha-Amylaseaktivität in Kulturüberständen wurden z.B. für den erfindungsgemäßen Expressionsvektor pGH72 bei einer Promotorlänge von 53 bp drei potentiell multiple Integranten isoliert, so dass für diesen Integrationsvektor eine Wiederholbarkeit der Ergebnisse nachgewiesen wurde. Bei Kenntnis der hier beschriebenen Verfahren ist es dem Fachmann unter Zuhilfenahme routinemäßiger Methoden und Kenntnisse ohne weiteres möglich, etwa bei Durchführung eines weiteren, ggf. hochskalierten Screenings, auch unter veränderten Screening-Bedingungen weitere multiple genomische Integrantenstämme auch von anderen erfindungsgemäßen Expressionsvektoren etwa mit vergleichbar reduzierter *ALEU2*-Promotoraktivität zu isolieren.

Sollte es gewünscht sein, die mit Hilfe der erfindungsgemäßen Expressionsvektoren hergestellten heterologen Proteine aus dem Überstand der Kultur zu isolieren, so empfiehlt es sich, dass die Protein-Expressionskassette eine Nukleinsäuresequenz aufweist, die für ein Signalpeptid kodiert. Dererlei für Signalpeptide kodierende Nukleinsäuren sind dem Fachmann allgemein bekannt und sind beispielsweise in Wolf K. 1996: Nonconventional yeasts in biotechnology. Springer Verlag, Berlin-Heidelberg-New York beschrieben. Bevorzugte Signalpeptide sind die MATα (*S. cerevisiae*), Glucoamylase (*A. adeninivorans*) bzw. Tannase (*A. adeninivorans*)*.* Geeignete Signalsequenzen und ihre Eigenschaften können den folgenden Referenzen entnommen werden (Wolf K. 1996: Nonconventional yeasts in biotechnology. Springer Verlag, Berlin-Heidelberg-New York, Bui D.M. et al. 1996, Appl. Microbiol. Biotechnol. 44: 610-619, DE 10 2004 032 216 A1).

Alternativ kann es auch wünschenswert sein, dass das hergestellte heterologe Protein in der Wirtszelle bleibt und danach durch geeignete, allgemein bekannte Verfahren geerntet und aufgereinigt wird.

Nach einer weiteren Ausführung der Erfindung kann die Protein-Expressionskassette und insbesondere die für das heterologe Protein kodierende Nukleinsäure eine Sequenz enthalten, die eine Aufreinigung, z.B. eine Affinitätschromatographie basierte Aufreinigung des exprimierten heterologen Proteins vereinfacht, etwa eine Sequenz die für ein His-Tag, GST-Tag oder ein Flag-Tag kodiert. Derlei Sequenzen sind dem Fachmann allgemein bekannt.

Ein weiterer bevorzugter Expressionsvektor ist ein Expressionsvektor, in dem die für ein heterologes Protein kodierende Nukleinsäure in den Vektor insertiert wurde. Verfahren zur gezielten Klonierung von Nukleinsäuresequenzen in einen Vektor sind allgemein bekannt und z.B. in Sambrook et al., 2001, supra, ausführlich beschrieben.

Nach einem zweiten Aspekt der Erfindung wird eine Wirtszelle der Hefegattung *Arxula* bereitgestellt, der den von den Abschnitten a) bis d) eingeschlossenen Teil eines erfindungsgemäßen Expressionsvektor enthält, wobei die für ein heterologes Protein kodierende Nukleinsäure in den Expressionsvektor insertiert wurde. Vorzugsweise liegt der Vektor multiple integriert im Genom der Wirtszelle vor und erlaubt vorzugsweise eine Expression des heterologen Proteins auf hohem quantitativem Niveau. Es ist weiterhin bevorzugt, dass die Wirtszelle und vor allem die multiple genomische Integration der HK des Expressionsvektors mitotisch stabil sind. Die durch chromosomale Integration der HK erhaltene Integrante kann zur Produktion heterologer Proteine vom Labormaßstab bis hin zum großtechnischen Maßstab eingesetzt werden. Außerdem können die Wirtszellen der Erfindung in den erfindungsgemäßen Verfahren eingesetzt werden.

Wirtszellen der Hefegattung *Arxula* sind dem Fachmann allgemein bekannt und schließen vorzugsweise *Arxula adenininvorans* und *Arxula terrestris* ein.

Vorzugsweise ist die Wirtszelle der Erfindung eine Hefezelle, die eine Auxotrophie aufweist, die mit dem Selektionsmarkergen komplementiert werden kann. Beispielhaft wird dies anhand einer Glutamin-Auxotrophie erläutert, wobei dieses Beispiel in analoger Weise bei Wahl entsprechender anderer Auxotrophien auf alternative Ausführungsformen der Erfindung übertragbar ist. Wenn die Wirtszelle daher etwa auxotroph in Bezug auf eine bestimmte Aminosäure wie Glutamin ist, weil ein für ein Enzym des Syntheseweges von Glutamin kodierendes Gen deletiert wurde, so könnte das Selektionsmarkergen z.B. das native in der Wirtszelle deletierte Gen sein, um so durch seine Expression den Gendefekt der Wirtszelle auszugleichen und die Synthese von Glutamin zu ermöglichen. Im Ergebnis wird so durch multiple Integration geeigneter Expressionsvektoren eine Prototrophie bezüglich von Glutamin erzielt, die zur Selektion von multiplen Integranten genutzt werden kann. Wie bereits an anderer Stelle erläutert und in den Beispielen illustriert, kann durch Einstellen des Niveaus der Expression des Selektionsmarkers, z.B. durch Wahl geeigneter nativer oder modifizierter Promotoren der Selektionsmarker-Expressionskassette (zweiter Promotoren) und/oder durch die Einstellung der Konzentration des Moleküls, für das die Wirtszelle eine Auxotrophie aufweist, der Selektionsdruck auf die kultivierten Hefezellen, einen gewissen Amplifikationsgrad multipler Integration der HK des Expressionsvektors als Voraussetzung für Wachstum aufzuweisen, bestimmt werden. Dieses Verfahren ermöglicht eine gezielte Entwicklung von erfindungsgemäßen Wirtszellen und ist in den Beispielen belegt worden.

Vorzugsweise weist die Hefezelle eine Aminosäure-Auxtrophie auf, insbesondere eine Tryptophan-, Leucin- bzw. lsoleucin-Auxotrophie. Derartige Zellen und ihre Eigenschaften können den folgenden Dokumenten entnommen werden (Steinborn, G., et al. 2006, J. Biotechnol, Doi:10.1016/j.jbiotec.2006.07.026; Samsonova I. et al. 1996, Yeast 12, 1209-1217). Eine bevorzugter Wirtsstamm mit Tryptophan-Auxotrophie ist *A. adeninivorans* G1212 [*aleu2 atrp1::ALEU2*]*.*

In einem dritten Aspekt der Erfindung wird ein Kit bereitgestellt, das einen erfindungsgemäßen Expressionsvektor, eine Anleitung zur Benutzung und optional eine Wirtszelle der Hefegattung *Arxula* enthält, wobei vorzugsweise die für das heterologe Protein kodierende Nukleinsäure entweder bereits in den Expressionsvektor kloniert wurde oder nicht.

In einer bevorzugten Ausführungsform der Erfindung ist die in dem Kit enthaltene Wirtszelle eine Hefezelle, die eine oben beschriebene Auxotrophie aufweist, die mit dem Selektionsmarkergen des Expressionsvektors komplementiert werden kann.

In einem vierten Aspekt der Erfindung wird ein Kit bereitgestellt, dass (i) eine erfindungsgemäße Wirtszelle mit multiple genomisch integriertem Vektor, die den von den Abschnitten a) bis d) eingeschlossenen Teil, der HK, des Expressionsvektors enthält, einschließlich einer für das heterologe Protein kodierende Nukleinsäure sowie (ii) eine Anleitung zur Benutzung umfasst. Vorzugsweise weist die Wirtszelle eine oben beschriebene Auxotrophie auf, die mit dem Selektionsmarkergen des Expressionsvektors komplementiert werden kann.

Es ist möglich, dass die Kits der Erfindung weitere Bestandteile enthalten, die für die Durchführung eines erfindungsgemäßen Verfahrens nützlich sind, wie Kulturmedien, Puffer und/oder Komponenten zur Durchführung von Kontrollversuchen.

Die erfindungsgemäßen Kits können zur Produktion von heterologen Proteinen vorzugsweise nach einem erfindungsgemäßen Verfahren eingesetzt werden und erlauben insbesondere eine Produktion heterologer Proteine auf hohem quantitativem Expressionsniveau, wobei vorzugsweise auch eine hohe mitotische Stabilität der genomischen Integration bei den generierten und selektierten lntegrantenstämmen sowie vorteilhafterweise eine hohe biologische Sicherheit hinzutreten.

In einem fünften Aspekt der Erfindung wird ein Verfahren zur Expression eines heterologen Proteins in eine Wirtszelle der Hefegattung *Arxula* bereitgestellt, umfassend:
1) Klonieren der Nukleinsäure, die für das heterologes Protein kodiert, in einen erfindungsgemäßen Expressionsvektor;
2) Einführen des von den Abschnitten a) bis d) eingeschlossenen Teils des in 1) erhaltenen Expressionsvektors, gegebenenfalls in linearisierter Form, in eine zur Expression des heterologen Proteins geeignete Wirtszelle der Hefegattung *Arxula,* die vorzugsweise auch zur Expression des Selektionsmarkers und/oder des Reportergens geeignet ist;
3) Kultivieren der in 2) erhaltenen Wirtszelle unter Bedingungen, die eine Expression des Selektionsmarkers sowie des heterologen Proteins erlauben;
4) Isolieren einer den Selektionsmarker sowie das heterologe Protein exprimierenden Wirtszelle (Integrante); und
5) Rekultivieren der in Schritt 4) isolierten Wirtszelle unter Bedingungen, die eine Expression des heterologen Proteins erlauben; und
6) Ernten des heterologen Proteins.

Verfahren zur Klonierung von Nukleinsäuren in einen Expressionsvektor sind dem Fachmann beispielsweise aus Sambrook et al., 2001, supra bekannt. Auch die Bedingungen der Kultivierung, die eine Expression des Selektionsmarkers, des heterologen Proteins bzw. des Reportergens erlauben, können vom Fachmann in Kenntnis der vorliegenden Anleitung im Rahmen routinemäßiger Maßnahmen ermittelt werden und hängen unter anderem von der Konstruktion der jeweiligen Expressionskassette ab, insbesondere von dem ausgewählten Promotor und der verwendeten Wirtszelle.

Der Begriff "Isolieren einer den Selektionsmarker sowie das heterologe Protein exprimierenden Zelle" im Sinne der Erfindung schließt neben dem Vorgang des physikalischen Abtrennens der Zelle aus ihrer Kultur vorzugsweise auch die Selektion geeigneter Zellen auf der Grundlage ihres Phänotyps ein. Bei dem Phänotyp ist hierbei vorzugsweise eine qualitative und/oder quantitative Bewertung der Expression des Selektionsmarkers, des Reportergens und/oder des heterologen Proteins durchzuführen, um geeignete Wirtszellen auszuwählen, d.h. Wirtszellen, die eine multiple genomische Integration des Expressionsvektors aufweisen, vorzugsweise verbunden mit hoher Expression des heterologen Proteins und weiterhin bevorzugt mitotische Stabilität der genomischen Integration in den Wirtszellen. Die erforderlichen Selektionskriterien zur Auswahl geeigneter zu isolierender Integranten können hierzu auf der Grundlage der hier bereitgestellten Anleitung und den detaillierten Beispielen von einem Fachmann im Rahmen routinemäßigen Arbeitens bestimmt werden.

Nach einer bevorzugten Ausführungsform der Erfindung können die Schritte 3) und 4) des Verfahrens ein- oder mehrfach wiederholt werden, um eine noch zuverlässigere Isolierung mitotisch stabiler Integrantenstämme zu ermöglichen.

Es ist weiterhin möglich, das Verfahren nach jedem Schritt zu unterbrechen und es zu einem späteren Zeitpunkt fortzuführen. Insbesondere im Anschluss an Schritt 4) können die isolierten Wirtszellen z.B. lyophilisiert oder tiefgefroren, gelagert, verschifft, verkauft, etc. werden, bevor die weiteren Verfahrensschritte 5) und 6) durchgeführt werden. Dem Fachmann sind die hierzu erforderlichen Maßnahmen bekannt, mit denen gewährleistet wird, dass die Eigenschaften der Expressionsvektoren und/oder Wirtszellen durch Unterbrechen und/oder Lagerung nicht signifikant beeinträchtigt werden.

Ebenso ist es allgemein bekannt, wie das verfahrensgemäß hergestellte heterologe Protein geerntet werden kann. Beispielsweise können die heterologen Proteine nach Zentrifugation über eine lmmunaffinitätschromatographie, wie unter Einsatz spezifisch das heterologe Protein bindender Antikörper, isoliert und so aus der Kultur in im wesentlichen reiner Form abgetrennt werden. Für den bevorzugten Fall, dass das heterologe Protein mit einem vorgenannten Tag, wie einem His-Tag versehen ist, kann durch geeignete Auswahl und Ausstattung der lmmunaffinitätschromatographiesäule mit selektiven Bindungspartnem für die Tags ebenfalls eine spezifische Isolierung der erfindungsgemäßen heterologen Proteine nach allgemein bekannten Verfahren erzielt werden.

Vorzugsweise wird in dem erfindungsgemäßen Verfahren eine Wirtszelle eingesetzt, die ausgewählt ist aus einem der vorgenannten Hefen der Gattung *Arxula;* insbesondere bevorzugt ist eine Wirtszelle von *A. adaninivorans* und *A. terrestris.*

Es ist ferner bevorzugt, dass die Hefezelle eine oben beschriebene Auxotrophie aufweist, die mit dem Selektionsmarkergen komplementiert werden kann, wie dies detailliert weiter oben beschrieben wurde.

Nach einer weiteren Ausführungsform des Verfahrens umfasst der Expressionsvektor ferner eine Reportergen-Expressionskassette, die einen vorzugsweise aus der Hefegattung *Arxula* stammenden oben definierten konstitutiven oder induzierbaren dritten Promotor enthält, sowie ein bereits beschriebenes Reportergen, das unter der Transkriptionskontrolle des Promotors steht, wobei die Reportergen-Expressionskassette in dem Sequenzabschnitt angeordnet ist, der von den rDNA Sequenzen AA und BB flankiert wird und in dem auch die Abschnitte b) und c) liegen; wobei in Schritt 2) die Wirtszelle unter Bedingungen kultiviert wird, die eine Expression des Selektionsmarkers sowie des Reportergens und gegebenenfalls auch des heterologen Proteins erlauben und in Schritt 4) eine Wirtszelle isoliert wird, die den Selektionsmarker sowie das Reportergen und gegebenenfalls auch das heterologe Protein exprimiert.

Bevorzugte Reportergene, die für ein erfindungsgemäßes Verfahren eingesetzt werden können, wurden bereits weiter oben beschrieben.

Übliche Materialien und Methoden zur Durchführung der erfindungsgemäßen Verfahren sowie der Herstellung und der Anwendung der erfindungsgemäßen Gegenstände sind allgemein bekannt. Kenntnisse zur Kultivierung von Bakterien und Hefen, zur Stammselektion, Isolierung von chromosomaler DNA, Plasmid-DNA und DNA-Fragmenten, sowie zur Restriktionsendonuklease-Spaltung von DNA, Ligation von DNA-Fragmenten, Plasmidtransformation, Elektrophorese, Southern-Hybridisierung und DNA-Sequenzanalyse gehören zu diesem allgemein bekannten molekularbiologischen Techniken und sind aus Publikationen wie z.B. Sambrook, et al., 2001, supra, allgemein zugänglich. Bei Nutzung von käuflichen Kits, Enzymen und anderen Materialien wird nach den Protokollen der Hersteller oder Lieferfirmen verfahren.

Beispielhaft können im Rahmen der vorliegenden Erfindung folgende Materialien und Methoden erfindungsgemäß verwendet werden:
Plasmid-DNA-Isolierungen aus Stämmen von *Escherichia coli* erfolgen z.B. in großem Maßstab z.B. mittels QIAGEN Plasmid-Kit, während sie in kleinem Maßstab (Minipräps) nach einer alkalischen Methode (Sambrook et al. 2001, supra) durchgeführt werden können.
Die Isolierung von DNA-Fragmenten aus Agarosegelen kann etwa mittels QIAEX II-Kit von QIAGEN ausgeführt werden, während die Plasmidtransformation in kompetente Zellen von *E. coli* nach Sambrook et al., 2001, supra möglich ist.

Eine Transformation von Plasmiden oder Expressionsvektoren bzw. der HK in Hefen kann nach Dohmen et al. (Dohmen , R. J., et al. 1991, Yeast 7, 691-692) durchgeführt werden. Die Transformation der Expressionsvektoren in die Wirtszelle kann in zirkulärer oder vorzugsweise in linearisierter Form nach allgemein bekannten Verfahren erfolgen.

Die Selektion auf plasmidkodierte Ampicillin-Resistenz kann mittels Zusatz von 75 µg/ml Ampicillin (Roth) erfolgen.

Eine Kultivierung von Hefezellen kann z.B. bei 30 °C in YPD-Vollmedium (Fluka) oder in einem Hefe-Minimalmedium (HMM; Tanaka, A., et al. 1967, J. Ferment. Technol. 45, 617-623) ausgeführt werden. Vorkulturen (z.B. 10 ml) können in 50 ml-Kulturflaschen auf Certomat-HK-Schüttler, 160 rpm erfolgen. Kulturen von z.B. 50 ml können wiederum in 250 ml-Erlenmeyer-Kolben auf einem Rotationsschüttelwasserbad (Typ 1092, GFL), 160 rpm, umgesetzt 1:50 bis 1:100 aus den Vorkulturen gewonnen und kultiviert werden. Gegebenenfalls erfolgt eine Supplementierung von 2% Glukose (für *Arxula*) oder 2% Fruktose (für *Sacharomyces*) als C-Quelle und, wenn erforderlich, je 50 µg/ml essentielle Aminosäuren und Uracil.

Die Messungen der Zelldichte können mittels Klett-Colorimeter für Minimalmedium bei 400-450 nm (Blaufilter), für Vollmedium bei 520-580 nm (Grünfilter) ausgeführt werden.

Die Ermittlung der α-Amylase-Aktivität in Kulturüberständen kann durch Bestimmung der Konzentration von enzymatisch erzeugtem reduzierendem Zucker mittels Dinitrosalicylat erfolgen (Büttner, R, et al. 1990, Acta Biotechnol. 10, 361-370).

Southernhybridisierung können nach einer früher beschriebenen Verfahrensweise durchgeführt werden (Wartmann, T., et al. 2002, FEMS Yeast Res. 2, 363-369).

Die DNA-Sequenzierung kann an einem automatischen Fluoreszenz-DNA-Sequenzierer (Pharmacia, Schweden) erfolgen.

In einem sechsten Aspekt der Erfindung wird ein Verfahren zur Expression eines heterologen Proteins in einer Wirtszelle der Hefegattung *Arxula* bereitgestellt umfassend:
1) Kultivieren einer nach einem vorstehend beschriebenen Verfahren isolierten Wirtszelle oder einer erfindungemäßen Wirtszelle (Integrante), die vorzugsweise einen erfindungsgemäße HK einschließlich eines darin enthaltenen Gens kodierend für ein heterologes Protein, multiple in ihr Genom integriert hat, unter Bedingungen, die eine Expression des heterologen Proteins erlauben; und
2) Ernten des heterologen Proteins.

In einer weiteren Ausführungsform der erfindungsgemäßen Verfahren wird das geerntete heterologe Protein gereinigt. Verfahren zur Reinigung von Proteinen sind dem Fachmann allgemein geläufig.

Die vorteilhaften Eigenschaften des Verfahrens werden aus den Beispielen deutlich. Das erfindungsgemäße Verfahren erlaubt die Herstellung einer weiten Palette heterologer Proteine in einem hohem quantitativem Niveau und macht sich dabei die bereits beschriebenen Vorteile der mitotisch stabilen, reproduzierbaren und multiplen genomischen Integration der HK der Expressionsvektoren zu Nutze.

Ferner beschrieben wird eine pharmazeutische Zusammensetzung die ein nach einem vorstehend beschriebenen Verfahren hergestelltes heterologes Proteins enthält und zusätzlich gegebenenfalls dem Fachmann geläufige, pharmazeutisch verträgliche Hilfs- und Zusatzstoffe enthält. Beispielsweise handelt es sich bei dem heterologen Protein um einen medizinischen Wirkstoff wie etwa Insulin, EPO, einen Wachstumsfaktor oder ein Zytokin. Der medizinische Einsatz der pharmazeutischen Zusammensetzung wird von der Natur des hergestellten heterologen Proteins abhängen und ist allgemein bekannt.

In einem weiteren Aspekt bezieht sich die Erfindung auf die Verwendung eines erfindungsgemäßen Expressionsvektors, einer erfindungsgemäßen Wirtszelle oder eines erfindungsgemäßen Kits zur Herstellung eines heterologen Proteins.

In einem weiteren Aspekt bezieht sich die Erfindung auf ein Verfahren zur Herstellung einer Wirtszelle (Integrante) der Gattung Arxula, die zur Expression eines heterologen Proteins geeignet ist, umfassend:
1) Klonieren der Nukleinsäure, die für das heterologes Protein kodiert, in einen erfindungsgemäßen Expressionsvektor;
2) Einführen des von den Abschnitten a) bis d) eingeschlossenen Teils, der HK, des in 1) erhaltenen Expressionsvektors, gegebenenfalls in linearisierter Form, in eine zur Expression des heterologen Proteins geeignete Wirtszelle der Gattung *Arxula,* die vorzugsweise auch zur Expression des Selektionsmarkers und/oder des Reportergens geeignet ist;
3) Kultivieren der in 2) erhaltenen Wirtszelle unter Bedingungen, die eine Expression des Selektionsmarkers sowie des heterologen Proteins erlauben; und
4) Isolieren einer den Selektionsmarker sowie das heterologe Protein exprimierenden Wirtszelle (Integrante), die den von den Abschnitten a) bis d) eingeschlossenen Teil des Expressionsvektors multiple und mitotisch stabil in ihr Genom integriert hat.

Bevorzugt ist ein Verfahren, bei dem der Expressionsvektor ferner eine Reportgen-Expressionskassette enthält und das Kultivieren in Schritt 3) unter Bedingungen erfolgt, die eine Expression des Selektionsmarkers sowie des Reportergens erlauben. Die einzelnen Verfahrensschritte und bevorzugte Ausführungsformen dieser Schritte wurden bereits weiter oben ausführlich beschrieben. Geeignete Wirtszellen der Gattung *Arxula* wurden ebenfalls bereits beschrieben und schließen insbesondere jene Wirtszellen ein, die eine oben spezifizierte Auxotrophie aufweisen. Das Verfahren erlaubt überraschenderweise die Herstellung von Hefezellen, die eine multiple genomische Integration der erfindungsgemäßen HK bei vorzugsweise mitotischer Stabilität der hergestellten Wirtszellen (Integranten) aufweisen. Diese Wirtszellen sind bevorzugt zur Expression der für das heterologe Protein kodierenden Nukleinsäure geeignet, die insbesondere ein hohes quantitatives Niveau der Expression des heterologen Proteins auszeichnet.

Unter "einer Wirtszelle der Gattung *Arxula,* die zur Expression eines heterologen Proteins geeignet ist", wird im Sinne der Erfindung ein Hefezelle verstanden, der im Bezug auf den gewählten erfindungsgemäßen Expressionsvektor eine Selektion von Wirtszellen (Integranten) auf der Basis des Selektionssystems erlaubt, die zu dem Selektionsmarkergen des Expressionsvektors korrespondiert. Wenn der Expressionsvektor daher z.B. ein *ATRP1-*Selektionsmarkergen enthält, so weist die Wirtszelle eine Tryptophan-Auxotrophie auf. Ferner ist es erforderlich, dass die Wirtszelle die Transkriptionskontrolle durch den ersten Promotor der Protein-Expressionskassette unterstützt, d.h. dass der erste Promotor des Expressionsvektors kompatibel ist mit der ausgewählten Hefezelle und daher seine Promotoraktivität zur Transkription des für das heterologe Protein kodierenden Gens entfalten kann. Die Auswahl geeigneter Hefezellen und Expressionsvektoren kann der Fachmann in Kenntnis der vorliegenden Anleitung und den Beispielen mit Routineversuchen bestimmen.

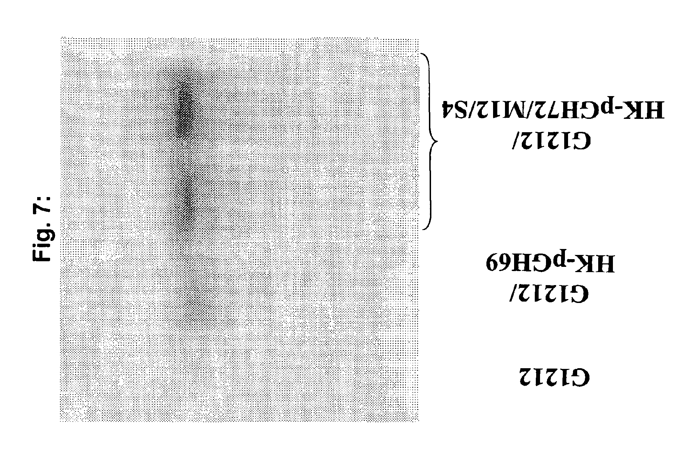

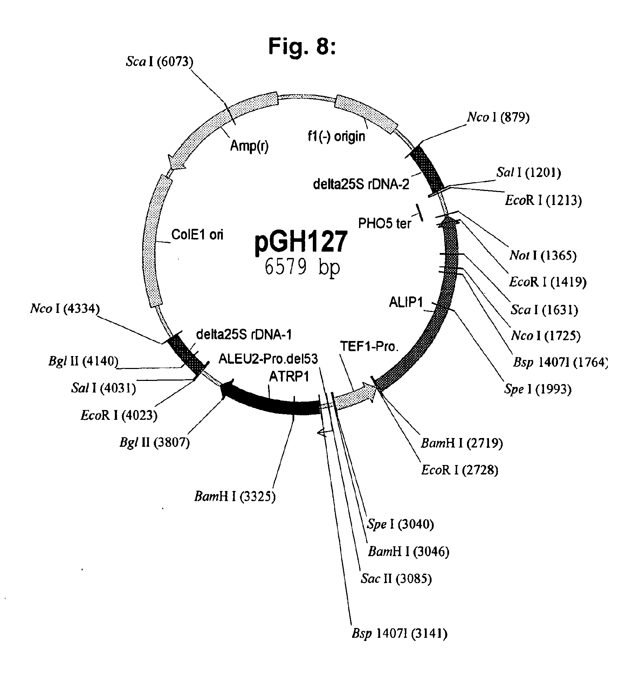

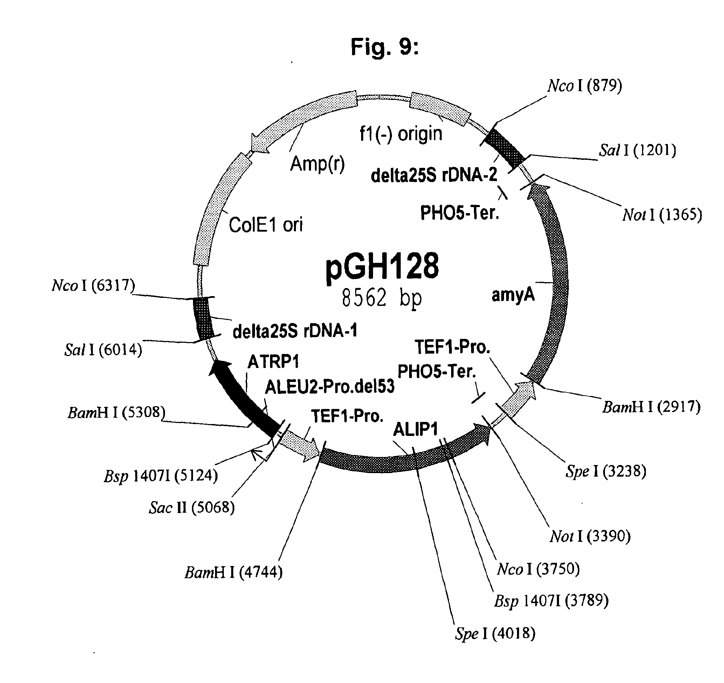

Als erfindungsgemäße multiple Integrante wurde G1212/HK_pGH72/M12/S4 eingehender charakterisiert, die durch Einsatz des Vektors pGH72 erzeugt wurde und die repräsentativ für mitotisch stabile multiple erfindungsgemäße Integranten mit hoher Steigerung der alpha-Amylaseaktivität steht. Bei der Kultivierung in Flüssigmedium war auch unter nicht-selektiven Bedingungen eine hohe mitotische Stabilität (Fig. 8) und eine ca. 5-fache Steigerung der alpha-Amylasebildung messbar (Fig. 7). Die hohe mitotische Stabilität wurde dadurch bestätigt, dass die gleiche Zellpopulation auch nach 4 Passagen (entsprechend 50 Generationen) unter nicht-selektiven Bedingungen noch die gleiche Steigerung der alpha-Amylasebildung zeigte. Durch Southernhybridisierung wurde schließlich gezeigt, dass die Steigerung der alpha-Amylasebildung tatsächlich auf multiple Integration zurückgeht und die Steigerung der alpha-Amylasebildung dem Amplifikationsgrad der HK entspricht (Fig. 9).

Die folgenden Figuren und Beispiele erläutern die Erfindung.

### Figuren und Sequenzen

**Fig. 1****:** zeigt die Konstruktion des Expressionsvektors pGH32 (SEQ ID Nr. 13) mit einer separat integrierenden, bevorzugten Hefe-Integrations-Expressions-Kassette (=HK), die durch Ncol-Restriktionsschnittstellen von der Plasmid-DNA (Plasmid-Amplifikationskassette , enthaltend f1(-) origin - Amp(r) - ColE1 ori) abtrennbar ist. Bei pGH32 entspricht die 25S rDNA 1 einer bevorzugten rDNA Sequenz AA, die 25S rDNA2 einer bevorzugten rDNA Sequenz BB. Die *TEF1-*Pro*. - amyA - PHO5*-Ter.-Kassette des pGH32 ist eine bevorzugte Reportergen-Expressionskassette bzw. eine bevorzugte Protein-Expressionskassette. Die *ATRP1*-Pro. - ATRP1-Kassette des pGH32 ist eine bevorzugte Selektionsmarker-Expressionskassette.
**Fig. 2****:** zeigt den Expressionsvektor pGH69 (SEQ ID Nr. 15) mit einem im Vergleich zu pGH32 invertierten *amyA*-Reporter-Gen, einem *ALEU2*-Promotor Transkriptionsgesteuertem *ATRP1*-Selektionsmarker und in der Länge reduzierten 25S rDNA-Teilfragmenten. Die delta25S rDNA-1 entspricht einer bevorzugten rDNA Sequenz AA, die delta25S rDNA-2 einer bevorzugten rDNA Sequenz BB. Die *TEF1-Pro. - amyA - PHO5-*Ter.-Kassette ist eine bevorzugte Reportergen-Expressionskassette bzw. eine bevorzugte Protein-Expressionskassette. Die *ALEU2*-Pro. - *ATRP1*-Kassette ist eine bevorzugte Selektionsmarker-Expressionskassette. Anstelle des *amyA*-Gens kann eine für ein heterologes Protein kodierende Nukleinsäure eingesetzt werden. Alternativ kann in den Vektor auch eine Proteinexpressionskassette sowie optional eine für ein heterologes Protein kodierende Nukleinsäure insertiert werden.
**Fig.3****:** ist eine Tabelle mit den durch PCR Reduktion von pGH69 abgeleiteten Integrationsvektoren mit unterschiedlich reduziertem *ALEU2*-Promotor des *ATRP1-*Selektionsmarkers. Das Wachstum der von ihnen erzeugten Integranten in selektivem HMM-Medium wurde bestimmt. Angegeben sind auch Länge des *ALEU2*-Promotors, die für die Promotorreduktion verwendeten Primer und ihre Positionen gemäß dem als Template (Ausgangssequenz) verwendeten Expressionsvektor pGH69.

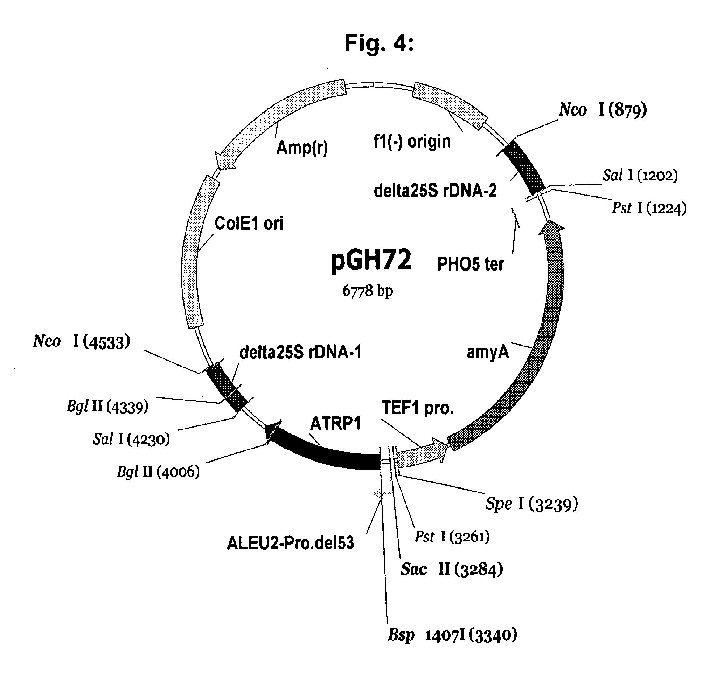

**Fig.4****:** zeigt den Integrationsvektor pGH72 mit einem auf 53 bp reduzierten *ALEU2* Promotor für multiple Integrationen, *ALEU2*-Pro.del53. Die delta25S rDNA-1 entspricht einer bevorzugten rDNA Sequenz AA, die delta25S rDNA-2 einer bevorzugten rDNA Sequenz BB. Die *TEF1-Pro. - amyA - PHO5*-Ter.-Kassette ist eine bevorzugte Reportergen-Expressionskassette bzw. eine bevorzugte Protein-Expressionskassette. Die *ALEU2-*Pro.del53 - ATRP1-Kassette ist eine bevorzugte Selektionsmarker-Expressionskassette.
Anstelle des *amyA*-Gens kann eine für ein heterologes Protein kodierende Nukleinsäure eingesetzt werden. Alternativ kann in den Vektor auch eine Proteinexpressionskassette sowie optional eine für ein heterologes Protein kodierende Nukleinsäure insertiert werden.

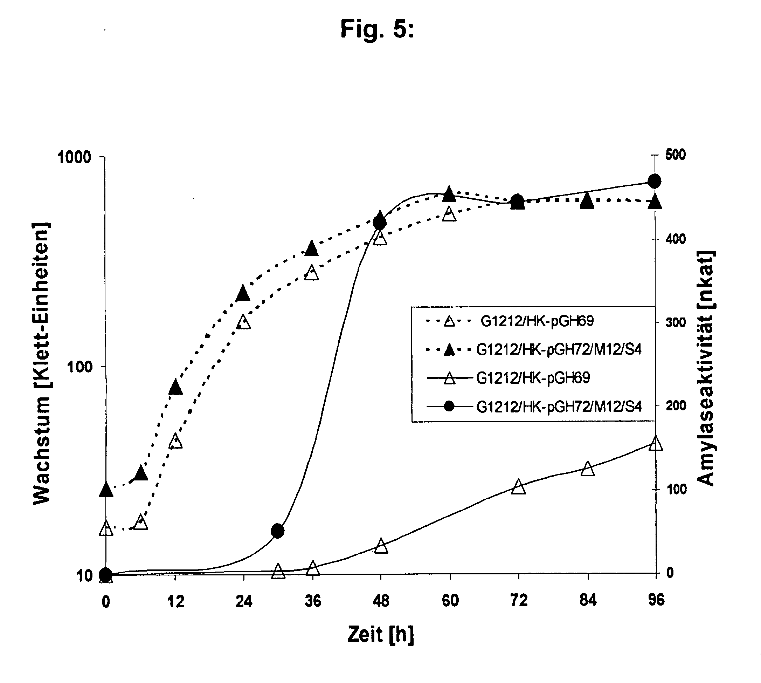

**Fig. 5****:** zeigt alpha-Amylasebildung (durchgezogene Linien) und Wachstum (gestrichelte Linien) bei der multiplen Integrante G1212/HK-72/M12/S4 und der singulären Integrante G1212/HK-pGH69 in nicht-selektivem HMM-Flüssigmedium.

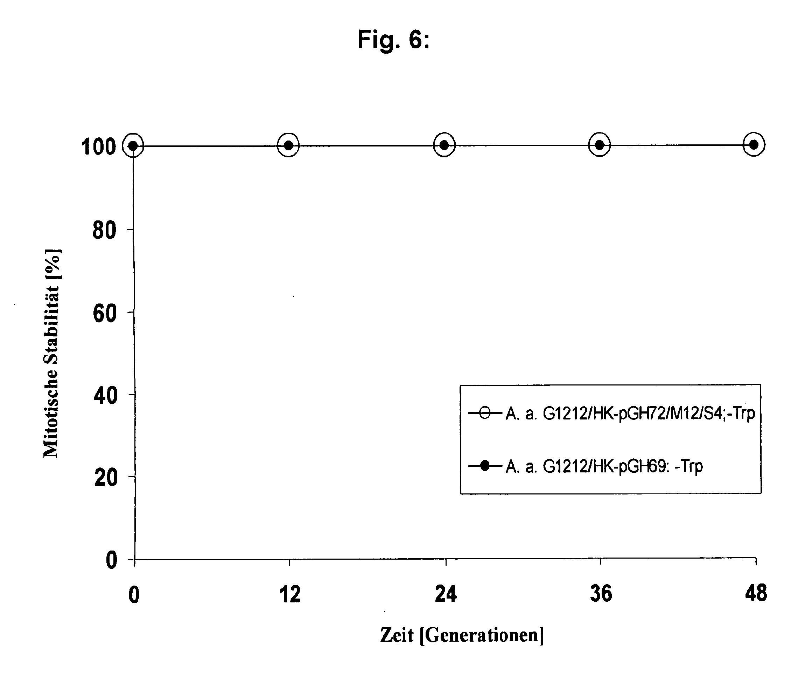

**Fig. 6****:** zeigt die mitotische Stabilität der multiplen Integrante G1212/HK-pGH72/M12/S4 und der singulären Integrante G1212/HK-pGH69. Beide Linien liegen über die gesamte gemessene Zeit bei einem Wert von 100%.
**Fig. 7****:** zeigt den Nachweis der multiplen Integration durch Southern-Hybridisierung. Die Stärke der Signale weist für die multiple Integrante G1212/HK-pGH72/M12/S4 im Vergleich zu der singulären Integrante G1212/HK-pGH69 eine ca. 5-fache Amplifikation nach.
**Fig. 8****:** zeigt den Integrationsvektor pGH127 zur multiplen Integration des homologen *ALIP1-*Gens. Anstelle des *ALIP1-*Gens kann eine für ein heterologes Protein kodierende Nukleinsäure eingesetzt werden. Alternativ kann in den Vektor auch eine Proteinexpressionskassette sowie optional eine für ein heterologes Protein kodierende Nukleinsäure insertiert werden.
**Fig. 9****:** zeigt den Integrationsvektor pGH128 zur multiplen Integration des homologen *ALIP1-*Gens mit dem heterologen *amyA*-Gen. Anstelle des *amyA*-Gens kann jede beliebige für ein heterologes Protein kodierende Nukleinsäure eingesetzt werden. Alternativ kann in den pGH128 Vektor auch zusätzlich eine Proteinexpressionskassette sowie optional eine für ein heterologes Protein kodierende Nukleinsäure insertiert werden.

**Tabelle 1: Sequenzen und zugehörige SEQ ID Nummern**

| **Sequenz** | **SEQ ID Nr.** |
|---|---|
| Modified ALEU2-Promotor (Figur 3) | 1 |
| Modified ALEU2-Promotor (Figur 3) | 2 |
| Modified ALEU2-Promotor (Figur 3) | 3 |
| Modified ALEU2-Promotor (Figur 3) | 4 |
| Modified ALEU2-Promotor (Figur 3) | 5 |
| Modified ALEU2-Promotor (Figur 3) | 6 |
| Modified ALEU2-Promotor (Figur 3) | 7 |
| Modified ALEU2-Promotor (Figur 3) | 8 |
| Modified ALEU2-Promotor (Figur 3) | 9 |
| Modified ALEU2-Promotor (Figur 3) | 10 |
| Modified ALEU2-Promotor (Figur 3) | 11 |
| Modified ALEU2-Promotor (Figur 3) | 12 |
| pGH32 | 13 |
| pGH32RA | 14 |
| pGH69 | 15 |
| pGH72 | 16 |
| pGH127 | 17 |
| pGH128 | 18 |
| Primer GS76 | 19 |
| Primer GS77 | 20 |
| Primer GS78 | 21 |
| Primer GS79 | 22 |
| Primer GS94 | 23 |
| Primer GS95 | 24 |
| Primer GS88 | 25 |
| Primer GS91 | 26 |
| Primer GS89 | 27 |
| Primer GS90 | 28 |
| Primer GS96 (Figur 3) | 29 |
| Primer GS97 (Figur 3) | 30 |
| Primer GS98 (Figur 3) | 31 |
| Primer GS99 (Figur 3) | 32 |
| Primer GS100 (Figur 3) | 33 |
| Primer GS101 (Figur 3) | 34 |
| Primer GS102 (Figur 3) | 35 |
| Primer GS103 (Figur 3) | 36 |
| Primer GS104 (Figur 3) | 37 |
| Primer GS105 (Figur 3) | 38 |
| Primer GS106 (Figur 3) | 39 |
| Primer GS107 (Figur 3) | 40 |
| Primer GS108 (Figur 3) | 41 |
| Primer GS109 (Figur 3) | 42 |

### Ausführungsbeispiele

### Beispiel 1

### Expressionsvektor pGH32 mit einer separat integrierenden "Hefe-Integrations-Expressions-Kassette" (=HK)

Zur Konstruktion eines Expressionsvektors pGH32 wurde ein Plasmid pGH30 (=pAL-ATRP1-AMY; Steinborn, G., et al. 2006: Constuction of an Arxula adeninivorans host-vector system based on trp1 complementation; J. Biotechnol, Doi:10.1016/j.jbiotec.2006.07.026) mittels *Sac*I gespalten und das 6609 bp-*Sac*I-Fragment "25S rDNA-(*ATRP1*-Pro.)-*ATRP1-TEF1*-Pro.-*amyA*-*PHO5*-Ter." (ohne *E. coli*-Plasmid-DNA) isoliert. Dieses wurde selbstligiert, isoliert und mittels *Nco*I linearisiert. pGH32 mit flankierenden 25S rDNA-Teilfragmenten wurde erhalten, indem das 6609 bp-Ncol-Fragment als HK mit dem *E.-coli*-Plasmidreplikon eines Plasmidvektors pBS-TEF-PHO5-BE ligiert wurde, nachdem dieser mittels PCR mit *Nco*I-Restriktionsschnittstellen flankiert wurde (Fig. 1). Für die PCR wurden die Primer GS76 (5'-CGA TCC ATG GCT ATA GTG AGT CGT ATT, Position 876-850; SEQ ID Nr. 19) und GS77 (5' CGA TCC ATG GCG AGC TTG GCG TAA TCA T, Position 1446-146399; SEQ ID Nr. 20) eingesetzt.

Im Ergebnis wurde mit pGH32 der SEQ ID Nr. 13 ein erfindungsgemäßer Expressionsvektor erhalten, der eine separat integrierende Hefe-Integrations-Expressions-Kassette umfasst (Fig. 1). Bestandteile dieser Kassette sind homologe 25S rDNA-Fragmente zur Adressierung der chromosomalen Integration, ein heterologes *amyA*-Gen für extrazelluläre α-Amylase von *B. amyloliquefaciens* als Reporter-Gen zum Nachweis von Integranten, ihrer mitotischen Stabilität und der Kopienzahl sowie ein Aminosäure-Auxotrophiemarker *ATRP1* als Selektionsmarker für die Isolierung von Integranten. Die Vorteile des *amyA*-Gens sind die experimentell wenig aufwendige Nachweisbarkeit des davon abgeleiteten Genproduktes (alpha-Amylase) durch visuell deutliche Stärkeabbauhöfe sowie seine schon langzeitige Verwendung für großtechnische Fermentationen, so dass hier ein anwendungsrelevantes Gen als Reporter verwendet wird. Ein relativ großer Stärkeabbauhof im Verhältnis zum Koloniedurchmesser wurde als erster Hinweis auf eine mögliche multiple Integration gewertet, Verlust der alpha-Amylasebildung dagegen als mitotische Instabilität. Die Trp-Auxotrophie wurde als ein vorteilhafter Selektionsmarker ausgewählt, da Tryptophan durch Autoklavieren inaktiviert wird und deshalb in sterilisierten Vollmedien ein selektiver Druck für Trp-auxotrophe Rezipientenhefezellen besteht.

Zur Integration wurde pGH32 mittels *Nco*I gespalten und das Fragmentgemisch oder nach Isolierung des 6609 bp-Ncol-Fragmentes nur diese HK in den Tryptophan (=Trp)-auxotrophen Rezipientenstamm *A. adeninivorans* G1212 [*atrp1*] transformiert. Nach Transformation von pGH32 wurden pro Versuch etwa 350 Integranten G1211/HK_pGH32 erhalten und damit im Vergleich zum Ausgangsplasmid pGH30 eine ca. 5-fache gesteigerte Integrantenhäufigkeit erzielt. Überraschenderweise zeigten Integranten von pGH32 im Vergleich zu pGH30 ein deutlich stärkeres Wachstum und eine homogene Koloniegröße. Die HK des pGH32 wurde also in adressierende rDNA inseriert und so konnte je ein Teilfragment der rDNA ohne Änderung ihrer ursprünglichen Orientierung die HK flankieren. Zusätzlich wurden die Enden der rDNA-Teilfragmente durch Restriktionsschnittstellen von dem Plasmid-Replikon abgegrenzt. So wurde nach Spaltung in diesen Restriktionsschnittstellen und Transformation nur noch die HK, nicht aber die Plasmid-DNA, in den Rezipientenstamm integriert. Durch Vermeidung der Integration von Plasmid-DNA, insbesondere eines Selektionsmarkers für Antibiotikaresistenz, wurde ein Beitrag zur biologischen Sicherheit geleistet.

### Beispiel 2

### Expressionsvektor pGH32RA mit Selektionsmarker ALEU2-Pro.-ATRP1

Von dem Expressionsvektor pGH32 (s. Beispiel 1) wurde dessen *ATRP1*-Promotor mittels PCR und der Primer GS78 (5'-CGA CTG TAC ACT CAA CTA GAF TGT CGG TA, Position 3326-3344; SEQ ID Nr. 21) und GS79 (5'-GTG TTG TAC AAT GAC AAC GTC GCC AAT CG; Position 3089-3071; SEQ ID Nr. 22) deletiert, wobei zugleich eine Restriktionsschnittstelle *Bsp*1407I eingeführt und so das Derivat pGH33 erzeugt wurde. Trotz Deletion des *ATRP1*-Promotors führte die Transformation von pGH33 unverändert mit hoher Häufigkeit zu schnell wachsenden Integranten-Kolonien. Von pGH32 wurde durch Pstl-Spaltung und Re-Ligation ein Plasmid pGH32R abgeleitet, das im Unterschied zu pGH32 eine gegenläufige Transkription des *amyA*-Gens zum *ATRP1*-Selektionsmarker aufweist und, wie pGH30, nach Transformation in G1212 nur langsam wachsende Integranten isolieren ließ. Ausgehend von pGH32R wurde dessen *ATRP1*-Promotor mittels PCR gegen den *ALEU2*-Promotor aus einem Plasmide pAL-ALEU2m (Wartmann, T. et al. 2003: The ALEU2 gene - a new component for an Arxula adeninivorans-based expression platform; FEMS Yeast Res. 3, 223-232) ausgetauscht und dadurch ein Derivat pGH32RA der SEQ ID Nr. 14 erzeugt, das mit hoher Häufigkeit schnell wachsende Integranten ergab. Als Primer wurden GS94 (5'-CAC CTG TAC AGG TTT TTG AAA GGG TTT GT, Position 5722-5740; SEQ ID Nr. 23) und GS95 (5'- ACA GCC GCG GTT TTC AAT CGA CGA ATT GCA; Position 6286-6267; SEQ ID Nr. 24) für die PCR des *ALEU2*-Promotors verwendet und dabei eine *Bsp*1407I-Restiktionsschnittstelle unmittelbar stromaufwärts in 5' Richtung vom ATG-Startcodons des *ATRP1*-ORF's und eine *Sac*II-Schnittstelle unmittelbar stromaufwärts in 5' Richtung des *ALEU2*-Promotors inseriert, die den *ALEU2*-Promotor flankieren.

### Beispiel 3

### Expressionssvektor pGH69 mit verkürzten 25S rDNA-Fraamenten zur singulären Integration des heterologen amyA-Gens

Der Expressionsvektor pGH69 (Fig. 2) mit der SEQ ID Nr. 15 wurde von pGH32RA ausgehend durch Reduktion der flankierenden 25S rDNA-Fragmente von 2063 und 1330 bp auf 323 bzw. 303 bp abgeleitet. Diese erfolgte mittels PCR bei Anwendung der Primer GS88 (5'-GAT CGT CGA CTC GAA CCA TCT AGT AGC TG; SEQ ID Nr. 25) und GS91 (5'-AGA GGT CGA CCA TCT CTT AGG ATC GAC TA; SEQ ID Nr. 26) sowie GS89 (5'-ACG TGT CGA CAC TGA ATT CGA TCG AGT CTG; SEQ ID Nr. 27) und GS90 (5'-TCT GGT CGA CGA AGC AGA ATT CCT GCA GAT; SEQ ID Nr. 28). Nach Transformation mit pGH69 wurden schnell wachsende Integranten (Fig. 3) mit vergleichbar hoher Häufigkeit, wie für den Ausgangsexpressionsvektor pGH32RA ermittelt. Ebenso wurde eine hohe mitotische Stabilität gefunden, die durch Trp-Zusatz nicht beeinflusst wurde.

### Integration des Expressionsvektors pGH69 in S. cerevisiae

Die Integration erfolgte in den Rezipientenstamm *S. cerevisiae* SEY6210 (MATα *leu2-3-112 ura3-52 his3-*Δ*200 trp1-*Δ*901 las2-801 suc2-*Δ*9 GAL;* Emr, S. D. et al. 1983: An MFα1/SUC2 (α-factor-invertase) gene fusion for study of protein location and gene expression in yeast. Proc. Natl. Acad. Sci. USA 80, 7080-7094) bei Anwendung der gleichen Transformationsmethode wie bei *A. adeninivorans.* Im Unterschied zu *A. adeninivorans* wurde SD Minimalmedium verwendet, das sich u. a. auch hinsichtlich der C- und N-Quelle von dem für *Arxcula* genutzten HMM unterscheidet.

Es wurde nur ein Transformationsversuch ausgeführt, bei dem 7 Integranten isoliert wurden. Subisolierungen von den Integranten ergaben Derivate unterschiedlicher Größe und alpha-Amylasebildung. Von 3 repräsentativen Integranten mittlerer Koloniegröße und mit relativ großem Stärkeabbauhof wurde die alpha-Amylasebildung in Bezug zum Wachstum verfolgt. Im Vergleich zu G1212/HK-pGH69 wurden in der stationären Phase etwa 50% der Zelldichte und 10% der alpha-Amylaseaktivität erreicht.

### Beispiel 4

### Expressionsvektoren zur multiplen Integration des heterologen amyA-Gens

Von pGH69 (s. Beispiel 2) ausgehend wurde mittels PCR und spezifischer Primer ein Spektrum von Expressionsvektoren abgeleitet, die eine zunehmend größere partielle Deletion des *ALEU2*-Promotors des *ATRP1*-Selektionsmarkers aufwiesen (Fig. 3). Von der ursprünglichen Promotor Länge von 565 bps in pGH69 wurde der Promotor schrittweise von seinem 5' Ende ausgehend auf eine Länge von 154, 148, 127, 120, 102, 92, 83, 75, 67, 53, 40 und 28 Basenpaaren deletiert und die Deletionen wurden anschließend durch Sequenzierung bestätigt. Pro Deletionstyp wurden 2 bis 5 der Expressionsvektoren in den Trp-auxotrophen Rezipientenstamm G1212 transformiert und die Selektionsplatten bis zu 4 Wochen inkubiert. Bei Deletion des *ALEU2* Promotors auf 127 bps und mit zunehmender Deletion resultierten eine verminderte Anzahl und drastische Wachstumsreduktion der Integranten, so dass Kolonien sehr unterschiedlicher Größe und unterschiedlicher alpha-Amylasebildung erst nach ca. 10 bis 30 Tagen isolierbar waren. Dagegen können Integrantenkolonien des Ausgangs-Expressionsvektors pGH69 schon 3 bis 4 Tage nach der Transformation isoliert werden.

### Beispiel 5

### Screening von multiplen Integranten des heterlogen amyA-Gens

Gemäß Beispiel 4 isolierte Integrantenkolonien verschiedener Größe, vorzugsweise solche mit erhöhter alpha-Amylasebildung; wurden in HMM suspendiert und in HMM mit oder ohne Trp-Supplementierung (20 µg/ml) kultiviert. Nach Suspendierung und Kultivierung mit oder ohne Passagierung erfolgten Vereinzelungsausstriche auf HMM-Agarplatten + 1% lösliche Stärke mit oder ohne Trp-Supplementierung. Nach 5 bis 10 Tagen Inkubation zeigten die Vereinzelungsplatten für die meisten Integrantenkolonien ein breites Spektrum von Derivaten, die sich hinsichtlich Koloniegröße, alpha-Amylasebildung und mitotischer Stabilität erheblich unterschieden. Auf Vereinzelungsplatten ohne Trp-Supplementierung wurden überwiegend extrem kleine Kolonien mit relativ großem Stärkeabbauhof beobachtet, die die primären, singulären Integranten repräsentieren. Größere Kolonien mit überdurchschnittlich großem Stärkeabbauhof ließen auf potentielle multiple Integranten schließen und wurden in HMM-Flüssigkulturen mit und ohne Trp-Supplementierung halbquantitativ auf Wachstum und alpha-Amylasebildung getestet. Pro Integrationsplasmid wurden etwa 20 bis maximal 323 (für pGH72) Vereinzelungen getestet, und 1 Derivat (Isolierungs-Nr. 259) und 2 Derivate (Isolierungs-Nr. 12 und 322) mit 3-fach bzw. 5- bis 10- fach erhöhter alpha-Amylasebildung konnten für den Integrationsvektor pGH72 (Fig. 4) isoliert werden.

### Beispiel 6

### Isolierung und quantitative Charakterisierung einer mitotisch stabilen, multiplen Integrante A. adeninivorans G1212/HK pGH72/M12/S4

Eine multiple Integrante G1212/HK_pGH721/M12/S4 wurde aus einer kleinen Kolonie mit relativ großem Stärkeabbauhof nach 20 Tagen Inkubation auf Selektionsplatten isoliert und wegen ihrer besonders hohen alpha-Amylasebildung quantitativ charakterisiert. Nach Kultivierung und Vereinzelung ohne Trp-Supplement wurde diese Integrante als eine normal wachsende Kolonie (vergleichbar mit der singulären Integrante G1212/HK-pGH69) isoliert. Bei Kultivierung in nicht-supplementiertem HMM-Flüssigmedium wuchs die Integrante mit hoher Wachstumsrate (Fig. 7) und zeigte bei Passagierungen eine hohe mitotische Stabilität (Fig. 8), wie sie auch für die singuläre Integrante G1212/HK_pGH69/2 ermittelt wurden. Quantitative Bestimmungen der alpha-Amylaseaktivität in HMM-Kulturüberständen ergaben für G1212/HK_pGH72/M12/S4 im Vergleich zu G1212/HK_pGH69/2 eine etwa 5-fache Steigerung der Produktbildung (Fig. 7).

Die Kopienzahl der HK wurde mittels Southernhybridisierung bestimmt. Nach Spaltung der chromosomalen DNA mittels *Bsp*120I, *Eco*RI, *Nco*I bzw. *Sac*I und Hybridisierung mit einem ³²P-markierten *ATRP1* bzw. *ALEU2* enthaltenen DNA Fragment wurde für G1212/HK_pGH72/M12/S4 im Vergleich zu G1212/HK-pGH69 eine Kopienzahl von 5 ermittelt. Folglich wurde die HK des Vektors bei dieser Integrante multiple und mitotisch stabil in das Genom integriert. In Fig. 7 sind die Ergebnisse für *Bsp*120I-gespaltene chromosomale DNA und für den ³²P-markierten *ALEU2*-Promotor gezeigt.

### Beispiel 7

### Integrationsvektoren pGH127 und pGH128 zur multiplen Integration des homologen ALIP1-Gens

Ausgehend von einem rekombinanten Plasmid pBS-TEF-ALIP1-PHO5-SS (Böer, E., et al.2005: An extracellular lipase from the dimorphic yeast Arxula adeninivorans: molecular cloning of the ALIP1 gene and characterization of the purified recombinant enzyme. Yeast 22, 523-535) wird das homologe *ALIP1*-Gen für eine extrazelluläre Lipase von *A. adeninivorans* nach Zweifachspaltung mittels Notl und *Spe*I in den Integrationsvektor pGH72 umkloniert, wobei *TEF1*-Pro.-*amyA* durch *TEF1*-Pro.-*ALIP1* ersetzt und so der Integrationsvektor pGH127 (Fig. 8) erhalten wird. Anstelle des *ALIP1-*Gens kann grundsätzlich jede beliebige andere für ein heterologes Protein kodierende Nukleinsäure in den pGH72 Vektor umkloniert werden, um einen erfindungsgemäßen Expressionsvektor zu erhalten.

Bei einer Konstruktionsvariante wird *TEF1*-Pro.-ALIP1-*PHO5*-Ter. mittels üblicher PCR und flankierender Restriktionsschnittstellen *Sac*II und *Spe*I zusätzlich in pGH72 inseriert, so dass in dem so erzeugten Integrationsvektor pGH128 (Fig. 9) je ein homologes und heterologes Protein-kodierendes Gen enthalten ist. Anstelle der *TEF1*-Pro.-*ALIP1-PHO5*-Ter. Protein-Expressionskassette kann grundsätzlich jede beliebige andere erfindungsgemäße Protein-Expressionskassette, vorzugsweise unter Einbeziehung einer Nukleinsäure, die für ein heterologes Protein kodiert, in den pGH72 Vektor umkloniert werden, um einen erfindungsgemäßen Expressionsvektor zu erhalten.

### Beispiel 8

### Multiple Integranten des homologen ALIP1-Gens

Entsprechend den Beispielen 4 und 5 wird die HK von pGH127 oder pGH128 in den Rezipientenstamm G1212 transformiert und multiple Integranten des *ALIP1-*Gens aufgrund von großen Tributyrin-Abbauhöfen (Methode zum Screening und zur quantitativen Lipasebestimmung s. Morin, M., et al. 2003: Lipase assay in Yarrowia lipolytica.: Springer lab manual. Wolf, K., Breunig, K., Barth, G. (Eds.): Non-conventional yeasts in genetics, biochemistry and biotechnology. Springer-Verlag Berlin Heidelberg) in Relation zum Koloniedurchmesser identifiziert. Alternativ können multiple Integranten nach Transformation von pGH127 primär aufgrund erhöhter alpha-Amylasebildung und erst sekundär durch erhöhte Lipasebildung identifiziert werden. Es werden mitotisch stabile, multiple Integranten G1212/HK-pGH127 und G1212/HK-pGH128 isoliert, die hinsichtlich des Amplifikationsgrades des Protein-kodierenden Gens und der Steigerung der Proteinbildung mit G1212/HK-pGH72/M12/S4 vergleichbar sind.

## Patentansprüche

1. Expressionsvektor zur multiplen genomischen Integration in einem Hefestamm der Gattung Arxula, umfassend:
a) eine rDNA Sequenz AA, deren Sequenz mindestens 80% Identität mit einer genomischen ribosomalen DNA Sequenz A des Hefestammes der Gattung Arxula aufweist;
b) eine Protein-Expressionskassette, enthaltend einen ersten konstitutiven oder induzierbaren Promotor an oder hinter dessen 3'-Ende eine für ein heterologes Protein kodierende Nukleinsäure insertiert werden kann, so dass die insertierte Nukleinsäure unter der Transkriptionskontrolle des Promotors steht;
c) eine Selektionsmarker-Expressionskassette, enthaltend einen zweiten konstitutiven oder induzierbaren Promotor sowie ein Selektionsmarkergen, das unter der Transkriptionskontrolle des zweiten Promotors steht;
d) eine rDNA Sequenz BB, deren Sequenz mindestens 80% Identität mit einer genomischen ribosomalen DNA Sequenz B des Hefestammes der Gattung Arxula aufweist;
e) eine Plasmid-Amplifikationskassette zur Amplifikation des Expressionsvektors in einem Wirt;
wobei der zweite Promotor der Selektionsmarker-Expressionskassette ein modifizierter ALEU2-Promotor ist, dessen Promotoraktivität höchstens etwa 20% der Aktivität des nativen ALEU2-Promotor beträgt; wobei sich die genomische ribosomale DNA Sequenz B im Genom des Hefestammes der Gattung Arxula im wesentlichen unmittelbar an das 5' Ende der genomischen ribosomalen DNA Sequenz A anschließt; wobei die rDNA Sequenzen AA und BB jeweils eine Länge von mindestens etwa 40 Nukleotiden und höchstens etwa 2.000 Nukleotide aufweisen; wobei die rDNA Sequenzen AA und BB den von den Vektorabschnitten b) und c) gebildeten Sequenzabschnitt des Expressionsvektors flankieren und wobei die Gesamtlänge des von den Abschnitten a) bis d) eingeschlossenen Abschnitts höchstens etwa 7.6kb beträgt, und wobei der modifizierte ALEU2-Promotor eine der folgenden Nukleinsäuresequenzen enthält oder daraus besteht:
a) eine Nukleinsäuresequenz der SEQ ID Nr. 2, wobei vom 5' Ende der Sequenz ausgehend etwa 0 bis etwa 100 Nukleotide deletiert wurden;
b) eine Nukleinsäuresequenz der SEQ ID Nr.3, wobei vom 5' Ende der Sequenz ausgehend etwa 0 bis etwa 92 Nukleotide deletiert wurden;
c) eine Nukleinsäuresequenz der SEQ ID Nr. 4, wobei vom 5' Ende der Sequenz ausgehend etwa 0 bis etwa 83 Nukleotide deletiert wurden;
d) eine Nukleinsäuresequenz der SEQ ID Nr. 5, wobei vom 5' Ende der Sequenz ausgehend etwa 0 bis etwa 74 Nukleotide deletiert wurden;
e) eine Nukleinsäuresequenz der SEQ ID Nr. 6, wobei vom 5' Ende der Sequenz ausgehend etwa 0 bis etwa 64 Nukleotide deletiert wurden;
f) eine Nukleinsäuresequenz der SEQ ID Nr. 7, wobei vom 5' Ende der Sequenz ausgehend etwa 0 bis etwa 55 Nukleotide deletiert wurden;
g) eine Nukleinsäuresequenz der SEQ ID Nr. 8, wobei vom 5' Ende der Sequenz ausgehend etwa 0 bis etwa 48 Nukleotide deletiert wurden;
h) eine Nukleinsäuresequenz der SEQ ID Nr. 9, wobei vom 5' Ende der Sequenz ausgehend etwa 0 bis etwa 39 Nukleotide deletiert wurden;
i) eine Nukleinsäuresequenz der SEQ ID Nr. 10, wobei vom 5' Ende der Sequenz ausgehend etwa 0 bis etwa 25 Nukleotide deletiert wurden;
j) eine Nukleinsäuresequenz der SEQ ID Nr. 11, wobei vom 5' Ende der Sequenz ausgehend etwa 0 bis etwa 12 Nukleotide deletiert wurden;
k) eine Nukleinsäuresequenz der SEQ ID Nr. 12, wobei vom 5' Ende der Sequenz ausgehend etwa 0 bis etwa 10 Nukleotide deletiert wurden;
l) eine Nukleinsäuresequenz, die mindestens 50% Sequenzidentität mit einer der Nukleinsäuresequenzen gemäß a) bisk) aufweist und höchstens etwa 20% der Promotoraktivität des nativen ALEU2-Promotors aufweist;
m) eine Nukleinsäuresequenz, die mit einem Gegenstrang einer der Nukleinsäuresequenzen gemäß a) bis I) unter stringenten Bedingungen hybridisiert und höchstens etwa 20% der Promotoraktivität des nativen ALEU2-Promotors aufweist; und
n) eine Nukleinsäuresequenz, die durch Substitution, Addition und/oder Deletion eines oder mehrerer, vorzugsweise von höchstens etwa 10% der Nukleotide der Nukleinsäuresequenzen gemäß a) bis I) erzeugt wird und höchstens etwa 20% der Promotoraktivität des nativen ALEU2-Promotors aufweist.

2. Expressionsvektor nach Anspruch 1, wobei das Selektionsmarkergen ein Auxotrophiemarkergen oder ein dominantes Markergen ist.

3. Expressionsvektor nach einem der Ansprüche 1 oder 2, wobei das Selektionsmarkergen ausgewählt ist aus *ALEU2, ATRP1, ALYS2* und *AILV1*, oder einer Variante davon, die die Funktion des Gens im wesentlichen beibehält.

4. Expressionsvektor nach einem der Ansprüche 1 bis 3, wobei der Vektor ferner eine Reportergen-Expressionskassette umfasst, die einen dritten konstitutiven oder induzierbaren Promotor sowie ein Reportergen enthält, das unter der Transkriptionskontrolle des dritten Promotors steht, wobei die Reportergen-Expressionskassette in dem Sequenzabschnitt angeordnet ist, der von den rDNA Sequenzen AA und BB flankiert wird und in dem auch die Abschnitte b) und c) liegen.

5. Expressionsvektor nach einem der Ansprüche 1 bis 3, wobei das Reportergen ausgewählt ist aus dem green fluorescent protein-Gen, dem yellow fluorescent protein-Gen und dem für extrazelluläre alpha-Amylase kodierenden AmyA-Gen .

6. Expressionsvektor nach einem der Ansprüche 1 bis 5, wobei der dritte Promotor ein starker Promotor ist, vorzugsweise ein Promotor ausgewählt aus TEF1-, GAA-, ALIP- und ATAN Promoter.

7. Expressionsvektor nach einem der Ansprüche 1 bis 6, wobei der erste Promotor ein starker Promotor ist, vorzugsweise ein Promotor ausgewählt aus TEF1-, GAA-, ALIP1- und ATAN-Promoter.

8. Expressionsvektor nach einem der Ansprüche 1 bis 7, wobei die rDNA Sequenzen AA und/oder BB eine Länge von etwa 50 bis etwa 2.000 Nukleotiden haben.

9. Expressionsvektor nach einem der Ansprüche 1 bis 8, wobei die die Bereiche b) und c) des Vektors flankierenden rDNA Sequenzen AA und BB in ihrer relativen Lage und Orientierung im Vektor zueinander der Lage und Orientierung der entsprechenden sequenzidentischen genomischen ribosomalen DNA Sequenzen A und B im Genom des Hefestammes entsprechen.

10. Expressionsvektor nach einem der Ansprüche 1 bis 9, wobei die genomische ribosomale DNA ausgewählt ist aus einer DNA, die für 5,8S RNA kodiert, einer DNA, die für 18S RNA kodiert, einer DNA, die für 25S RNA kodiert, einer DNA, die für 45S RNA kodiert und einer DNA, die aus einem flankierenden Bereich der vorgenannten RNA kodierenden Bereiche stammt.

11. Expressionsvektor nach einem der Ansprüche 1 bis 10, wobei der Vektor zusätzlich mindestens einen Polylinker enthält.

12. Expressionsvektor nach einem der Ansprüche 1 bis 10, wobei die Plasmid-Amplifikationskassette an oder hinter ihrem 3' Ende und/oder an oder hinter ihrem 5' Ende mindestens eine Restriktionsschnittstelle aufweist, die ein Abtrennen der Plasmid-Amplifikationskassette aus dem Expressionsvektors erlaubt, ohne den übrigen Expressionsvektorteil zu zerschneiden.

13. Expressionsvektor nach einem der Ansprüche 1 bis 12, wobei der Vektor ein Vektor ist, der eine Nukleinsäuresequenz, ausgewählt aus der SEQ ID Nr. 13, SEQ ID Nr. 14, SEQ ID Nr. 15, SEQ ID Nr. 16, SEQ ID Nr. 17 und SEQ ID Nr. 18, enthält, wobei in den Vektor eine Protein-Expressionskassette insertiert werden kann oder wobei die für das heterologe Protein kodierende Nukleinsäure anstelle des AmyA Gens insertiert werden kann, und funktionelle Varianten der vorgenannten Vektoren.

14. Expressionsvektor nach einem der Ansprüche 1 bis 13, wobei die Protein-Expressionskassette eine Nukleinsäuresequenz aufweist, die für ein Signalpeptid kodiert.

15. Expressionsvektor nach einem der Ansprüche 1 bis 13, wobei die für ein heterologes Protein kodierende Nukleinsäure in den Vektor insertiert wurde.

16. Wirtszelle der Gattung Arxula, enthaltend den von den Abschnitten a) bis d) eingeschlossenen Teil eines Expressionsvektors nach einem der Ansprüche 1 bis 14, wobei die für ein heterologes Protein kodierende Nukleinsäure in den Expressionsvektor insertiert wurde.

17. Wirtszelle nach Anspruch 16, wobei die Wirtszelle von einem Hefestamm abgeleitet ist, der eine Auxotrophie aufweist, die mit dem Selektionsmarkergen komplementiert werden kann.

18. Wirtszelle nach einem der Ansprüche 16 oder 17, wobei der Hefestamm eine Tryptophan-Auxotrophie aufweist.

19. Kit, enthaltend einen Expressionsvektor nach einem der Ansprüche 1 bis 15, eine Anleitung zur Benutzung und optional eine Wirtszelle der Gattung Arxula.

20. Kit nach Anspruch 19, wobei die Wirtszelle eine Auxotrophie aufweist, die mit dem Selektionsmarkergen komplementiert werden kann.

21. Kit enthaltend eine Wirtszelle nach einem der Ansprüche 16 bis 18 sowie eine Anleitung zur Benutzung.

22. Verfahren zur Expression eines heterologen Proteins in einem Hefestamm der Gattung Arxula umfassend:
1) Klonieren der Nukleinsäure, die für das heterologe Protein kodiert, in einen Expressionsvektor nach einem der Ansprüche 1 bis 14;
2) Einführen des von den Abschnitten a) bis d) eingeschlossenen Teils des in 1) erhaltenen Expressionsvektors, gegebenenfalls in linearisierter Form, in eine zur Expression des heterologen Proteins geeignete Wirtszelle der Gattung Arxula;
3) Kultivieren der in 2) erhaltenen Wirtszelle unter Bedingungen, die eine Expression des Selektionsmarkers sowie des heterologen Proteins erlauben;
4) Isolieren einer den Selektionsmarker sowie das heterologe Protein exprimierenden Zelle; und
5) Rekultivieren der in Schritt 4) isolierten Zelle unter Bedingungen, die eine Expression des heterologen Proteins erlauben; und
6) Ernten des heterologen Proteins.

23. Verfahren nach Anspruch 22, wobei der Hefestamm ausgewählt ist aus *Arxula adeninivorans* und *Arxula terrestris.*

24. Verfahren nach Anspruch 22 oder 23, wobei der Hefestamm eine Auxotrophie aufweist, die mit dem Selektionsmarkergen komplementiert werden kann.

25. Verfahren nach einem der Ansprüche 22 bis 24, wobei der Hefestamm eine Tryptophan-Auxotrophie aufweist.

26. Verfahren nach einem der Ansprüche 22 bis 25, wobei der Vektor ferner eine Reportergen-Expressionskassette umfasst, die einen dritten konstitutiven oder induzierbaren Promotor sowie ein Reportergen enthält, das unter der Transkriptionskontrolle des dritten Promotors steht, wobei die Reportergen-Expressionskassette in dem Sequenzabschnitt angeordnet ist, der von den rDNA Sequenzen AA und BB flankiert wird und in dem auch die Abschnitte b) und c) liegen; wobei in Schritt 2) die Wirtszelle unter Bedingungen kultiviert wird, die eine Expression des Selektionsmarkers sowie des Reportergens erlauben und in Schritt 4) eine Zelle isoliert wird, die den Selektionsmarker sowie das Reportergen exprimiert.

27. Verfahren nach Anspruch 26, wobei das Reportergen ausgewählt ist aus dem green fluorescent protein-Gen, dem yellow fluorescent protein-Gen und dem AmyA-Gen kodierend für extrazelluläre alpha-Amylase.

28. Verfahren zur Expression eines heterologen Proteins in einem Hefestamm der Gattung Arxula umfassend:
1) Kultivieren einer nach einem der Ansprüche 24 bis 29 in Schritt 4) isolierten Wirtszelle oder einer Wirtszelle nach einem der Ansprüche 18 bis 20, unter Bedingungen, die eine Expression des heterologen Proteins erlauben; und
2) Ernten des heterologen Proteins.

29. Verfahren nach einem der Ansprüche 22 bis 28, wobei das geerntete heterologe Protein gereinigt wird.

30. Verwenden eines Expressionsvektors nach einem der Ansprüche 1 bis 15, einer Wirtszelle nach einem der Ansprüche 16 bis 18 oder eines Kits nach Anspruch 19 bis 21 zur Herstellung eines heterologen Proteins.

31. Verfahren zur Herstellung einer Hefezelle der Gattung Arxula, die zur Expression eines heterologen Proteins geeignet ist, umfassend:
1) Klonieren der Nukleinsäure, die für das heterologes Protein kodiert, in einen Expressionsvektor nach einem der Ansprüche 1 bis 14;
2) Einführen des von den Abschnitten a) bis d) eingeschlossenen Teils des in 1) erhaltenen Expressionsvektors, gegebenenfalls in linearisierter Form, in eine zur Expression des heterologen Proteins geeignete Wirtszelle der Gattung Arxula;
3) Kultivieren der in 2) erhaltenen Wirtszelle unter Bedingungen, die eine Expression des Selektionsmarkers sowie des heterologen Proteins erlauben; und
4) Isolieren einer den Selektionsmarker sowie das heterologe Protein exprimierenden Zelle, die den von den Abschnitten a) bis d) eingeschlossenen Teils des Expressionsvektors multiple und mitotisch stabil in ihr Genom integriert hat.

32. Verfahren nach Anspruch 31, wobei der Expressionsvektor ferner eine Reportergen-Expressionskassette enthält und das Kultivieren in Schritt 3) unter Bedingungen erfolgt, die eine Expression des Selektionsmarkers sowie des Reportergens erlauben.

## Claims

1. Expression vector for multiple genomic integration in a yeast strain of the genus Arxula, comprising:
a) an rDNA sequence AA, the sequence of which has at least 80 % identity with a genomic ribosomal DNA sequence A of the yeast strain of the genus Arxula;
b) a protein expression cassette containing a first constitutive or inducible promoter at or behind the 3' end of which a nucleic acid which codes for a heterologous protein can be inserted, so that the nucleic acid inserted is under the transcription control of the promoter;
c) a selection marker expression cassette containing a second constitutive or inducible promoter and a selection marker gene which is under the transcription control of the second promoter;
d) an rDNA sequence BB, the sequence of which has at least 80 % identity with a genomic ribosomal DNA sequence B of the yeast strain of the genus Arxula;
e) a plasmid amplification cassette for
amplification of the expression vector in a host;
wherein the second promoter of the selection marker expression cassette is a modified ALEU2 promoter, the promoter activity of which is at most about 20 % of the activity of the native ALEU2 promoter; wherein the genomic ribosomal DNA sequence B in the genome of the yeast strain of the genus Arxula is essentially joined directly to the 5' end of the genomic ribosomal DNA sequence A; wherein the rDNA sequences AA and BB each have a length of at least about 40 nucleotides and at most about 2,000 nucleotides; wherein the rDNA sequences AA and BB flank the sequence section of the expression vector formed by the vector sections b) and c) and wherein the total length of the section enclosed by the sections a) to d) is at most about 7.6 kb, and wherein the modified ALEU2 promoter contains one of the following nucleic acid sequences or consists of this:
a) a nucleic acid sequence of SEQ ID No. 2, wherein starting from the 5' end of the sequence about 0 to about 100 nucleotides have been deleted;
b) a nucleic acid sequence of SEQ ID No. 3, wherein starting from the 5' end of the sequence about 0 to about 92 nucleotides have been deleted;
c) a nucleic acid sequence of SEQ ID No. 4, wherein starting from the 5' end of the sequence about 0 to about 83 nucleotides have been deleted;
d) a nucleic acid sequence of SEQ ID No. 5, wherein starting from the 5' end of the sequence about 0 to about 74 nucleotides have been deleted;
e) a nucleic acid sequence of SEQ ID No. 6, wherein starting from the 5' end of the sequence about 0 to about 64 nucleotides have been deleted;
f) a nucleic acid sequence of SEQ ID No. 7, wherein starting from the 5' end of the sequence about 0 to about 55 nucleotides have been deleted;
g) a nucleic acid sequence of SEQ ID No. 8, wherein starting from the 5' end of the sequence about 0 to about 48 nucleotides have been deleted;
h) a nucleic acid sequence of SEQ ID No. 9, wherein starting from the 5' end of the sequence about 0 to about 39 nucleotides have been deleted;
i) a nucleic acid sequence of SEQ ID No. 10, wherein starting from the 5' end of the sequence about 0 to about 25 nucleotides have been deleted;
j) a nucleic acid sequence of SEQ ID No. 11, wherein starting from the 5' end of the sequence about 0 to about 12 nucleotides have been deleted;
k) a nucleic acid sequence of SEQ ID No. 12, wherein starting from the 5' end of the sequence about 0 to about 10 nucleotides have been deleted;
l) a nucleic acid sequence which has at least 50 % sequence identity with one of the nucleic acid sequences according to a) to k) and has at most about 20 % of the promoter activity of the native ALEU2 promoter;
m) a nucleic acid sequence which hybridizes with a counter-strand of one of the nucleic acid sequences according to a) to 1) under stringent conditions and has at most about 20 % of the promoter activity of the native ALEU2 promoter; and
n) a nucleic acid sequence which is produced by substitution, addition and/or deletion of one or more, preferably of at most about 10 % of the nucleotides of the nucleic acid sequences according to a) to 1) and has at most about 20 % of the promoter activity of the native ALEU2 promoter.

2. Expression vector according to claim 1, wherein the selection marker gene is an auxotrophy marker gene or a dominant marker gene.

3. Expression vector according to one of claims 1 or 2, wherein the selection marker gene is chosen from *ALEU2, ATRP1, ALYS2* and *AILV1*, or a variant thereof which essentially retains the function of the gene.

4. Expression vector according to one of claims 1 to 3, wherein the vector furthermore comprises a reporter gene expression cassette which contains a third constitutive or inducible promoter and a reporter gene which is under the transcription control of the third promoter, wherein the reporter gene expression cassette is located in the sequence section which is flanked by the rDNA sequences AA and BB and in which the sections b) and c) also lie.

5. Expression vector according to one of claims 1 to 3, wherein the reporter gene is chosen from the green fluorescent protein gene, the yellow fluorescent protein gene and the AmyA gene which codes for extracellular alpha-amylase.

6. Expression vector according to one of claims 1 to 5, wherein the third promoter is a potent promoter, preferably a promoter chosen from the TEF1, GAA, ALIP and ATAN promoter.

7. Expression vector according to one of claims 1 to 6, wherein the first promoter is a potent promoter, preferably a promoter chosen from the TEF1, GAA, ALIP1 and ATAN promoter.

8. Expression vector according to one of claims 1 to 7, wherein the rDNA sequences AA and/or BB have a length of from about 50 to about 2,000 nucleotides.

9. Expression vector according to one of claims 1 to 8, wherein the rDNA sequences AA and BB flanking the regions b) and c) of the vector correspond in their relative position and orientation to one another in the vector to the position and orientation of the corresponding sequence-identical genomic ribosomal DNA sequences A and B in the genome of the yeast strain.

10. Expression vector according to one of claims 1 to 9, wherein the genomic ribosomal DNA is chosen from a DNA which codes for 5.8S RNA, a DNA which codes for 18S RNA, a DNA which codes for 25S RNA, a DNA which codes for 45S RNA and a DNA which originates from a flanking region of the abovementioned RNA-coding regions.

11. Expression vector according to one of claims 1 to 10, wherein the vector additionally contains at least one polylinker.

12. Expression vector according to one of claims 1 to 10, wherein the plasmid amplification cassette has at or behind its 3' end and/or at or behind its 5' end at least one restriction cleavage site which allows the plasmid amplification cassette to be separated off from the expression vector without cutting the remaining expression vector part.

13. Expression vector according to one of claims 1 to 12, wherein the vector is a vector which contains a nucleic acid sequence chosen from SEQ ID No. 13, SEQ ID No. 14, SEQ ID No. 15, SEQ ID No. 16, SEQ ID No. 17 and SEQ ID No. 18, wherein a protein expression cassette can be inserted into the vector or wherein the nucleic acid which codes for the heterologous protein can be inserted instead of the AmyA gene, and functional variants of the abovementioned vectors.

14. Expression vector according to one of claims 1 to 13, wherein the protein expression cassette has a nucleic acid sequence which codes for a signal peptide.

15. Expression vector according to one of claims 1 to 13, wherein the nucleic acid which codes for a heterologous protein has been inserted into the vector.

16. Host cell of the genus Arxula, containing the part of an expression vector according to one of claims 1 to 14 enclosed by the sections a) to d), wherein the nucleic acid which codes for a heterologous protein has been inserted into the expression vector.

17. Host cell according to claim 16, wherein the host cell is derived from a yeast strain which has an auxotrophy which can be complemented with the selection marker gene.

18. Host cell according to one of claims 16 or 17, wherein the yeast strain has a tryptophan auxotrophy.

19. Kit containing an expression vector according to one of claims 1 to 15, instructions for use and optionally a host cell of the genus Arxula.

20. Kit according to claim 19, wherein the host cell has an auxotrophy which can be complemented with the selection marker gene.

21. Kit containing a host cell according to one of claims 16 to 18 and instructions for use.

22. Method for expression of a heterologous protein in a yeast strain of the genus Arxula, comprising:
1) cloning of the nucleic acid which codes for the heterologous protein into an expression vector according to one of claims 1 to 14;
2) introduction of the part of the expression vector obtained in 1) enclosed by the sections a) to d), optionally in linearized form, into a host cell of the genus Arxula suitable for expression of the heterologous protein;
3) cultivation of the host cell obtained in 2) under conditions which allow an expression of the selection marker and of the heterologous protein;
4) isolation of a cell which expresses the selection marker and the heterologous protein; and
5) recultivation of the cell isolated in step 4) under conditions which allow an expression of the heterologous protein; and
6) harvesting of the heterologous protein.

23. Method according to claim 22, wherein the yeast strain is chosen from *Arxula adeninivorans* and *Arxula terrestris.*

24. Method according to claim 22 or 23, wherein the yeast strain has an auxotrophy which can be complemented with the selection marker gene.

25. Method according to one of claims 22 to 24, wherein the yeast strain has a tryptophan auxotrophy.

26. Method according to one of claims 22 to 25, wherein the vector furthermore comprises a reporter gene expression cassette which contains a third constitutive or inducible promoter and a reporter gene which is under the transcription control of the third promoter, wherein the reporter gene expression cassette is located in the sequence section which is flanked by the rDNA sequences AA and BB and in which the sections b) and c) also lie; wherein in step 2) the host cell is cultivated under conditions which allow expression of the selection marker and of the reporter gene and in step 4) a cell which expresses the selection marker and the reporter gene is isolated.

27. Method according to claim 26, wherein the reporter gene is chosen from the green fluorescent protein gene, the yellow fluorescent protein gene and the AmyA gene coding for extracellular alpha-amylase.

28. Method for expression of a heterologous protein in a yeast strain of the genus Arxula, comprising:
1) cultivation of a host cell isolated according to one of claims 24 to 29 in step 4) or a host cell according to one of claims 18 to 20, under conditions which allow an expression of the heterologous protein; and
2) harvesting of the heterologous protein.

29. Method according to one of claims 22 to 28, wherein the harvested heterologous protein is purified.

30. Use of an expression vector according to one of claims 1 to 15, a host cell according to one of claims 16 to 18 or a kit according to claim 19 to 21 for the preparation of a heterologous protein.

31. Method for the production of a yeast cell of the genus Arxula which is suitable for expression of a heterologous protein, comprising:
1) cloning of the nucleic acid which codes for the heterologous protein into an expression vector according to one of claims 1 to 14;
2) introduction of the part of the expression vector obtained in 1) enclosed by the sections a) to d), optionally in linearized form, into a host cell of the genus Arxula suitable for expression of the heterologous protein;
3) cultivation of the host cell obtained in 2) under conditions which allow an expression of the selection marker and of the heterologous protein; and
4) isolation of a cell which expresses the selection marker and the heterologous protein and which has integrated into its genome multiply and in a mitotically stable manner the part of the expression vector enclosed by the sections a) to d) .

32. Method according to claim 31, wherein the expression vector furthermore contains a reporter gene expression cassette and the cultivation in step 3) is carried out under conditions which allow an expression of the selection marker and of the reporter gene.

## Revendications

1. Vecteur d'expression pour l'intégration génomique multiple dans une souche de levure de l'espèce Arxula, comprenant :
a) une séquence AA d'ADNr dont la séquence présente au moins 80 % d'identité avec une séquence A d'ADN ribosomal génomique de la souche de levure de l'espèce Arxula ;
b) une cassette d'expression de protéine, contenant un premier promoteur constitutif ou inductible, un acide nucléique codant pour une protéine hétérologue pouvant être inséré au niveau de son extrémité 3' ou à l'arrière de celle-ci, de sorte que l'acide nucléique inséré se trouve sous le contrôle transcriptionnel du promoteur ;
c) une cassette d'expression de marqueur de sélection contenant un deuxième promoteur constitutif ou inductible et un gène marqueur de sélection qui se trouve sous le contrôle transcriptionnel du deuxième promoteur ;
d) une séquence BB d'ADNr dont la séquence présente au moins 80 % d'identité avec une séquence B d'ADN ribosomal génomique de la souche de levure de l'espèce Arxula ;
e) une cassette d'amplification de plasmide pour l'amplification du vecteur d'expression chez un hôte ;
le deuxième promoteur de la cassette d'expression du marqueur de sélection étant un promoteur ALEU2 modifié dont l'activité du promoteur est au plus environ 20 % de l'activité du promoteur ALEU2 natif ; la séquence B d'ADN ribosomal génomique dans le génome de la souche de levure de l'espèce Arxula étant essentiellement immédiatement adjacente à l'extrémité 5' de la séquence A d'ADN ribosomal génomique ; les séquences AA et BB d'ADNr présentant respectivement une longueur d'au moins environ 40 nucléotides et d'au plus environ 2000 nucléotides ; les séquences AA et BB d'ADNr flanquant le segment de séquence formé des segments de vecteur b) et c) et la longueur totale du segment inclus des segments a) à d) étant au plus d'environ 7,6 kb et le promoteur ALEU2 modifié contenant l'une des séquences d'acide nucléique suivante ou étant constitué de celles-ci :
a) une séquence d'acide nucléique de SEQ ID N° : 2, dans laquelle à partir de l'extrémité 5' de la séquence, environ 0 à environ 100 nucléotides ont été supprimés ;
b) une séquence d'acide nucléique de SEQ ID N° : 3, dans laquelle à partir de l'extrémité 5' de la séquence, environ 0 à environ 92 nucléotides ont été supprimés ;
c) une séquence d'acide nucléique de SEQ ID N° : 4, dans laquelle à partir de l'extrémité 5' de la séquence, environ 0 à environ 83 nucléotides ont été supprimés ;
d) une séquence d'acide nucléique de SEQ ID N° : 5, dans laquelle à partir de l'extrémité 5' de la séquence, environ 0 à environ 74 nucléotides ont été supprimés ;
e) une séquence d'acide nucléique de SEQ ID N° : 6, dans laquelle à partir de l'extrémité 5' de la séquence, environ 0 à environ 64 nucléotides ont été supprimés ;
f) une séquence d'acide nucléique de SEQ ID N° : 7, dans laquelle à partir de l'extrémité 5' de la séquence, environ 0 à environ 55 nucléotides ont été supprimés ;
g) une séquence d'acide nucléique de SEQ ID N° : 8, dans laquelle à partir de l'extrémité 5' de la séquence, environ 0 à environ 48 nucléotides ont été supprimés ;
h) une séquence d'acide nucléique de SEQ ID N° : 9, dans laquelle à partir de l'extrémité 5' de la séquence, environ 0 à environ 39 nucléotides ont été supprimés ;
i) une séquence d'acide nucléique de SEQ ID N° : 10, dans laquelle à partir de l'extrémité 5' de la séquence, environ 0 à environ 25 nucléotides ont été supprimés ;
j) une séquence d'acide nucléique de SEQ ID N° : 11, dans laquelle à partir de l'extrémité 5' de la séquence, environ 0 à environ 12 nucléotides ont été supprimés ;
k) une séquence d'acide nucléique de SEQ ID N° : 12, dans laquelle à partir de l'extrémité 5' de la séquence, environ 0 à environ 10 nucléotides ont été supprimés ;
l) une séquence d'acide nucléique qui présente au moins 50 % d'identité de séquence avec l'une des séquences d'acide nucléique selon les points a) à k) et présente au plus environ 20 % de l'activité de promoteur du promoteur ALEU2 natif ;
m) une séquence d'acide nucléique qui s'hybride avec un brin anti-sens de l'une des séquences d'acide nucléique selon les points a) à 1) dans des conditions stringentes et présente au plus environ 20 % de l'activité de promoteur du promoteur ALEU2 natif ;
n) une séquence d'acide nucléique qui est produite par substitution, addition et/ou délétion d'un ou plusieurs, de préférence au plus d'environ 10 % des nucléotides des séquences d'acide nucléique selon les points a) à 1) et présente au plus environ 20 % de l'activité de promoteur du promoteur ALEU2 natif ;

2. Vecteur d'expression selon la revendication 1, dans lequel le gène du marqueur de sélection est un gène marqueur d'auxotrophie ou un gène marqueur dominant.

3. Vecteur d'expression selon l'une des revendications 1 ou 2, dans lequel le gène du marqueur de sélection est choisi parmi ALEU2, ATRP1, ALYS2 et AILV1 ou une variante de ceux-ci qui conserve essentiellement la fonction du gène.

4. Vecteur d'expression selon l'une des revendications 1 à 3, le vecteur comprenant en outre une cassette d'expression du gène reporter qui contient un troisième promoteur constitutif ou inductible et un gène reporter qui se trouve sous le contrôle transcriptionnel du troisième promoteur, la cassette d'expression du gène reporter étant disposée dans le segment de séquence qui est flanqué des séquences AA et BB d'ADNr et dans laquelle se trouvent également les segments b) et c).

5. Vecteur d'expression selon l'une des revendications 1 à 3, dans lequel le gène reporter est choisi parmi le gène de protéine fluorescente verte, le gène de protéine fluorescente jaune et le gène d'AmyA codant pour une alpha-amylase extracellulaire.

6. Vecteur d'expression selon l'une des revendications 1 à 5, dans lequel le troisième promoteur est un promoteur plus puissant, de préférence un promoteur choisi parmi TEF1, GAA, ALIP et ATAN.

7. Vecteur d'expression selon l'une des revendications 1 à 6, le premier promoteur étant un promoteur plus puissant, de préférence un promoteur choisi parmi TEF1, GAA, ALIP1 et ATAN.

8. Vecteur d'expression selon l'une des revendications 1 à 7, dans lequel les séquences AA et/ou BB d'ADNr ont une longueur d'environ 50 à environ 2000 nucléotides.

9. Vecteur d'expression selon l'une des revendications 1 à 8, dans lequel les séquences AA et BB d'ADNr flanquant les régions b) et c) du vecteur correspondent dans leur situation et orientation relatives dans le vecteur aux situation et orientation des séquences A et B correspondantes, à identité de séquence, d'ADN ribosomal génomique dans le génome de la souche de levure.

10. Vecteur d'expression selon l'une des revendications 1 à 9, dans lequel l'ADN ribosomal génomique est choisi parmi un ADN qui code pour l'ARN 5,8S, un ADN qui code pour l'ARN 18S, un ADN qui code pour l'ARN 25S, un ADN qui code pour l'ARN 45S et un ADN qui provient d'une région flanquante des régions codant pour les ARN mentionnés précédemment.

11. Vecteur d'expression selon l'une des revendications 1 à 10, le vecteur contenant en outre au moins un lieur multisite.

12. Vecteur d'expression selon l'une des revendications 1 à 10, dans lequel la cassette d'amplification de plasmide présente sur ou à l'arrière de son extrémité 3' et/ou sur ou à l'arrière de son extrémité 5', au moins un site de coupure de restriction qui permet une séparation de la cassette d'amplification de plasmide du vecteur d'expression sans découper la partie de vecteur d'expression.

13. Vecteur d'expression selon l'une des revendications 1 à 12, le vecteur étant un vecteur qui contient une séquence d'acide nucléique choisie parmi SEQ ID N° : 13, SEQ ID N° : 14, SEQ ID N° : 15, SEQ ID N° : 16, SEQ ID N° : 17 et SEQ ID N° : 18, une cassette d'expression de protéine pouvant être insérée dans le vecteur ou l'acide nucléique codant pour la protéine hétérologue pouvant être inséré à la place du gène AmyA et des variantes fonctionnelles des vecteurs précédemment cités.

14. Vecteur d'expression selon l'une des revendications 1 à 13, dans lequel la cassette d'expression de protéine présente une séquence d'acide nucléique qui code pour un peptide de signal.

15. Vecteur d'expression selon l'une des revendications 1 à 13, dans lequel l'acide nucléique codant pour une protéine hétérologue a été inséré dans le vecteur.

16. Cellule hôte de l'espèce Arxula, contenant la partie incluse des segments a) à d) d'un vecteur d'expression selon l'une des revendications 1 à 14, dans laquelle l'acide nucléique codant pour une protéine hétérologue a été inséré dans le vecteur d'expression.

17. Cellule hôte selon la revendication 16, la cellule hôte étant dérivée d'une souche de levure qui présente une auxotrophie qui peut être complémentée par le gène marqueur de sélection.

18. Cellule hôte selon l'une des revendications 16 ou 17, dans laquelle la souche de levure présente une auxotrophie au tryptophane.

19. Kit contenant un vecteur d'expression selon l'une des revendications 1 à 15, des instructions d'utilisation et éventuellement une cellule hôte de l'espèce arxula.

20. Kit selon la revendication 19, dans lequel la cellule hôte présente une auxotrophie qui peut être complémentée par le gène marqueur de sélection.

21. Kit contenant une cellule hôte selon l'une des revendications 16 à 18, et des instructions d'utilisation.

22. Procédé d'expression d'une protéine hétérologique dans une souche de levure de l'espèce Arxula comprenant :
1) le clonage de l'acide nucléique qui code pour la protéine hétérologue dans un vecteur d'expression selon l'une des revendications 1 à 14 ;
2) l'introduction de la partie incluse des segments a) à d) du vecteur d'expression obtenu au point 1), éventuellement dans une forme linéarisée, dans une cellule hôte de l'espèce Arxula, appropriée pour l'expression de la protéine hétérologue ;
3) la culture des cellules hôtes obtenues au point 2) dans des conditions qui permettent une expression du marqueur de sélection et de la protéine hétérologue ;
4) l'isolement d'une cellule exprimant le marqueur de sélection et la protéine hétérologue ; et
5) la nouvelle culture des cellules isolées à l'étape 4) dans des conditions qui permettent une pression de la protéine hétérologue ; et
6) la récolte de la protéine hétérologue.

23. Procédé selon la revendication 22, dans lequel la souche de levure est choisie parmi *Arxula adeninivorans* et *Arxula terrestris.*

24. Procédé selon la revendication 22 ou 23, dans lequel la souche de levure présente une auxotrophie qui peut être complémentée par le gène marqueur de sélection.

25. Procédé selon l'une des revendications 22 à 24, dans lequel la souche de levure présente une auxotrophie au tryptophane.

26. Procédé selon l'une des revendications 22 à 25, dans lequel le vecteur comprend en outre une cassette d'expression du gène reporter qui contient un troisième promoteur constitutif ou inductible et un gène reporter qui se trouve sous le contrôle transcriptionnel du troisième promoteur, la cassette d'expression du gène reporter étant disposée dans le segment de séquence qui est flanqué des séquences AA et BB d'ADNr et dans lequel se trouvent également les segments b) et c) ; à l'étape 2), la cellule
hôte étant cultivée dans des conditions qui permettent une expression du marqueur de sélection et du gène reporter et à l'étape 4), une cellule est isolée, qui exprime le marqueur de sélection et le gène reporter.

27. Procédé selon la revendication 26, dans lequel le gène reporter est choisi parmi le gène de la protéine fluorescente verte, le gène de la protéine fluorescente jaune et le gène AmyA codant pour l'alpha-amylase extracellulaire.

28. Procédé d'expression d'une protéine hétérologue dans une souche de levure de l'espèce Arxula comprenant :
1) la culture d'une cellule hôte isolée à l'étape 4) selon l'une des revendications 24 à 29 ou d'une cellule hôte selon l'une des revendications 18 à 20, dans des conditions qui permettent une expression de la protéine hétérologue ; et
2) la récolte de la protéine hétérologue.

29. Procédé selon l'une des revendications 22 à 28, dans lequel la protéine hétérologue récoltée est purifiée.

30. Utilisation d'un vecteur d'expression selon l'une des revendications 1 à 15, d'une cellule hôte selon l'une des revendications 16 à 18 ou d'un kit selon les revendications 19 à 21 pour la production d'une protéine hétérologue.

31. Procédé de production d'une cellule de levure de l'espèce Arxula qui est appropriée pour l'expression d'une protéine hétérologue, comprenant :
1) le clonage de l'acide nucléique qui code pour la protéine hétérologue dans un vecteur d'expression selon l'une des revendications 1 à 14 ;
2) l'introduction de la partie incluse des segments a) à d) du vecteur d'expression obtenu au point 1), éventuellement dans une forme linéarisée, dans une cellule hôte de l'espèce Arxula, appropriée pour l'expression de la protéine hétérologue
3) la culture des cellules hôtes obtenues au point 2) dans des conditions qui permettent une expression du marqueur de sélection et de la protéine hétérologue ; et
4) l'isolement d'une cellule exprimant le marqueur de sélection et la protéine hétérologue, qui a intégré la partie incluse des segments a) à d) du vecteur d'expression de façon multiple et stable d'un point de vue mitotique dans son génome.

32. Procédé selon la revendication 31, dans lequel le vecteur d'expression contient en outre une cassette d'expression du gène reporter et la culture s'effectue à l'étape 3) dans des conditions qui permettent une expression du marqueur de sélection et du gène reporter.


### SEQUENCE LISTING

<110> Leibnitz-Institut für Pflanzengenetik und
   Kulturpflanzenforschung
<120> Plasmidvektoren zur multiplen Gen-Integration und Überexpression von heterologen Proteinen in Arxula adeninivorans und anderen Hefen
<130> EP46213
<140> noch nicht bekannt
   <141> hiermit
<160> 42
<170> PatentIn version 3.3
<210> 1
   <211> 148
   <212> DNA
   <213> Artificial
<220>
   <223> Modified ALEU2-Promotor
<400> 1
   catgctgtcc tttcctcccc ccgctcggaa ggatacgcag aatgctttat cgtggggctg      60
   ccccgggccg aaaccggcat tttgggttga ctcttttaag gaacgacatg cccaggggac     120
   tctccactca caaacccttt caaaaacc                                        148
<210> 2
   <211> 128
   <212> DNA
   <213> Artificial
<220>
   <223> Modified ALEU2-Promotor
<400> 2
   ccgctcggaa ggatacgcag aatgctttat cgtggggctg ccccgggccg aaaccggcat      60
   tttgggttga ctcttttaag gaacgacatg cccaggggac tctccactca caaacccttt     120
   caaaaacc                                                              128
<210> 3
   <211> 120
   <212> DNA
   <213> Artificial
<220>
   <223> Modified ALEU2-Promotor
<400> 3
   aaggatacgc agaatgcttt atcgtggggc tgccccgggc cgaaaccggc attttgggtt 60
   gactctttta aggaacgaca tgcccagggg actctccact cacaaaccct ttcaaaaacc 120
<210> 4
   <211> 111
   <212> DNA
   <213> Artificial
<220>
   <223> Modified ALEU2-Promotor
<400> 4
   cagaatgctt tatcgtgggg ctgccccggg ccgaaaccgg cattttgggt tgactctttt 60
   aaggaacgac atgcccaggg gactctccac tcacaaaccc tttcaaaaac c 111
<210> 5
   <211> 102
   <212> DNA
   <213> Artificial
<220>
   <223> Modified ALEU2-Promotor
<400> 5
   ttatcgtggg gctgccccgg gccgaaaccg gcattttggg ttgactcttt taaggaacga 60
   catgcccagg ggactctcca ctcacaaacc ctttcaaaaa cc 102
<210> 6
   <211> 92
   <212> DNA
   <213> Artificial
<220>
   <223> Modified ALEU2-Promotor
<400> 6
   gctgccccgg gccgaaaccg gcattttggg ttgactcttt taaggaacga catgcccagg 60
   ggactctcca ctcacaaacc ctttcaaaaa cc 92
<210> 7
   <211> 83
   <212> DNA
   <213> Artificial
<220>
   <223> Modified ALEU2-Promotor
<400> 7
   ggccgaaacc ggcattttgg gttgactctt ttaaggaacg acatgcccag gggactctcc 60
   actcacaaac cctttcaaaa acc 83
<210> 8
   <211> 76
   <212> DNA
   <213> Artificial
<220>
<223> Modified ALEU2-Promotor
<400> 8
   accggcattt tgggttgact cttttaagga acgacatgcc caggggactc tccactcaca 60
   aaccctttca aaaacc 76
<210> 9
   <211> 67
   <212> DNA
   <213> Artificial
<220>
   <223> Modified ALEU2-Promotor
<400> 9
   ttgggttgac tcttttaagg aacgacatgc ccaggggact ctccactcac aaaccctttc 60
   aaaaacc 67
<210> 10
   <211> 53
   <212> DNA
   <213> Artificial
<220>
   <223> Modified ALEU2-Promotor
<400> 10
   ttaaggaacg acatgcccag gggactctcc actcacaaac cctttcaaaa acc 53
<210> 11
   <211> 40
   <212> DNA
   <213> Artificial
<220>
   <223> Modified ALEU2-Promotor
<400> 11
   tgcccagggg actctccact cacaaaccct ttcaaaaacc 40
<210> 12
   <211> 28
   <212> DNA
   <213> Artificial
<220>
   <223> Modified ALEU2-Promotor
<400> 12
   tctccactca caaacccttt caaaaacc 28
<210> 13
   <211> 9733
   <212> DNA
   <213> Artificial
<220>
   <223> pGH32
<400> 13
   tcgcgcgttt cggtgatgac ggtgaaaacc tctgacacat gcagctcccg gagacggtca     60
   cagcttgtct gtaagcggat gccgggagca gacaagcccg tcagggcgcg tcagcgggtg    120
   ttggcgggtg tcggggctgg cttaactatg cggcatcaga gcagattgta ctgagagtgc    180
   accatatgcg gtgtgaaata ccgcacagat gcgtaaggag aaaataccgc atcaggcgac    240
   gcgccctgta gcggcgcatt aagcgcggcg ggtgtggtgg ttacgcgcag cgtgaccgct    300
   acacttgcca gcgccctagc gcccgctcct ttcgctttct tcccttcctt tctcgccacg    360
   ttcgccggct ttccccgtca agctctaaat cgggggctcc ctttagggtt ccgatttagt    420
   gctttacggc acctcgaccc caaaaaactt gattagggtg atggttcacg tagtgggcca    480
   tcgccctgat agacggtttt tcgccctttg acgttggagt ccacgttctt taatagtgga    540
   ctcttgttcc aaactggaac aacactcaac cctatctcgg tctattcttt tgatttataa    600
   gggattttgc cgatttcggc ctattggtta aaaaatgagc tgatttaaca aaaatttaac    660
   gcgaatttta acaaaatatt aacgtttaca atttcgccat tcgccattca ggctgcgcaa    720
   ctgttgggaa gggcgatcgg tgcgggcctc ttcgctatta cgccagctgg cgaaaggggg    780
   atgtgctgca aggcgattaa gttgggtaac gccagggttt tcccagtcac gacgttgtaa    840
   aacgacggcc agtgaattgt aatacgactc actatagcca tggccaccgt ccggctgtct    900
   agatgaacta acaccttttc tggtgtctga tgagcgtgca ttccggcacc ttaactctac    960
   gttcggttca tcccgcatcg ccagttctgc ttaccaaaaa tggcccacta aaagctcttc   1020
   attctaatgt ccacgttcaa ttaagtaaca aggacttctt acatattcaa agtttgagaa   1080
   taggtcaaga tcgtttcaat cccaatacct ctaatcattc gctttacctc ataaaactga   1140
   tacgagcttc tgctatcctg agggaaactt cggcaggaac cagctactag atggttcgat   1200
   tagtctttcg cccctatacc caaatttgac gatcgatttg cacgtcagaa tcgctacgag   1260
   cctccaccag agtttcctct ggcttcaccc tattcaggca tagttcacca tctttcgggt   1320
   cccaacagct atgctcttac tcaaatccat ccgagaacat caggatcggt cgatgatgca   1380
   ccctcagggg ttctcatcta cgttcacttt cattacgcgt acgggtttta cacccaaaca   1440
   ctcgcataga cgttagactc cttggtccgt gtttcaagac gggtggctta aagtcattac   1500
   gccaacatcc tagcagatgc gcagtcctcg gtcccagcag gcgatattta caaaggctat   1560
   aacactccat ccgaagatag agccacgttc caatgcattt atctcgccgc caaaaccgat   1620
   gttggcccag taaacagcga gtgcacagtc ccgaaagaca gttgatcact gaataccaag   1680
   tctgacttca agcccttccc tttcaacaat ttcacgtact ttttcactct cttttcaaag   1740
   ttcttttcat ctttccgatc actgtacttg ttcgctatcg gtctctcggc caatatttag   1800
   tcttagatgg aatttaccac ccacttagag ctgcattccc aaacaactcg actcttcgaa 1860
   ggcactttac aaaggaatgg gcatcttacc atgaacggga ttctcaccct ctatgacgtc 1920
   ctgttccaag gaacttatgc aagagccaaa cccaaagtta ccatcttcaa attacaactc 1980
   ggacgcccgt aggacgccag atttcaaatt tggacttttg gccttcgctc gccgttacta 2040
   gggcaatccc ggttggtttc ttttcctccg cttattgata tgcttaagtt cagcgggtag 2100
   tcttgcctga tttgaggtca aacttttaaa agaactgtta taaaggccag ctcagaaaac 2160
   agttactagg ccagcgtagt tggtaaaacc taagacactg taaagcactg aattcgatcg 2220
   agtctgtatt gaagcttagc ttgtataaat agtaaattgt caatcacctc aagtttcaaa 2280
   tgagaagtaa tatagctgta ttctgctgtc agacctatta gtacacatta cctgtcgtca 2340
   gtatcaccta ttgatgctgc tgctggagtg ttaatacttc acagtaaccc ccttggcagc 2400
   tttgacaaac cgctcaatct cgttgagatc tttgaccccc gggcttgatt cgactcctcc 2460
   gctgacatca acaccacagg caccctcgac cgctagagct tgggcaacat tgtcggctgt 2520
   caatcctcca gccataagga acctcgctcc cattcgggcc cgagactgga ctccgctcca 2580
   gtccaccatc ttgccctctc cgccaacctc accgtctatg agggataggc tgtggtatcc 2640
   agtaaccggc gaggcataga aatctggagt atcgggggtg aaccgacgga tcacaggaca 2700
   tggaatcagt ctacaccatt ctaggggctc tgatccgtga agctgaacga aatccagcga 2760
   caattcctgt tgcaaccgca ggacttctga cagcggctgg ttgcggaaca cgcctacgat 2820
   tgctggccgg gtccttgtct tggcttcaat gatgtgggcg agtgcagagt gagtcgatcc 2880
   gcccagctca ttagtgtcca gctcaaagga tccttgacgt ctatatgatc gtaccagtcg 2940
   tgatatcttg cctgctgtct ccatatcaac agtgcgcgct ctattaggga ccataatcat 3000
   tccaatcatg ttggctccag catccaaagc gacctgggcc gcctccacgg tcctgattcc 3060
   gcacaccttg acgattggcg acgttgtcat atcttcagct tgtttgacag gaatgtcctc 3120
   ggcctcggtg tacggagtgg ggatcccaga cgaacttagg tgtacatgaa ctaaagatga 3180
   gtcgactctc agacggacag tcgggcctcg ttgaaagtta ctattccatc aggctttacg 3240
   ctcaagctgt taatcgcctc tagatcgcca tgtccctgaa cttcaatcag actagatagt 3300
   ccattgtttc ggcacttttc aatgttctca actagagtgt cggtatcctt gagctccacc 3360
   gcggcggccc cagcttgcat gcctgcagat tttaatcttt cggcaaaatt tagataaaaa 3420
   atttgggtat tcgtatttag tttccaatat tatttagtta tacaaaacta ttgtctcaat 3480
   agactggcgt tgtaatgagt agtgttcccc tcgactctag ggaattagcg gccgcttatt 3540
   tctgaacata aatggagacg gacccatcgt ttacatgaaa ctctccccag ccgtcagatc 3600
   cgatttttac agtatctgaa cggttgcccg ttatgtcata ccatgtctcg ccggcatttt 3660
   tcaggccggc atacatccgc tttgatccgc cgggtccgtc cgtgattaaa gcggccaaac 3720
   ctgatttggc ggcggagctg tcaccttccc tcgtccatcc gatcacatcc gggtggtcaa 3780
   tataatcgtg ctggggcccg tatgcgtact ccttacgcgc ttttaaaatc ggctctatat 3840
   tatctttcag tgagggaatt tcctttggcg atgtcccttt tgtcccgtac atatccccat 3900
   agaacacctg aggataaccg gattctcttg tcaaaataaa ggcgtatgca agcggtttaa 3960
   accaagtttg gactgtcgat tccaatgact gtcccggctg tgtgtcatga ttttcaacaa 4020
   atgtaaccgc cttttccgga tgcctggaca caacggtacc gtccagcaaa cgcctcatat 4080
   catatccgcc tccttgtgag gaagccgcct gtaaattgaa atgaagcgga acatcaaaca 4140
   cggattgatt aaagcttgtt ttattcaagt agttttcgag tttcccggca ttattctgcc 4200
   aatactccgc aaccgtaaac atttcttttc ccgtcgcctg tctgaccgcc tgaacccaat 4260
   cacgcagaaa tgaaaattta atatgtttgg cggcatcaat acggaagccg tctaatgaca 4320
   gttcattcgc ataccagata ccccattttt ttgtctctgc cacgacatca gggtggtcgt 4380
   agtcaacatc agcatacatt aaatagtcat agttgccgtt ttcacttgat acttcccaat 4440
   cccacgcttt tccttcccca cgaaacttaa agatgcggct gatcttccgg gattcatccc 4500
   agtccgctcc gtcgaaatga taccaatgcc atttaaaatc actgtacgtg tttccacggc 4560
   ccggaaaacg aaaatccgtc cacgctttga tttgatattc ctccgaagtt tcctgatttc 4620
   tattggccgg attgacttcg acggcagtta catcttctgt tgcatcagca ccagccttat 4680
   gattcaaaac cacatctccg tatacttgga cgttccggga atgcagtgag ccgatcgcat 4740
   cttgaagctc tgattttgtg ccgtatttcg ttctgaccgt ccctttttgc tggaattctc 4800
   ctaaatcata caaatcataa ggtccgtatc cgttatcgga ttggctcaat cctttgtatg 4860
   cgggaggaat ccagacggca gtgattccga tatccgataa atgttccgca tcattctgca 4920
   atcgtttcca atgctggccg tcgttcggcg tataccattc aaaatactgc atcagcgtgc 4980
   catttacggc tgatgttttt gtaatcggca aactgacaaa taacagcgtg cacataagca 5040
   caagtctgaa cgaaactgtc cgctttcgtt tttgaatcat ggatccagag aattctgttg 5100
   attatgtttt taagaactac tcagaatgaa atgccttgat ttccggtgtg agttgacaag 5160
   gaaaaaaatt ccaggagcag ggggagcttt atataacacc agattagcgg cgaaaaattt 5220
   tttttctcga ggggttaccc ggtcgtcctt tgcctaatgg ggaaacatcc cgctcgccgc 5280
   acgtaccact tagggctccc caaaaagcaa ccatcctccg ctcatctcga gggagatcac 5340
   taaacgatat caactttgag ggctcttgtt gactatggac tgattataga ttgaagtcga 5400
   gactagtgga tcccccgggc tgcaggaatt ctgcttcggt atgataggaa gagccgacat 5460
   cgaaggatca aaaagcaatg tcgctatgaa cgcttgactg ccacaagcca gttccgtact 5520
   accatgacca actacttctc aggttgtgcg cccggtaggt agttgcaccc tctcgggtgg 5580
   cgtagcccgt accttaagcc gaccattaat ggtcaaccca catcttagcg agcgttgaac 5640
   tgcctagcta ctttgtaaat atacttttgg ccaggtcagc tgcggattgt ctctattctt 5700
   tggattttta cccgtctgcc ccaacagact ctacattggg gtgttaccgc caatgtttgg 5760
   taccaaagac ctagcgagag ttccccgcat ccagacgtgt tgcccttaca ctcttctaga 5820
   gagctaacta tagcaagcac aagtaagcta tagccggact aggcatcaat gggagtcgat 5880
   gcctgggacg ttatgttgta aatttgtctt aatagactta gcacatccct gtggtaactt 5940
   ttctggcacc tctagcctca aattctgagg gactaaagga tcgataggcc acactttcat 6000
   ggtttgtatt cacactgaaa atcaaaatca aggggacttt tacccttttg ttctactgga   6060
   gatttctgtt ctccatgagt cccccttagg acatctgcgt tatcttttaa cagatgtgcc   6120
   gccccagcca aactccccac ctgacaatgt cttcaacccg gatcagcccc gaaggacctt 6180
   aagtctagaa gttggacggt gaagtccagt tccgcttaat tgaataagta aaaaaacgat 6240
   aaaggtagtg gtatttcacc ggcgccgaag ctcccactta ttctacaccc tctatgtctt 6300
   ttcacaatgt caaactagag tcaagctcaa cagggtcttc tttccccgct gattctgcca 6360
   agcccgttcc cttggctgtg gtttcgctag atagtagata gggacagtgg gaatctcgtt 6420
   aatccattca tgcgcgtcac taattagatg acgaggcatt tggctacctt aagagagtca 6480
   tagttactcc cgccgtttac ccgcgcttgg ttgaatttct tcactttgac attcagagca 6540
   ctgggcagaa atcacattcc gtcaacatca ctttctgacc atcggaatgc tatgttttaa 6600
   ttagacagtc agattcccct tgtccgtacc agttctaagt tagtcgttcg gtgtgaatcg 6660
   gacggtcgaa aagacctgcc aagcacgtcc gccgaaggat cccagtcagc tccacacaag 6720
   gttgcccctg cgcagttccg tccgggttcc ccgggttctg aaacaaggcc cgaaacactc 6780
   gacccttaga gccaatcctt atcccgaagt tacggatcta ttttgccgac ttcccttacc 6840
   tacattattc tatcaactag aggctgttca ccttggagac ctgctgcggt tatgagtacg 6900
   acttggcatg aaaacaaaac cttccagtgg attttcaagg gacgtcataa gcgcaccgga 6960
   cctgacagag gtgtcaggct cttccagccc taagacccta gctctggaca aaccaatttc 7020
   agggtgatag actgttaaga agaaaagata actcctccca gggcttatgc cgacgtctcc 7080
   actttcagtt acgttaccgt gaagattcca tatccaagtt ccggaatatt aaccggattc 7140
   cctttcgatg gtggctcgaa aaatcgagca cattgaaacg gagcttcccc atctcttagg 7200
   atcgactaac ccgtggccaa ctgctgttca cacggaacct ttccccactt cagtcttcaa 7260
   agttctcatt tgaatatttg ctactaccac caagatctgc actagaggcc gttcgaccca 7320
   gcatcactgc aaaggcttcg tcactgacct ccacgcctgc ctactcgtta gggcatcgca 7380
   ttaacctaac ggtagagtat aggtaacacg cttgagcgcc atccattttc agggctagtt 7440
   cattcggccg gtgagttgtt acacactcct tagcggattc cgacttccat ggcgagcttg 7500
   gcgtaatcat ggtcatagct gtttcctgtg tgaaattgtt atccgctcac aattccacac 7560
   aacatacgag ccggaagcat aaagtgtaaa gcctggggtg cctaatgagt gagctaactc 7620
   acattaattg cgttgcgctc actgcccgct ttccagtcgg gaaacctgtc gtgccagctg 7680
   cattaatgaa tcggccaacg cgcggggaga ggcggtttgc gtattgggcg ctcttccgct 7740
   tcctcgctca ctgactcgct gcgctcggtc gttcggctgc ggcgagcggt atcagctcac 7800
   tcaaaggcgg taatacggtt atccacagaa tcaggggata acgcaggaaa gaacatgtga 7860
   gcaaaaggcc agcaaaaggc caggaaccgt aaaaaggccg cgttgctggc gtttttccat 7920
   aggctccgcc cccctgacga gcatcacaaa aatcgacgct caagtcagag gtggcgaaac 7980
   ccgacaggac tataaagata ccaggcgttt ccccctggaa gctccctcgt gcgctctcct 8040
   gttccgaccc tgccgcttac cggatacctg tccgcctttc tcccttcggg aagcgtggcg 8100
   ctttctcata gctcacgctg taggtatctc agttcggtgt aggtcgttcg ctccaagctg 8160
   ggctgtgtgc acgaaccccc cgttcagccc gaccgctgcg ccttatccgg taactatcgt 8220
   cttgagtcca acccggtaag acacgactta tcgccactgg cagcagccac tggtaacagg 8280
   attagcagag cgaggtatgt aggcggtgct acagagttct tgaagtggtg gcctaactac 8340
   ggctacacta gaaggacagt atttggtatc tgcgctctgc tgaagccagt taccttcgga 8400
   aaaagagttg gtagctcttg atccggcaaa caaaccaccg ctggtagcgg tggttttttt 8460
   gtttgcaagc agcagattac gcgcagaaaa aaaggatctc aagaagatcc tttgatcttt 8520
   tctacggggt ctgacgctca gtggaacgaa aactcacgtt aagggatttt ggtcatgaga 8580
   ttatcaaaaa ggatcttcac ctagatcctt ttaaattaaa aatgaagttt taaatcaatc 8640
   taaagtatat atgagtaaac ttggtctgac agttaccaat gcttaatcag tgaggcacct 8700
   atctcagcga tctgtctatt tcgttcatcc atagttgcct gactccccgt cgtgtagata 8760
   actacgatac gggagggctt accatctggc cccagtgctg caatgatacc gcgagaccca 8820
   cgctcaccgg ctccagattt atcagcaata aaccagccag ccggaagggc cgagcgcaga 8880
   agtggtcctg caactttatc cgcctccatc cagtctatta attgttgccg ggaagctaga 8940
   gtaagtagtt cgccagttaa tagtttgcgc aacgttgttg ccattgctac aggcatcgtg 9000
   gtgtcacgct cgtcgtttgg tatggcttca ttcagctccg gttcccaacg atcaaggcga 9060
   gttacatgat cccccatgtt gtgcaaaaaa gcggttagct ccttcggtcc tccgatcgtt 9120
   gtcagaagta agttggccgc agtgttatca ctcatggtta tggcagcact gcataattct 9180
   cttactgtca tgccatccgt aagatgcttt tctgtgactg gtgagtactc aaccaagtca 9240
   ttctgagaat agtgtatgcg gcgaccgagt tgctcttgcc cggcgtcaat acgggataat 9300
   accgcgccac atagcagaac tttaaaagtg ctcatcattg gaaaacgttc ttcggggcga 9360
   aaactctcaa ggatcttacc gctgttgaga tccagttcga tgtaacccac tcgtgcaccc 9420
   aactgatctt cagcatcttt tactttcacc agcgtttctg ggtgagcaaa aacaggaagg 9480
   caaaatgccg caaaaaaggg aataagggcg acacggaaat gttgaatact catactcttc 9540
   ctttttcaat attattgaag catttatcag ggttattgtc tcatgagcgg atacatattt 9600
   gaatgtattt agaaaaataa acaaataggg gttccgcgca catttccccg aaaagtgcca 9660
   cctgacgtct aagaaaccat tattatcatg acattaacct ataaaaatag gcgtatcacg 9720
   aggccctttc gtc 9733
<210> 14
   <211> 9733
   <212> DNA
   <213> Artificial
<220>
   <223> pGH32RA
<400> 14
   tcgcgcgttt cggtgatgac ggtgaaaacc tctgacacat gcagctcccg gagacggtca 60
   cagcttgtct gtaagcggat gccgggagca gacaagcccg tcagggcgcg tcagcgggtg 120
   ttggcgggtg tcggggctgg cttaactatg cggcatcaga gcagattgta ctgagagtgc 180
   accatatgcg gtgtgaaata ccgcacagat gcgtaaggag aaaataccgc atcaggcgac 240
   gcgccctgta gcggcgcatt aagcgcggcg ggtgtggtgg ttacgcgcag cgtgaccgct 300
   acacttgcca gcgccctagc gcccgctcct ttcgctttct tcccttcctt tctcgccacg 360
   ttcgccggct ttccccgtca agctctaaat cgggggctcc ctttagggtt ccgatttagt 420
   gctttacggc acctcgaccc caaaaaactt gattagggtg atggttcacg tagtgggcca 480
   tcgccctgat agacggtttt tcgccctttg acgttggagt ccacgttctt taatagtgga 540
   ctcttgttcc aaactggaac aacactcaac cctatctcgg tctattcttt tgatttataa 600
   gggattttgc cgatttcggc ctattggtta aaaaatgagc tgatttaaca aaaatttaac 660
   gcgaatttta acaaaatatt aacgtttaca atttcgccat tcgccattca ggctgcgcaa 720
   ctgttgggaa gggcgatcgg tgcgggcctc ttcgctatta cgccagctgg cgaaaggggg 780
   atgtgctgca aggcgattaa gttgggtaac gccagggttt tcccagtcac gacgttgtaa 840
   aacgacggcc agtgaattgt aatacgactc actatagcca tggccaccgt ccggctgtct 900
   agatgaacta acaccttttc tggtgtctga tgagcgtgca ttccggcacc ttaactctac 960
   gttcggttca tcccgcatcg ccagttctgc ttaccaaaaa tggcccacta aaagctcttc 1020
   attctaatgt ccacgttcaa ttaagtaaca aggacttctt acatattcaa agtttgagaa 1080
   taggtcaaga tcgtttcaat cccaatacct ctaatcattc gctttacctc ataaaactga 1140
   tacgagcttc tgctatcctg agggaaactt cggcaggaac cagctactag atggttcgat 1200
   tagtctttcg cccctatacc caaatttgac gatcgatttg cacgtcagaa tcgctacgag 1260
   cctccaccag agtttcctct ggcttcaccc tattcaggca tagttcacca tctttcgggt 1320
   cccaacagct atgctcttac tcaaatccat ccgagaacat caggatcggt cgatgatgca 1380
   ccctcagggg ttctcatcta cgttcacttt cattacgcgt acgggtttta cacccaaaca 1440
   ctcgcataga cgttagactc cttggtccgt gtttcaagac gggtggctta aagtcattac 1500
   gccaacatcc tagcagatgc gcagtcctcg gtcccagcag gcgatattta caaaggctat 1560
   aacactccat ccgaagatag agccacgttc caatgcattt atctcgccgc caaaaccgat 1620
   gttggcccag taaacagcga gtgcacagtc ccgaaagaca gttgatcact gaataccaag 1680
   tctgacttca agcccttccc tttcaacaat ttcacgtact ttttcactct cttttcaaag 1740
   ttcttttcat ctttccgatc actgtacttg ttcgctatcg gtctctcggc caatatttag 1800
   tcttagatgg aatttaccac ccacttagag ctgcattccc aaacaactcg actcttcgaa 1860
   ggcactttac aaaggaatgg gcatcttacc atgaacggga ttctcaccct ctatgacgtc 1920
   ctgttccaag gaacttatgc aagagccaaa cccaaagtta ccatcttcaa attacaactc 1980
   ggacgcccgt aggacgccag atttcaaatt tggacttttg gccttcgctc gccgttacta 2040
   gggcaatccc ggttggtttc ttttcctccg cttattgata tgcttaagtt cagcgggtag 2100
   tcttgcctga tttgaggtca aacttttaaa agaactgtta taaaggccag ctcagaaaac 2160
   agttactagg ccagcgtagt tggtaaaacc taagacactg taaagcactg aattcgatcg 2220
   agtctgtatt gaagcttagc ttgtataaat agtaaattgt caatcacctc aagtttcaaa 2280
   tgagaagtaa tatagctgta ttctgctgtc agacctatta gtacacatta cctgtcgtca 2340
   gtatcaccta ttgatgctgc tgctggagtg ttaatacttc acagtaaccc ccttggcagc 2400
   tttgacaaac cgctcaatct cgttgagatc tttgaccccc gggcttgatt cgactcctcc 2460
   gctgacatca acaccacagg caccctcgac cgctagagct tgggcaacat tgtcggctgt 2520
   caatcctcca gccataagga acctcgctcc cattcgggcc cgagactgga ctccgctcca 2580
   gtccaccatc ttgccctctc cgccaacctc accgtctatg agggataggc tgtggtatcc 2640
   agtaaccggc gaggcataga aatctggagt atcgggggtg aaccgacgga tcacaggaca 2700
   tggaatcagt ctacaccatt ctaggggctc tgatccgtga agctgaacga aatccagcga 2760
   caattcctgt tgcaaccgca ggacttctga cagcggctgg ttgcggaaca cgcctacgat 2820
   tgctggccgg gtccttgtct tggcttcaat gatgtgggcg agtgcagagt gagtcgatcc 2880
   gcccagctca ttagtgtcca gctcaaagga tccttgacgt ctatatgatc gtaccagtcg 2940
   tgatatcttg cctgctgtct ccatatcaac agtgcgcgct ctattaggga ccataatcat 3000
   tccaatcatg ttggctccag catccaaagc gacctgggcc gcctccacgg tcctgattcc 3060
   gcacaccttg acgattggcg acgttgtcat atcttcagct tgtttgacag gaatgtcctc 3120
   ggcctcggtg tacggagtgg ggatcccaga cgaacttagg tgtacatgaa ctaaagatga 3180
   gtcgactctc agacggacag tcgggcctcg ttgaaagtta ctattccatc aggctttacg 3240
   ctcaagctgt taatcgcctc tagatcgcca tgtccctgaa cttcaatcag actagatagt 3300
   ccattgtttc ggcacttttc aatgttctca actagagtgt cggtatcctt gagctccacc 3360
   gcggcggccc cagcttgcat gcctgcagcc cgggggatcc actagtctcg acttcaatct 3420
   ataatcagtc catagtcaac aagagccctc aaagttgata tcgtttagtg atctccctcg 3480
   agatgagcgg aggatggttg ctttttgggg agccctaagt ggtacgtgcg gcgagcggga 3540
   tgtttcccca ttaggcaaag gacgaccggg taacccctcg agaaaaaaaa tttttcgccg 3600
   ctaatctggt gttatataaa gctccccctg ctcctggaat ttttttcctt gtcaactcac 3660
   accggaaatc aaggcatttc attctgagta gttcttaaaa acataatcaa cagaattctc 3720
   tggatccatg attcaaaaac gaaagcggac agtttcgttc agacttgtgc ttatgtgcac 3780
   gctgttattt gtcagtttgc cgattacaaa aacatcagcc gtaaatggca cgctgatgca 3840
   gtattttgaa tggtatacgc cgaacgacgg ccagcattgg aaacgattgc agaatgatgc 3900
   ggaacattta tcggatatcg gaatcactgc cgtctggatt cctcccgcat acaaaggatt 3960
   gagccaatcc gataacggat acggacctta tgatttgtat gatttaggag aattccagca 4020
   aaaagggacg gtcagaacga aatacggcac aaaatcagag cttcaagatg cgatcggctc 4080
   actgcattcc cggaacgtcc aagtatacgg agatgtggtt ttgaatcata aggctggtgc 4140
   tgatgcaaca gaagatgtaa ctgccgtcga agtcaatccg gccaatagaa atcaggaaac 4200
   ttcggaggaa tatcaaatca aagcgtggac ggattttcgt tttccgggcc gtggaaacac 4260
   gtacagtgat tttaaatggc attggtatca tttcgacgga gcggactggg atgaatcccg 4320
   gaagatcagc cgcatcttta agtttcgtgg ggaaggaaaa gcgtgggatt gggaagtatc 4380
   aagtgaaaac ggcaactatg actatttaat gtatgctgat gttgactacg accaccctga 4440
   tgtcgtggca gagacaaaaa aatggggtat ctggtatgcg aatgaactgt cattagacgg 4500
   cttccgtatt gatgccgcca aacatattaa attttcattt ctgcgtgatt gggttcaggc 4560
   ggtcagacag gcgacgggaa aagaaatgtt tacggttgcg gagtattggc agaataatgc 4620
   cgggaaactc gaaaactact tgaataaaac aagctttaat caatccgtgt ttgatgttcc 4680
   gcttcatttc aatttacagg cggcttcctc acaaggaggc ggatatgata tgaggcgttt 4740
   gctggacggt accgttgtgt ccaggcatcc ggaaaaggcg gttacatttg ttgaaaatca 4800
   tgacacacag ccgggacagt cattggaatc gacagtccaa acttggttta aaccgcttgc 4860
   atacgccttt attttgacaa gagaatccgg ttatcctcag gtgttctatg gggatatgta 4920
   cgggacaaaa gggacatcgc caaaggaaat tccctcactg aaagataata tagagccgat 4980
   tttaaaagcg cgtaaggagt acgcatacgg gccccagcac gattatattg accacccgga 5040
   tgtgatcgga tggacgaggg aaggtgacag ctccgccgcc aaatcaggtt tggccgcttt 5100
   aatcacggac ggacccggcg gatcaaagcg gatgtatgcc ggcctgaaaa atgccggcga 5160
   gacatggtat gacataacgg gcaaccgttc agatactgta aaaatcggat ctgacggctg 5220
   gggagagttt catgtaaacg atgggtccgt ctccatttat gttcagaaat aagcggccgc 5280
   taattcccta gagtcgaggg gaacactact cattacaacg ccagtctatt gagacaatag 5340
   ttttgtataa ctaaataata ttggaaacta aatacgaata cccaaatttt ttatctaaat 5400
   tttgccgaaa gattaaaatc tgcaggaatt ctgcttcggt atgataggaa gagccgacat 5460
   cgaaggatca aaaagcaatg tcgctatgaa cgcttgactg ccacaagcca gttccgtact 5520
   accatgacca actacttctc aggttgtgcg cccggtaggt agttgcaccc tctcgggtgg 5580
   cgtagcccgt accttaagcc gaccattaat ggtcaaccca catcttagcg agcgttgaac 5640
   tgcctagcta ctttgtaaat atacttttgg ccaggtcagc tgcggattgt ctctattctt 5700
   tggattttta cccgtctgcc ccaacagact ctacattggg gtgttaccgc caatgtttgg 5760
   taccaaagac ctagcgagag ttccccgcat ccagacgtgt tgcccttaca ctcttctaga 5820
   gagctaacta tagcaagcac aagtaagcta tagccggact aggcatcaat gggagtcgat 5880
   gcctgggacg ttatgttgta aatttgtctt aatagactta gcacatccct gtggtaactt 5940
   ttctggcacc tctagcctca aattctgagg gactaaagga tcgataggcc acactttcat 6000
   ggtttgtatt cacactgaaa atcaaaatca aggggacttt tacccttttg ttctactgga   6060
   gatttctgtt ctccatgagt cccccttagg acatctgcgt tatcttttaa cagatgtgcc   6120
   gccccagcca aactccccac ctgacaatgt cttcaacccg gatcagcccc gaaggacctt 6180
   aagtctagaa gttggacggt gaagtccagt tccgcttaat tgaataagta aaaaaacgat 6240
   aaaggtagtg gtatttcacc ggcgccgaag ctcccactta ttctacaccc tctatgtctt 6300
   ttcacaatgt caaactagag tcaagctcaa cagggtcttc tttccccgct gattctgcca 6360
   agcccgttcc cttggctgtg gtttcgctag atagtagata gggacagtgg gaatctcgtt 6420
   aatccattca tgcgcgtcac taattagatg acgaggcatt tggctacctt aagagagtca 6480
   tagttactcc cgccgtttac ccgcgcttgg ttgaatttct tcactttgac attcagagca 6540
   ctgggcagaa atcacattcc gtcaacatca ctttctgacc atcggaatgc tatgttttaa 6600
   ttagacagtc agattcccct tgtccgtacc agttctaagt tagtcgttcg gtgtgaatcg 6660
   gacggtcgaa aagacctgcc aagcacgtcc gccgaaggat cccagtcagc tccacacaag 6720
   gttgcccctg cgcagttccg tccgggttcc ccgggttctg aaacaaggcc cgaaacactc 6780
   gacccttaga gccaatcctt atcccgaagt tacggatcta ttttgccgac ttcccttacc 6840
   tacattattc tatcaactag aggctgttca ccttggagac ctgctgcggt tatgagtacg 6900
   acttggcatg aaaacaaaac cttccagtgg attttcaagg gacgtcataa gcgcaccgga 6960
   cctgacagag gtgtcaggct cttccagccc taagacccta gctctggaca aaccaatttc 7020
   agggtgatag actgttaaga agaaaagata actcctccca gggcttatgc cgacgtctcc 7080
   actttcagtt acgttaccgt gaagattcca tatccaagtt ccggaatatt aaccggattc 7140
   cctttcgatg gtggctcgaa aaatcgagca cattgaaacg gagcttcccc atctcttagg 7200
   atcgactaac ccgtggccaa ctgctgttca cacggaacct ttccccactt cagtcttcaa 7260
   agttctcatt tgaatatttg ctactaccac caagatctgc actagaggcc gttcgaccca 7320
   gcatcactgc aaaggcttcg tcactgacct ccacgcctgc ctactcgtta gggcatcgca 7380
   ttaacctaac ggtagagtat aggtaacacg cttgagcgcc atccattttc agggctagtt 7440
   cattcggccg gtgagttgtt acacactcct tagcggattc cgacttccat ggcgagcttg 7500
   gcgtaatcat ggtcatagct gtttcctgtg tgaaattgtt atccgctcac aattccacac 7560
   aacatacgag ccggaagcat aaagtgtaaa gcctggggtg cctaatgagt gagctaactc 7620
   acattaattg cgttgcgctc actgcccgct ttccagtcgg gaaacctgtc gtgccagctg 7680
   cattaatgaa tcggccaacg cgcggggaga ggcggtttgc gtattgggcg ctcttccgct 7740
   tcctcgctca ctgactcgct gcgctcggtc gttcggctgc ggcgagcggt atcagctcac 7800
   tcaaaggcgg taatacggtt atccacagaa tcaggggata acgcaggaaa gaacatgtga 7860
   gcaaaaggcc agcaaaaggc caggaaccgt aaaaaggccg cgttgctggc gtttttccat 7920
   aggctccgcc cccctgacga gcatcacaaa aatcgacgct caagtcagag gtggcgaaac 7980
   ccgacaggac tataaagata ccaggcgttt ccccctggaa gctccctcgt gcgctctcct 8040
   gttccgaccc tgccgcttac cggatacctg tccgcctttc tcccttcggg aagcgtggcg 8100
   ctttctcata gctcacgctg taggtatctc agttcggtgt aggtcgttcg ctccaagctg 8160
   ggctgtgtgc acgaaccccc cgttcagccc gaccgctgcg ccttatccgg taactatcgt 8220
   cttgagtcca acccggtaag acacgactta tcgccactgg cagcagccac tggtaacagg 8280
   attagcagag cgaggtatgt aggcggtgct acagagttct tgaagtggtg gcctaactac 8340
   ggctacacta gaaggacagt atttggtatc tgcgctctgc tgaagccagt taccttcgga 8400
   aaaagagttg gtagctcttg atccggcaaa caaaccaccg ctggtagcgg tggttttttt 8460
   gtttgcaagc agcagattac gcgcagaaaa aaaggatctc aagaagatcc tttgatcttt 8520
   tctacggggt ctgacgctca gtggaacgaa aactcacgtt aagggatttt ggtcatgaga 8580
   ttatcaaaaa ggatcttcac ctagatcctt ttaaattaaa aatgaagttt taaatcaatc 8640
   taaagtatat atgagtaaac ttggtctgac agttaccaat gcttaatcag tgaggcacct 8700
   atctcagcga tctgtctatt tcgttcatcc atagttgcct gactccccgt cgtgtagata 8760
   actacgatac gggagggctt accatctggc cccagtgctg caatgatacc gcgagaccca 8820
   cgctcaccgg ctccagattt atcagcaata aaccagccag ccggaagggc cgagcgcaga 8880
   agtggtcctg caactttatc cgcctccatc cagtctatta attgttgccg ggaagctaga 8940
   gtaagtagtt cgccagttaa tagtttgcgc aacgttgttg ccattgctac aggcatcgtg 9000
   gtgtcacgct cgtcgtttgg tatggcttca ttcagctccg gttcccaacg atcaaggcga 9060
   gttacatgat cccccatgtt gtgcaaaaaa gcggttagct ccttcggtcc tccgatcgtt 9120
   gtcagaagta agttggccgc agtgttatca ctcatggtta tggcagcact gcataattct 9180
   cttactgtca tgccatccgt aagatgcttt tctgtgactg gtgagtactc aaccaagtca 9240
   ttctgagaat agtgtatgcg gcgaccgagt tgctcttgcc cggcgtcaat acgggataat 9300
   accgcgccac atagcagaac tttaaaagtg ctcatcattg gaaaacgttc ttcggggcga 9360
   aaactctcaa ggatcttacc gctgttgaga tccagttcga tgtaacccac tcgtgcaccc 9420
   aactgatctt cagcatcttt tactttcacc agcgtttctg ggtgagcaaa aacaggaagg 9480
   caaaatgccg caaaaaaggg aataagggcg acacggaaat gttgaatact catactcttc 9540
   ctttttcaat attattgaag catttatcag ggttattgtc tcatgagcgg atacatattt 9600
   gaatgtattt agaaaaataa acaaataggg gttccgcgca catttccccg aaaagtgcca 9660
   cctgacgtct aagaaaccat tattatcatg acattaacct ataaaaatag gcgtatcacg 9720
   aggccctttc gtc 9733
<210> 15
   <211> 7288
   <212> DNA
   <213> Artificial
<220>
   <223> pGH69
<400> 15
   tcgcgcgttt cggtgatgac ggtgaaaacc tctgacacat gcagctcccg gagacggtca 60
   cagcttgtct gtaagcggat gccgggagca gacaagcccg tcagggcgcg tcagcgggtg 120
   ttggcgggtg tcggggctgg cttaactatg cggcatcaga gcagattgta ctgagagtgc 180
   accatatgcg gtgtgaaata ccgcacagat gcgtaaggag aaaataccgc atcaggcgac 240
   gcgccctgta gcggcgcatt aagcgcggcg ggtgtggtgg ttacgcgcag cgtgaccgct 300
   acacttgcca gcgccctagc gcccgctcct ttcgctttct tcccttcctt tctcgccacg 360
   ttcgccggct ttccccgtca agctctaaat cgggggctcc ctttagggtt ccgatttagt 420
   gctttacggc acctcgaccc caaaaaactt gattagggtg atggttcacg tagtgggcca 480
   tcgccctgat agacggtttt tcgccctttg acgttggagt ccacgttctt taatagtgga 540
   ctcttgttcc aaactggaac aacactcaac cctatctcgg tctattcttt tgatttataa    600
   gggattttgc cgatttcggc ctattggtta aaaaatgagc tgatttaaca aaaatttaac    660
   gcgaatttta acaaaatatt aacgtttaca atttcgccat tcgccattca ggctgcgcaa    720
   ctgttgggaa gggcgatcgg tgcgggcctc ttcgctatta cgccagctgg cgaaaggggg    780
   atgtgctgca aggcgattaa gttgggtaac gccagggttt tcccagtcac gacgttgtaa    840
   aacgacggcc agtgaattgt aatacgactc actatagcca tggccaccgt ccggctgtct    900
   agatgaacta acaccttttc tggtgtctga tgagcgtgca ttccggcacc ttaactctac    960
   gttcggttca tcccgcatcg ccagttctgc ttaccaaaaa tggcccacta aaagctcttc   1020
   attctaatgt ccacgttcaa ttaagtaaca aggacttctt acatattcaa agtttgagaa   1080
   taggtcaaga tcgtttcaat cccaatacct ctaatcattc gctttacctc ataaaactga   1140
   tacgagcttc tgctatcctg agggaaactt cggcaggaac cagctactag atggttcgag   1200
   tcgacgaagc agaattcctg cagattttaa tctttcggca aaatttagta aaaaatttgg   1260
   gtattcgtat ttagtttcca atattattta gttatacaaa actattgtct caatagactg   1320
   gcgttgtaat gagtagtgtt cccctcgact ctagggaatt agcggccgct tatttctgaa   1380
   cataaatgga gacggaccca tcgtttacat gaaactctcc ccagccgtca gatccgattt   1440
   ttacagtatc tgaacggttg cccgttatgt cataccatgt ctcgccggca tttttcaggc   1500
   cggcatacat ccgctttgat ccgccgggtc cgtccgtgat taaagcggcc aaacctgatt   1560
   tggcggcgga gctgtcacct tccctcgtcc atccgatcac atccgggtgg tcaatataat   1620
   cgtgctgggg cccgtatgcg tactccttac gcgcttttaa aatcggctct atattatctt   1680
   tcagtgaggg aatttccttt ggcgatgtcc cttttgtccc gtacatatcc ccatagaaca   1740
   cctgaggata accggattct cttgtcaaaa taaaggcgta tgcaagcggt ttaaaccaag   1800
   tttggactgt cgattccaat gactgtcccg gctgtgtgtc atgattttca acaaatgtaa   1860
   ccgccttttc cggatgcctg gacacaacgg taccgtccag caaacgcctc atatcatatc   1920
   cgcctccttg tgaggaagcc gcctgtaaat tgaaatgaag cggaacatca aacacggatt   1980
   gattaaagct tgttttattc aagtagtttt cgagtttccc ggcattattc tgccaatact   2040
   ccgcaaccgt aaacatttct tttcccgtcg cctgtctgac cgcctgaacc caatcacgca   2100
   gaaatgaaaa tttaatatgt ttggcggcat caatacggaa gccgtctaat gacagttcat   2160
   tcgcatacca gataccccat ttttttgtct ctgccacgac atcagggtgg tcgtagtcaa   2220
   catcagcata cattaaatag tcatagttgc cgttttcact tgatacttcc caatcccacg   2280
   cttttccttc cccacgaaac ttaaagatgc ggctgatctt ccgggattca tcccagtccg   2340
   ctccgtcgaa atgataccaa tgccatttaa aatcactgta cgtgtttcca cggcccggaa   2400
   aacgaaaatc cgtccacgct ttgatttgat attcctccga agtttcctga tttctattgg 2460
   ccggattgac ttcgacggca gttacatctt ctgttgcatc agcaccagcc ttatgattca 2520
   aaaccacatc tccgtatact tggacgttcc gggaatgcag tgagccgatc gcatcttgaa 2580
   gctctgattt tgtgccgtat ttcgttctga ccgtcccttt ttgctggaat tctcctaaat 2640
   catacaaatc ataaggtccg tatccgttat cggattggct caatcctttg tatgcgggag 2700
   gaatccagac ggcagtgatt ccgatatccg ataaatgttc cgcatcattc tgcaatcgtt 2760
   tccaatgctg gccgtcgttc ggcgtatacc attcaaaata ctgcatcagc gtgccattta 2820
   cggctgatgt ttttgtaatc ggcaaactga caaataacag cgtgcacata agcacaagtc 2880
   tgaacgaaac tgtccgcttt cgtttttgaa tcatggatcc agagaattct gttgattatg 2940
   tttttaagaa ctactcagaa tgaaatgcct tgatttccgg tgtgagttga caaggaaaaa 3000
   aattccagga gcagggggag ctttatataa caccagatta gcggcgaaaa attttttttc 3060
   tcgaggggtt acccggtcgt cctttgccta atggggaaac atcccgctcg ccgcacgtac 3120
   cacttagggc tccccaaaaa gcaaccatcc tccgctcatc tcgagggaga tcactaaacg 3180
   atatcaactt tgagggctct tgttgactat ggactgatta tagattgaag tcgagactag 3240
   tggatccccc gggctgcagg catgcaagct ggggccgccg cggttttcaa tcgacgaatt 3300
   gcaattgaca aagagagaac atgctcttaa gactgaacat cttatcagac aagggccgca 3360
   tcgagcggct acctcgtaat tgtggtcagt cattgacaac cattagcaag ccattggcaa 3420
   cgggacaacg caggcgaatc tcactcgcag ttactgcctg caatgacaat tgctcctctt 3480
   cgcgcaaaca taatagactt tgataagcac cacaatcgct tcttccctcg gtcgtactgg 3540
   caaatttcat gcatgaatgc ggctgcagcc tcctgtcgat atgggacgga gctctgttgc 3600
   tttcggcgcc ctttccaagc ttcaatccac taatcggaga atgttcatca actggctcag 3660
   ctaatcagat aggtcaactt gttccccagc ggtttcacca catgctgtcc tttcctcccc 3720
   ccgctcggaa ggatacgcag aatgctttat cgtggggctg ccccgggccg aaaccggcat 3780
   tttgggttga ctcttttaag gaacgacatg cccaggggac tctccactca caaacccttt 3840
   caaaaacctg tacaatgaca acgtckgcca atcgtcaagg tgtgcggaat caggaccgtg 3900
   gaggcggccc aggtcgcttt ggatgctgga gccaacatga ttggaatgat tatggtccct 3960
   aatagagcgc gcactgttga tatggagaca gcaggcaaga tatcacgact ggtacgatca 4020
   tatagacgtc aaggatcctt tgagctggac actaatgagc tgggcggatc gactcactct 4080
   gcactcgccc acatcattga agccaagaca aggacccggc cagcaatcgt aggcgtgttc 4140
   cgcaaccagc cgctgtcaga agtcctgcgg ttgcaacagg aattgtcgct ggatttcgtt 4200
   cagcttcacg gatcagagcc cctagaatgg tgtagactga ttccatgtcc tgtgatccgt 4260
   cggttcaccc ccgatactcc agatttctat gcctcgccgg ttactggata ccacagccta 4320
   tccctcatag acggtgaggt tggcggagag ggcaagatgg tggactggag cggagtccag 4380
   tctcgggccc gaatgggagc gaggttcctt atggctggag gattgacagc cgacaatgtt 4440
   gcccaagctc tagcggtcga gggtgcctgt ggtgttgatg tcagcggagg agtcgaatca 4500
   agcccggggg tcaaagatct caacgagatt gagcggtttg tcaaagctgc caagggggtt 4560
   actgtgaagt attaacactc cagcagcagc atcaataggt gatactgacg acaggtaatg 4620
   tgtactaata ggtctgacag cagaatacag ctatattact tctcatttga aacttgaggt 4680
   gattgacaat ttactattta tacaagctaa gcttcaatac agactcgatc gaattcaggt 4740
   cgaccatctc ttaggatcga ctaacccgtg gccaactgct gttcacacgg aacctttccc 4800
   cacttcagtc ttcaaagttc tcatttgaat atttgctact accaccaaga tctgcactag 4860
   aggccgttcg acccagcatc actgcaaagg cttcgtcact gacctccacg cctgcctact 4920
   cgttagggca tcgcattaac ctaacggtag agtataggta acacgcttga gcgccatcca 4980
   ttttcagggc tagttcattc ggccggtgag ttgttacaca ctccttagcg gattccgact 5040
   tccatggcga gcttggcgta atcatggtca tagctgtttc ctgtgtgaaa ttgttatccg 5100
   ctcacaattc cacacaacat acgagccgga agcataaagt gtaaagcctg gggtgcctaa 5160
   tgagtgagct aactcacatt aattgcgttg cgctcactgc ccgctttcca gtcgggaaac 5220
   ctgtcgtgcc agctgcatta atgaatcggc caacgcgcgg ggagaggcgg tttgcgtatt 5280
   gggcgctctt ccgcttcctc gctcactgac tcgctgcgct cggtcgttcg gctgcggcga 5340
   gcggtatcag ctcactcaaa ggcggtaata cggttatcca cagaatcagg ggataacgca 5400
   ggaaagaaca tgtgagcaaa aggccagcaa aaggccagga accgtaaaaa ggccgcgttg 5460
   ctggcgtttt tccataggct ccgcccccct gacgagcatc acaaaaatcg acgctcaagt 5520
   cagaggtggc gaaacccgac aggactataa agataccagg cgtttccccc tggaagctcc 5580
   ctcgtgcgct ctcctgttcc gaccctgccg cttaccggat acctgtccgc ctttctccct 5640
   tcgggaagcg tggcgctttc tcatagctca cgctgtaggt atctcagttc ggtgtaggtc 5700
   gttcgctcca agctgggctg tgtgcacgaa ccccccgttc agcccgaccg ctgcgcctta 5760
   tccggtaact atcgtcttga gtccaacccg gtaagacacg acttatcgcc actggcagca 5820
   gccactggta acaggattag cagagcgagg tatgtaggcg gtgctacaga gttcttgaag 5880
   tggtggccta actacggcta cactagaagg acagtatttg gtatctgcgc tctgctgaag 5940
   ccagttacct tcggaaaaag agttggtagc tcttgatccg gcaaacaaac caccgctggt 6000
   agcggtggtt tttttgtttg caagcagcag attacgcgca gaaaaaaagg atctcaagaa 6060
   gatcctttga tcttttctac ggggtctgac gctcagtgga acgaaaactc acgttaaggg 6120
   attttggtca tgagattatc aaaaaggatc ttcacctaga tccttttaaa ttaaaaatga 6180
   agttttaaat caatctaaag tatatatgag taaacttggt ctgacagtta ccaatgctta 6240
   atcagtgagg cacctatctc agcgatctgt ctatttcgtt catccatagt tgcctgactc 6300
   cccgtcgtgt agataactac gatacgggag ggcttaccat ctggccccag tgctgcaatg 6360
   ataccgcgag acccacgctc accggctcca gatttatcag caataaacca gccagccgga 6420
   agggccgagc gcagaagtgg tcctgcaact ttatccgcct ccatccagtc tattaattgt 6480
   tgccgggaag ctagagtaag tagttcgcca gttaatagtt tgcgcaacgt tgttgccatt 6540
   gctacaggca tcgtggtgtc acgctcgtcg tttggtatgg cttcattcag ctccggttcc 6600
   caacgatcaa ggcgagttac atgatccccc atgttgtgca aaaaagcggt tagctccttc 6660
   ggtcctccga tcgttgtcag aagtaagttg gccgcagtgt tatcactcat ggttatggca 6720
   gcactgcata attctcttac tgtcatgcca tccgtaagat gcttttctgt gactggtgag 6780
   tactcaacca agtcattctg agaatagtgt atgcggcgac cgagttgctc ttgcccggcg 6840
   tcaatacggg ataataccgc gccacatagc agaactttaa aagtgctcat cattggaaaa 6900
   cgttcttcgg ggcgaaaact ctcaaggatc ttaccgctgt tgagatccag ttcgatgtaa 6960
   cccactcgtg cacccaactg atcttcagca tcttttactt tcaccagcgt ttctgggtga 7020
   gcaaaaacag gaaggcaaaa tgccgcaaaa aagggaataa gggcgacacg gaaatgttga 7080
   atactcatac tcttcctttt tcaatattat tgaagcattt atcagggtta ttgtctcatg 7140
   agcggataca tatttgaatg tatttagaaa aataaacaaa taggggttcc gcgcacattt 7200
   ccccgaaaag tgccacctga cgtctaagaa accattatta tcatgacatt aacctataaa 7260
   aataggcgta tcacgaggcc ctttcgtc 7288
<210> 16
   <211> 6777
   <212> DNA
   <213> Artificial
<220>
   <223> pGH72
<400> 16
   tcgcgcgttt cggtgatgac ggtgaaaacc tctgacacat gcagctcccg gagacggtca 60
   cagcttgtct gtaagcggat gccgggagca gacaagcccg tcagggcgcg tcagcgggtg 120
   ttggcgggtg tcggggctgg cttaactatg cggcatcaga gcagattgta ctgagagtgc 180
   accatatgcg gtgtgaaata ccgcacagat gcgtaaggag aaaataccgc atcaggcgac 240
   gcgccctgta gcggcgcatt aagcgcggcg ggtgtggtgg ttacgcgcag cgtgaccgct 300
   acacttgcca gcgccctagc gcccgctcct ttcgctttct tcccttcctt tctcgccacg 360
   ttcgccggct ttccccgtca agctctaaat cgggggctcc ctttagggtt ccgatttagt 420
   gctttacggc acctcgaccc caaaaaactt gattagggtg atggttcacg tagtgggcca 480
   tcgccctgat agacggtttt tcgccctttg acgttggagt ccacgttctt taatagtgga 540
   ctcttgttcc aaactggaac aacactcaac cctatctcgg tctattcttt tgatttataa 600
   gggattttgc cgatttcggc ctattggtta aaaaatgagc tgatttaaca aaaatttaac 660
   gcgaatttta acaaaatatt aacgtttaca atttcgccat tcgccattca ggctgcgcaa 720
   ctgttgggaa gggcgatcgg tgcgggcctc ttcgctatta cgccagctgg cgaaaggggg 780
   atgtgctgca aggcgattaa gttgggtaac gccagggttt tcccagtcac gacgttgtaa 840
   aacgacggcc agtgaattgt aatacgactc actatagcca tggccaccgt ccggctgtct 900
   agatgaacta acaccttttc tggtgtctga tgagcgtgca ttccggcacc ttaactctac 960
   gttcggttca tcccgcatcg ccagttctgc ttaccaaaaa tggcccacta aaagctcttc 1020
   attctaatgt ccacgttcaa ttaagtaaca aggacttctt acatattcaa agtttgagaa 1080
   taggtcaaga tcgtttcaat cccaatacct ctaatcattc gctttacctc ataaaactga 1140
   tacgagcttc tgctatcctg agggaaactt cggcaggaac cagctactag atggttcgag 1200
   tcgacgaagc agaattcctg cagattttaa tctttcggca aaatttagat aaaaaatttg 1260
   ggtattcgta tttagtttcc aatattattt agttatacaa aactattgtc tcaatagact 1320
   ggcgttgtaa tgagtagtgt tcccctcgac tctagggaat tagcggccgc ttatttctga 1380
   acataaatgg agacggaccc atcgtttaca tgaaactctc cccagccgtc agatccgatt 1440
   tttacagtat ctgaacggtt gcccgttatg tcataccatg tctcgccggc atttttcagg 1500
   ccggcataca tccgctttga tccgccgggt ccgtccgtga ttaaagcggc caaacctgat 1560
   ttggcggcgg agctgtcacc ttccctcgtc catccgatca catccgggtg gtcaatataa 1620
   tcgtgctggg gcccgtatgc gtactcctta cgcgctttta aaatcggctc tatattatct 1680
   ttcagtgagg gaatttcctt tggcgatgtc ccttttgtcc cgtacatatc cccatagaac 1740
   acctgaggat aaccggattc tcttgtcaaa ataaaggcgt atgcaagcgg tttaaaccaa 1800
   gtttggactg tcgattccaa tgactgtccc ggctgtgtgt catgattttc aacaaatgta 1860
   accgcctttt ccggatgcct ggacacaacg gtaccgtcca gcaaacgcct catatcatat 1920
   ccgcctcctt gtgaggaagc cgcctgtaaa ttgaaatgaa gcggaacatc aaacacggat 1980
   tgattaaagc ttgttttatt caagtagttt tcgagtttcc cggcattatt ctgccaatac 2040
   tccgcaaccg taaacatttc ttttcccgtc gcctgtctga ccgcctgaac ccaatcacgc 2100
   agaaatgaaa atttaatatg tttggcggca tcaatacgga agccgtctaa tgacagttca 2160
   ttcgcatacc agatacccca tttttttgtc tctgccacga catcagggtg gtcgtagtca 2220
   acatcagcat acattaaata gtcatagttg ccgttttcac ttgatacttc ccaatcccac 2280
   gcttttcctt ccccacgaaa cttaaagatg cggctgatct tccgggattc atcccagtcc 2340
   gctccgtcga aatgatacca atgccattta aaatcactgt acgtgtttcc acggcccgga 2400
   aaacgaaaat ccgtccacgc tttgatttga tattcctccg aagtttcctg atttctattg 2460
   gccggattga cttcgacggc agttacatct tctgttgcat cagcaccagc cttatgattc 2520
   aaaaccacat ctccgtatac ttggacgttc cgggaatgca gtgagccgat cgcatcttga 2580
   agctctgatt ttgtgccgta tttcgttctg accgtccctt tttgctggaa ttctcctaaa 2640
   tcatacaaat cataaggtcc gtatccgtta tcggattggc tcaatccttt gtatgcggga 2700
   ggaatccaga cggcagtgat tccgatatcc gataaatgtt ccgcatcatt ctgcaatcgt 2760
   ttccaatgct ggccgtcgtt cggcgtatac cattcaaaat actgcatcag cgtgccattt 2820
   acggctgatg tttttgtaat cggcaaactg acaaataaca gcgtgcacat aagcacaagt 2880
   ctgaacgaaa ctgtccgctt tcgtttttga atcatggatc cagagaattc tgttgattat 2940
   gtttttaaga actactcaga atgaaatgcc ttgatttccg gtgtgagttg acaaggaaaa 3000
   aaattccagg agcaggggga gctttatata acaccagatt agcggcgaaa aatttttttt 3060
   ctcgaggggt tacccggtcg tcctttgcct aatggggaaa catcccgctc gccgcacgta 3120
   ccacttaggg ctccccaaaa agcaaccatc ctccgctcat ctcgagggag atcactaaac 3180
   gatatcaact ttgagggctc ttgttgacta tggactgatt atagattgaa gtcgagacta 3240
   gtggatcccc cgggctgcag gcatgcaagc tggggccgcc gcggttaagg aacgacatgc 3300
   ccaggggact ctccactcac aaaccctttc aaaaacctgt acaatgacaa cgtckgccaa 3360
   tcgtcaaggt gtgcggaatc aggaccgtgg aggcggccca ggtcgctttg gatgctggag 3420
   ccaacatgat tggaatgatt atggtcccta atagagcgcg cactgttgat atggagacag 3480
   caggcaagat atcacgactg gtacgatcat atagacgtca aggatccttt gagctggaca 3540
   ctaatgagct gggcggatcg actcactctg cactcgccca catcattgaa gccaagacaa 3600
   ggacccggcc agcaatcgta ggcgtgttcc gcaaccagcc gctgtcagaa gtcctgcggt 3660
   tgcaacagga attgtcgctg gatttcgttc agcttcacgg atcagagccc ctagaatggt 3720
   gtagactgat tccatgtcct gtgatccgtc ggttcacccc cgatactcca gatttctatg 3780
   cctcgccggt tactggatac cacagcctat ccctcataga cggtgaggtt ggcggagagg 3840
   gcaagatggt ggactggagc ggagtccagt ctcgggcccg aatgggagcg aggttcctta 3900
   tggctggagg attgacagcc gacaatgttg cccaagctct agcggtcgag ggtgcctgtg 3960
   gtgttgatgt cagcggagga gtcgaatcaa gcccgggggt caaagatctc aacgagattg 4020
   agcggtttgt caaagctgcc aagggggtta ctgtgaagta ttaacactcc agcagcagca 4080
   tcaataggtg atactgacga caggtaatgt gtactaatag gtctgacagc agaatacagc 4140
   tatattactt ctcatttgaa acttgaggtg attgacaatt tactatttat acaagctaag 4200
   cttcaataca gactcgatcg aattcaggtc gaccatctct taggatcgac taacccgtgg 4260
   ccaactgctg ttcacacgga acctttcccc acttcagtct tcaaagttct catttgaata 4320
   tttgctacta ccaccaagat ctgcactaga ggccgttcga cccagcatca ctgcaaaggc 4380
   ttcgtcactg acctccacgc ctgcctactc gttagggcat cgcattaacc taacggtaga 4440
   gtataggtaa cacgcttgag cgccatccat tttcagggct agttcattcg gccggtgagt 4500
   tgttacacac tccttagcgg attccgactt ccatggcgag cttggcgtaa tcatggtcat 4560
   agctgtttcc tgtgtgaaat tgttatccgc tcacaattcc acacaacata cgagccggaa 4620
   gcataaagtg taaagcctgg ggtgcctaat gagtgagcta actcacatta attgcgttgc 4680
   gctcactgcc cgctttccag tcgggaaacc tgtcgtgcca gctgcattaa tgaatcggcc 4740
   aacgcgcggg gagaggcggt ttgcgtattg ggcgctcttc cgcttcctcg ctcactgact 4800
   cgctgcgctc ggtcgttcgg ctgcggcgag cggtatcagc tcactcaaag gcggtaatac 4860
   ggttatccac agaatcaggg gataacgcag gaaagaacat gtgagcaaaa ggccagcaaa 4920
   aggccaggaa ccgtaaaaag gccgcgttgc tggcgttttt ccataggctc cgcccccctg 4980
   acgagcatca caaaaatcga cgctcaagtc agaggtggcg aaacccgaca ggactataaa 5040
   gataccaggc gtttccccct ggaagctccc tcgtgcgctc tcctgttccg accctgccgc 5100
   ttaccggata cctgtccgcc tttctccctt cgggaagcgt ggcgctttct catagctcac 5160
   gctgtaggta tctcagttcg gtgtaggtcg ttcgctccaa gctgggctgt gtgcacgaac 5220
   cccccgttca gcccgaccgc tgcgccttat ccggtaacta tcgtcttgag tccaacccgg 5280
   taagacacga cttatcgcca ctggcagcag ccactggtaa caggattagc agagcgaggt 5340
   atgtaggcgg tgctacagag ttcttgaagt ggtggcctaa ctacggctac actagaagga 5400
   cagtatttgg tatctgcgct ctgctgaagc cagttacctt cggaaaaaga gttggtagct 5460
   cttgatccgg caaacaaacc accgctggta gcggtggttt ttttgtttgc aagcagcaga 5520
   ttacgcgcag aaaaaaagga tctcaagaag atcctttgat cttttctacg gggtctgacg 5580
   ctcagtggaa cgaaaactca cgttaaggga ttttggtcat gagattatca aaaaggatct 5640
   tcacctagat ccttttaaat taaaaatgaa gttttaaatc aatctaaagt atatatgagt 5700
   aaacttggtc tgacagttac caatgcttaa tcagtgaggc acctatctca gcgatctgtc 5760
   tatttcgttc atccatagtt gcctgactcc ccgtcgtgta gataactacg atacgggagg 5820
   gcttaccatc tggccccagt gctgcaatga taccgcgaga cccacgctca ccggctccag 5880
   atttatcagc aataaaccag ccagccggaa gggccgagcg cagaagtggt cctgcaactt 5940
   tatccgcctc catccagtct attaattgtt gccgggaagc tagagtaagt agttcgccag 6000
   ttaatagttt gcgcaacgtt gttgccattg ctacaggcat cgtggtgtca cgctcgtcgt 6060
   ttggtatggc ttcattcagc tccggttccc aacgatcaag gcgagttaca tgatccccca 6120
   tgttgtgcaa aaaagcggtt agctccttcg gtcctccgat cgttgtcaga agtaagttgg   6180
   ccgcagtgtt atcactcatg gttatggcag cactgcataa ttctcttact gtcatgccat   6240
   ccgtaagatg cttttctgtg actggtgagt actcaaccaa gtcattctga gaatagtgta   6300
   tgcggcgacc gagttgctct tgcccggcgt caatacggga taataccgcg ccacatagca   6360
   gaactttaaa agtgctcatc attggaaaac gttcttcggg gcgaaaactc tcaaggatct   6420
   taccgctgtt gagatccagt tcgatgtaac ccactcgtgc acccaactga tcttcagcat   6480
   cttttacttt caccagcgtt tctgggtgag caaaaacagg aaggcaaaat gccgcaaaaa   6540
   agggaataag ggcgacacgg aaatgttgaa tactcatact cttccttttt caatattatt   6600
   gaagcattta tcagggttat tgtctcatga gcggatacat atttgaatgt atttagaaaa   6660
   ataaacaaat aggggttccg cgcacatttc cccgaaaagt gccacctgac gtctaagaaa   6720
   ccattattat catgacatta acctataaaa ataggcgtat cacgaggccc tttcgtc      6777
<210> 17
   <211> 6579
   <212> DNA
   <213> Artificial
<220>
   <223> pGH127
<400> 17
   tcgcgcgttt cggtgatgac ggtgaaaacc tctgacacat gcagctcccg gagacggtca      60
   cagcttgtct gtaagcggat gccgggagca gacaagcccg tcagggcgcg tcagcgggtg     120
   ttggcgggtg tcggggctgg cttaactatg cggcatcaga gcagattgta ctgagagtgc     180
   accatatgcg gtgtgaaata ccgcacagat gcgtaaggag aaaataccgc atcaggcgac     240
   gcgccctgta gcggcgcatt aagcgcggcg ggtgtggtgg ttacgcgcag cgtgaccgct     300
   acacttgcca gcgccctagc gcccgctcct ttcgctttct tcccttcctt tctcgccacg     360
   ttcgccggct ttccccgtca agctctaaat cgggggctcc ctttagggtt ccgatttagt     420
   gctttacggc acctcgaccc caaaaaactt gattagggtg atggttcacg tagtgggcca     480
   tcgccctgat agacggtttt tcgccctttg acgttggagt ccacgttctt taatagtgga     540
   ctcttgttcc aaactggaac aacactcaac cctatctcgg tctattcttt tgatttataa     600
   gggattttgc cgatttcggc ctattggtta aaaaatgagc tgatttaaca aaaatttaac     660
   gcgaatttta acaaaatatt aacgtttaca atttcgccat tcgccattca ggctgcgcaa     720
   ctgttgggaa gggcgatcgg tgcgggcctc ttcgctatta cgccagctgg cgaaaggggg     780
   atgtgctgca aggcgattaa gttgggtaac gccagggttt tcccagtcac gacgttgtaa     840
   aacgacggcc agtgaattgt aatacgactc actatagcca tggccaccgt ccggctgtct     900
   agatgaacta acaccttttc tggtgtctga tgagcgtgca ttccggcacc ttaactctac     960
   gttcggttca tcccgcatcg ccagttctgc ttaccaaaaa tggcccacta aaagctcttc 1020
   attctaatgt ccacgttcaa ttaagtaaca aggacttctt acatattcaa agtttgagaa 1080
   taggtcaaga tcgtttcaat cccaatacct ctaatcattc gctttacctc ataaaactga 1140
   tacgagcttc tgctatcctg agggaaactt cggcaggaac cagctactag atggttcgag 1200
   tcgacgaagc agaattcctg cagattttaa tctttcggca aaatttagat aaaaaatttg 1260
   ggtattcgta tttagtttcc aatattattt agttatacaa aactattgtc tcaatagact 1320
   ggcgttgtaa tgagtagtgt tcccctcgac tctagggaat tagcggccgc ttaactgatg 1380
   gcagccggtc caacaggggc tccgagcaaa cccttgagaa ttccccaaat ttcggcagag 1440
   taggtaggca atgcatcttc atcgaaggcg ttagaaaaca catcgtgtct ctggcatccc 1500
   gagctcacgg agccaccatc gagcaggttc ttgacataat tgtaagcttt agggaatcca 1560
   gtgatcattt caagaacatg ctctgatccc tcctctctgt agtattccac ggtaacgcca 1620
   ttggaacagt actcgtaata aagcttgtcc acatcagcaa ttggcataac ctcatcatgg 1680
   acaccgtggt aaaacagcaa tggaatctga ggtttctgct ttcccatggc attctcatcc 1740
   aggacttttt gaatggtctc attgtacaaa actcggtccc cagcctttgt gtatgaaaac 1800
   caatcctgga acgcataagt cagaacgtct actgaaagac actgctttcc ggccttttca 1860
   aagtcatctc tcttgtcggg aagaagttga tcgttaatgt aatcttctag ctctggatat 1920
   tgggatgata atcccttgat tccagccggt accagaccaa caaacgggcc cttattcacc 1980
   tgaacggcca cactagtaat gttctggatt actcctccga gagctgctcc tgcaattttg 2040
   agctctggcg catacgtggg ttggagagct gctgcccaac ctgatgcgat tgatccccca 2100
   gagtatcccc acatggctac ctttgaatca ggcttgaccc cggtaaacga ctccgacttg 2160
   agcactgctc taatcgagtc cagagtggcc tgtccggaga tgatggctga cgtgaacgaa 2220
   gactttggac cctcataatc gggagttgaa acaatccaac cctcattgag agcggattga 2280
   agagtaagca tatccacctg agaaatgatc ccttgagggt tagcaccaaa ctgaattgca 2340
   tacgacggag cacagttgat ccaagcagag tcttcccagg actggtacga cagcagcttt 2400
   ccttcggcat ggttgtgagg gatcatgaca gtcaccacag tggccgtggg ctgtccctga 2460
   gtatcagtag tacggtacag gatctgaatg gacgcattaa gattctgctt aaaaacagta 2520
   aaagcaggca tctgaggagc gtgtctccat cgcagaattg taccaggctc ttccttctca 2580
   taaccatcat ctggcgtgta gaacggatct tcagagggtg gggtcacagc atcgcgagca 2640
   tatacacgct cttcaggcag cgctgcacca aatacaaaag ccaccagttg ggccacaagt 2700
   agcaggagct tcagcatgga tccagagaat tctgttgatt atgtttttaa gaactactca 2760
   gaatgaaatg ccttgatttc cggtgtgagt tgacaaggaa aaaaattcca ggagcagggg 2820
   gagctttata taacaccaga ttagcggcga aaaatttttt ttctcgaggg gttacccggt 2880
   cgtcctttgc ctaatgggga aacatcccgc tcgccgcacg taccacttag ggctccccaa 2940
   aaagcaacca tcctccgctc atctcgaggg agatcactaa acgatatcaa ctttgagggc 3000
   tcttgttgac tatggactga ttatagattg aagtcgagac tagtggatcc cccgggctgc   3060
   aggcatgcaa gctggggccg ccgcggttaa ggaacgacat gcccagggga ctctccactc   3120
   acaaaccctt tcaaaaacct gtacaatgac aacgtckgcc aatcgtcaag gtgtgcggaa 3180
   tcaggaccgt ggaggcggcc caggtcgctt tggatgctgg agccaacatg attggaatga 3240
   ttatggtccc taatagagcg cgcactgttg atatggagac agcaggcaag atatcacgac 3300
   tggtacgatc atatagacgt caaggatcct ttgagctgga cactaatgag ctgggcggat 3360
   cgactcactc tgcactcgcc cacatcattg aagccaagac aaggacccgg ccagcaatcg 3420
   taggcgtgtt ccgcaaccag ccgctgtcag aagtcctgcg gttgcaacag gaattgtcgc 3480
   tggatttcgt tcagcttcac ggatcagagc ccctagaatg gtgtagactg attccatgtc 3540
   ctgtgatccg tcggttcacc cccgatactc cagatttcta tgcctcgccg gttactggat 3600
   accacagcct atccctcata gacggtgagg ttggcggaga gggcaagatg gtggactgga 3660
   gcggagtcca gtctcgggcc cgaatgggag cgaggttcct tatggctgga ggattgacag 3720
   ccgacaatgt tgcccaagct ctagcggtcg agggtgcctg tggtgttgat gtcagcggag 3780
   gagtcgaatc aagcccgggg gtcaaagatc tcaacgagat tgagcggttt gtcaaagctg 3840
   ccaagggggt tactgtgaag tattaacact ccagcagcag catcaatagg tgatactgac 3900
   gacaggtaat gtgtactaat aggtctgaca gcagaataca gctatattac ttctcatttg 3960
   aaacttgagg tgattgacaa tttactattt atacaagcta agcttcaata cagactcgat 4020
   cgaattcagg tcgaccatct cttaggatcg actaacccgt ggccaactgc tgttcacacg 4080
   gaacctttcc ccacttcagt cttcaaagtt ctcatttgaa tatttgctac taccaccaag 4140
   atctgcacta gaggccgttc gacccagcat cactgcaaag gcttcgtcac tgacctccac 4200
   gcctgcctac tcgttagggc atcgcattaa cctaacggta gagtataggt aacacgcttg 4260
   agcgccatcc attttcaggg ctagttcatt cggccggtga gttgttacac actccttagc 4320
   ggattccgac ttccatggcg agcttggcgt aatcatggtc atagctgttt cctgtgtgaa 4380
   attgttatcc gctcacaatt ccacacaaca tacgagccgg aagcataaag tgtaaagcct 4440
   ggggtgccta atgagtgagc taactcacat taattgcgtt gcgctcactg cccgctttcc 4500
   agtcgggaaa cctgtcgtgc cagctgcatt aatgaatcgg ccaacgcgcg gggagaggcg 4560
   gtttgcgtat tgggcgctct tccgcttcct cgctcactga ctcgctgcgc tcggtcgttc 4620
   ggctgcggcg agcggtatca gctcactcaa aggcggtaat acggttatcc acagaatcag 4680
   gggataacgc aggaaagaac atgtgagcaa aaggccagca aaaggccagg aaccgtaaaa 4740
   aggccgcgtt gctggcgttt ttccataggc tccgcccccc tgacgagcat cacaaaaatc 4800
   gacgctcaag tcagaggtgg cgaaacccga caggactata aagataccag gcgtttcccc 4860
   ctggaagctc cctcgtgcgc tctcctgttc cgaccctgcc gcttaccgga tacctgtccg 4920
   cctttctccc ttcgggaagc gtggcgcttt ctcatagctc acgctgtagg tatctcagtt 4980
   cggtgtaggt cgttcgctcc aagctgggct gtgtgcacga accccccgtt cagcccgacc 5040
   gctgcgcctt atccggtaac tatcgtcttg agtccaaccc ggtaagacac gacttatcgc 5100
   cactggcagc agccactggt aacaggatta gcagagcgag gtatgtaggc ggtgctacag 5160
   agttcttgaa gtggtggcct aactacggct acactagaag gacagtattt ggtatctgcg 5220
   ctctgctgaa gccagttacc ttcggaaaaa gagttggtag ctcttgatcc ggcaaacaaa 5280
   ccaccgctgg tagcggtggt ttttttgttt gcaagcagca gattacgcgc agaaaaaaag 5340
   gatctcaaga agatcctttg atcttttcta cggggtctga cgctcagtgg aacgaaaact 5400
   cacgttaagg gattttggtc atgagattat caaaaaggat cttcacctag atccttttaa 5460
   attaaaaatg aagttttaaa tcaatctaaa gtatatatga gtaaacttgg tctgacagtt 5520
   accaatgctt aatcagtgag gcacctatct cagcgatctg tctatttcgt tcatccatag 5580
   ttgcctgact ccccgtcgtg tagataacta cgatacggga gggcttacca tctggcccca 5640
   gtgctgcaat gataccgcga gacccacgct caccggctcc agatttatca gcaataaacc 5700
   agccagccgg aagggccgag cgcagaagtg gtcctgcaac tttatccgcc tccatccagt 5760
   ctattaattg ttgccgggaa gctagagtaa gtagttcgcc agttaatagt ttgcgcaacg 5820
   ttgttgccat tgctacaggc atcgtggtgt cacgctcgtc gtttggtatg gcttcattca 5880
   gctccggttc ccaacgatca aggcgagtta catgatcccc catgttgtgc aaaaaagcgg 5940
   ttagctcctt cggtcctccg atcgttgtca gaagtaagtt ggccgcagtg ttatcactca 6000
   tggttatggc agcactgcat aattctctta ctgtcatgcc atccgtaaga tgcttttctg 6060
   tgactggtga gtactcaacc aagtcattct gagaatagtg tatgcggcga ccgagttgct 6120
   cttgcccggc gtcaatacgg gataataccg cgccacatag cagaacttta aaagtgctca 6180
   tcattggaaa acgttcttcg gggcgaaaac tctcaaggat cttaccgctg ttgagatcca 6240
   gttcgatgta acccactcgt gcacccaact gatcttcagc atcttttact ttcaccagcg 6300
   tttctgggtg agcaaaaaca ggaaggcaaa atgccgcaaa aaagggaata agggcgacac 6360
   ggaaatgttg aatactcata ctcttccttt ttcaatatta ttgaagcatt tatcagggtt 6420
   attgtctcat gagcggatac atatttgaat gtatttagaa aaataaacaa ataggggttc 6480
   cgcgcacatt tccccgaaaa gtgccacctg acgtctaaga aaccattatt atcatgacat 6540
   taacctataa aaataggcgt atcacgaggc cctttcgtc 6579
<210> 18
   <211> 8562
   <212> DNA
   <213> Artificial
<220>
   <223> pGH128
<400> 18
   tcgcgcgttt cggtgatgac ggtgaaaacc tctgacacat gcagctcccg gagacggtca      60
   cagcttgtct gtaagcggat gccgggagca gacaagcccg tcagggcgcg tcagcgggtg     120
   ttggcgggtg tcggggctgg cttaactatg cggcatcaga gcagattgta ctgagagtgc     180
   accatatgcg gtgtgaaata ccgcacagat gcgtaaggag aaaataccgc atcaggcgac     240
   gcgccctgta gcggcgcatt aagcgcggcg ggtgtggtgg ttacgcgcag cgtgaccgct     300
   acacttgcca gcgccctagc gcccgctcct ttcgctttct tcccttcctt tctcgccacg     360
   ttcgccggct ttccccgtca agctctaaat cgggggctcc ctttagggtt ccgatttagt     420
   gctttacggc acctcgaccc caaaaaactt gattagggtg atggttcacg tagtgggcca     480
   tcgccctgat agacggtttt tcgccctttg acgttggagt ccacgttctt taatagtgga     540
   ctcttgttcc aaactggaac aacactcaac cctatctcgg tctattcttt tgatttataa     600
   gggattttgc cgatttcggc ctattggtta aaaaatgagc tgatttaaca aaaatttaac     660
   gcgaatttta acaaaatatt aacgtttaca atttcgccat tcgccattca ggctgcgcaa     720
   ctgttgggaa gggcgatcgg tgcgggcctc ttcgctatta cgccagctgg cgaaaggggg     780
   atgtgctgca aggcgattaa gttgggtaac gccagggttt tcccagtcac gacgttgtaa     840
   aacgacggcc agtgaattgt aatacgactc actatagcca tggccaccgt ccggctgtct     900
   agatgaacta acaccttttc tggtgtctga tgagcgtgca ttccggcacc ttaactctac     960
   gttcggttca tcccgcatcg ccagttctgc ttaccaaaaa tggcccacta aaagctcttc    1020
   attctaatgt ccacgttcaa ttaagtaaca aggacttctt acatattcaa agtttgagaa    1080
   taggtcaaga tcgtttcaat cccaatacct ctaatcattc gctttacctc ataaaactga    1140
   tacgagcttc tgctatcctg agggaaactt cggcaggaac cagctactag atggttcgag    1200
   tcgacgaagc agaattcctg cagattttaa tctttcggca aaatttagat aaaaaatttg    1260
   ggtattcgta tttagtttcc aatattattt agttatacaa aactattgtc tcaatagact    1320
   ggcgttgtaa tgagtagtgt tcccctcgac tctagggaat tagcggccgc ttatttctga    1380
   acataaatgg agacggaccc atcgtttaca tgaaactctc cccagccgtc agatccgatt    1440
   tttacagtat ctgaacggtt gcccgttatg tcataccatg tctcgccggc atttttcagg    1500
   ccggcataca tccgctttga tccgccgggt ccgtccgtga ttaaagcggc caaacctgat    1560
   ttggcggcgg agctgtcacc ttccctcgtc catccgatca catccgggtg gtcaatataa    1620
   tcgtgctggg gcccgtatgc gtactcctta cgcgctttta aaatcggctc tatattatct 1680
   ttcagtgagg gaatttcctt tggcgatgtc ccttttgtcc cgtacatatc cccatagaac 1740
   acctgaggat aaccggattc tcttgtcaaa ataaaggcgt atgcaagcgg tttaaaccaa 1800
   gtttggactg tcgattccaa tgactgtccc ggctgtgtgt catgattttc aacaaatgta 1860
   accgcctttt ccggatgcct ggacacaacg gtaccgtcca gcaaacgcct catatcatat 1920
   ccgcctcctt gtgaggaagc cgcctgtaaa ttgaaatgaa gcggaacatc aaacacggat 1980
   tgattaaagc ttgttttatt caagtagttt tcgagtttcc cggcattatt ctgccaatac 2040
   tccgcaaccg taaacatttc ttttcccgtc gcctgtctga ccgcctgaac ccaatcacgc 2100
   agaaatgaaa atttaatatg tttggcggca tcaatacgga agccgtctaa tgacagttca 2160
   ttcgcatacc agatacccca tttttttgtc tctgccacga catcagggtg gtcgtagtca 2220
   acatcagcat acattaaata gtcatagttg ccgttttcac ttgatacttc ccaatcccac 2280
   gcttttcctt ccccacgaaa cttaaagatg cggctgatct tccgggattc atcccagtcc 2340
   gctccgtcga aatgatacca atgccattta aaatcactgt acgtgtttcc acggcccgga 2400
   aaacgaaaat ccgtccacgc tttgatttga tattcctccg aagtttcctg atttctattg 2460
   gccggattga cttcgacggc agttacatct tctgttgcat cagcaccagc cttatgattc 2520
   aaaaccacat ctccgtatac ttggacgttc cgggaatgca gtgagccgat cgcatcttga 2580
   agctctgatt ttgtgccgta tttcgttctg accgtccctt tttgctggaa ttctcctaaa 2640
   tcatacaaat cataaggtcc gtatccgtta tcggattggc tcaatccttt gtatgcggga 2700
   ggaatccaga cggcagtgat tccgatatcc gataaatgtt ccgcatcatt ctgcaatcgt 2760
   ttccaatgct ggccgtcgtt cggcgtatac cattcaaaat actgcatcag cgtgccattt 2820
   acggctgatg tttttgtaat cggcaaactg acaaataaca gcgtgcacat aagcacaagt 2880
   ctgaacgaaa ctgtccgctt tcgtttttga atcatggatc cagagaattc tgttgattat 2940
   gtttttaaga actactcaga atgaaatgcc ttgatttccg gtgtgagttg acaaggaaaa 3000
   aaattccagg agcaggggga gctttatata acaccagatt agcggcgaaa aatttttttt 3060
   ctcgaggggt tacccggtcg tcctttgcct aatggggaaa catcccgctc gccgcacgta 3120
   ccacttaggg ctccccaaaa agcaaccatc ctccgctcat ctcgagggag atcactaaac 3180
   gatatcaact ttgagggctc ttgttgacta tggactgatt atagattgaa gtcgagacta 3240
   gtctgcagat tttaatcttt cggcaaaatt tagataaaaa atttgggtat tcgtatttag 3300
   tttccaatat tatttagtta tacaaaacta ttgtctcaat agactggcgt tgtaatgagt 3360
   agtgttcccc tcgactctag ggaattagcg gccgcttaac tgatggcagc cggtccaaca 3420
   ggggctccga gcaaaccctt gagaattccc caaatttcgg cagagtaggt aggcaatgca 3480
   tcttcatcga aggcgttaga aaacacatcg tgtctctggc atcccgagct cacggagcca 3540
   ccatcgagca ggttcttgac ataattgtaa gctttaggga atccagtgat catttcaaga 3600
   acatgctctg atccctcctc tctgtagtat tccacggtaa cgccattgga acagtactcg 3660
   taataaagct tgtccacatc agcaattggc ataacctcat catggacacc gtggtaaaac 3720
   agcaatggaa tctgaggttt ctgctttccc atggcattct catccaggac tttttgaatg 3780
   gtctcattgt acaaaactcg gtccccagcc tttgtgtatg aaaaccaatc ctggaacgca 3840
   taagtcagaa cgtctactga aagacactgc tttccggcct tttcaaagtc atctctcttg 3900
   tcgggaagaa gttgatcgtt aatgtaatct tctagctctg gatattggga tgataatccc 3960
   ttgattccag ccggtaccag accaacaaac gggcccttat tcacctgaac ggccacacta 4020
   gtaatgttct ggattactcc tccgagagct gctcctgcaa ttttgagctc tggcgcatac 4080
   gtgggttgga gagctgctgc ccaacctgat gcgattgatc ccccagagta tccccacatg 4140
   gctacctttg aatcaggctt gaccccggta aacgactccg acttgagcac tgctctaatc 4200
   gagtccagag tggcctgtcc ggagatgatg gctgacgtga acgaagactt tggaccctca 4260
   taatcgggag ttgaaacaat ccaaccctca ttgagagcgg attgaagagt aagcatatcc 4320
   acctgagaaa tgatcccttg agggttagca ccaaactgaa ttgcatacga cggagcacag 4380
   ttgatccaag cagagtcttc ccaggactgg tacgacagca gctttccttc ggcatggttg 4440
   tgagggatca tgacagtcac cacagtggcc gtgggctgtc cctgagtatc agtagtacgg 4500
   tacaggatct gaatggacgc attaagattc tgcttaaaaa cagtaaaagc aggcatctga 4560
   ggagcgtgtc tccatcgcag aattgtacca ggctcttcct tctcataacc atcatctggc 4620
   gtgtagaacg gatcttcaga gggtggggtc acagcatcgc gagcatatac acgctcttca 4680
   ggcagcgctg caccaaatac aaaagccacc agttgggcca caagtagcag gagcttcagc 4740
   atggatccag agaattctgt tgattatgtt tttaagaact actcagaatg aaatgccttg 4800
   atttccggtg tgagttgaca aggaaaaaaa ttccaggagc agggggagct ttatataaca 4860
   ccagattagc ggcgaaaaat tttttttctc gaggggttac ccggtcgtcc tttgcctaat 4920
   ggggaaacat cccgctcgcc gcacgtacca cttagggctc cccaaaaagc aaccatcctc 4980
   cgctcatctc gagggagatc actaaacgat atcaactttg agggctcttg ttgactatgg 5040
   actgattata gattgaagtc gagccgcggt taaggaacga catgcccagg ggactctcca 5100
   ctcacaaacc ctttcaaaaa cctgtacaat gacaacgtck gccaatcgtc aaggtgtgcg 5160
   gaatcaggac cgtggaggcg gcccaggtcg ctttggatgc tggagccaac atgattggaa 5220
   tgattatggt ccctaataga gcgcgcactg ttgatatgga gacagcaggc aagatatcac 5280
   gactggtacg atcatataga cgtcaaggat cctttgagct ggacactaat gagctgggcg 5340
   gatcgactca ctctgcactc gcccacatca ttgaagccaa gacaaggacc cggccagcaa 5400
   tcgtaggcgt gttccgcaac cagccgctgt cagaagtcct gcggttgcaa caggaattgt 5460
   cgctggattt cgttcagctt cacggatcag agcccctaga atggtgtaga ctgattccat 5520
   gtcctgtgat ccgtcggttc acccccgata ctccagattt ctatgcctcg ccggttactg 5580
   gataccacag cctatccctc atagacggtg aggttggcgg agagggcaag atggtggact 5640
   ggagcggagt ccagtctcgg gcccgaatgg gagcgaggtt ccttatggct ggaggattga 5700
   cagccgacaa tgttgcccaa gctctagcgg tcgagggtgc ctgtggtgtt gatgtcagcg 5760
   gaggagtcga atcaagcccg ggggtcaaag atctcaacga gattgagcgg tttgtcaaag 5820
   ctgccaaggg ggttactgtg aagtattaac actccagcag cagcatcaat aggtgatact 5880
   gacgacaggt aatgtgtact aataggtctg acagcagaat acagctatat tacttctcat 5940
   ttgaaacttg aggtgattga caatttacta tttatacaag ctaagcttca atacagactc 6000
   gatcgaattc aggtcgacca tctcttagga tcgactaacc cgtggccaac tgctgttcac 6060
   acggaacctt tccccacttc agtcttcaaa gttctcattt gaatatttgc tactaccacc 6120
   aagatctgca ctagaggccg ttcgacccag catcactgca aaggcttcgt cactgacctc 6180
   cacgcctgcc tactcgttag ggcatcgcat taacctaacg gtagagtata ggtaacacgc 6240
   ttgagcgcca tccattttca gggctagttc attcggccgg tgagttgtta cacactcctt 6300
   agcggattcc gacttccatg gcgagcttgg cgtaatcatg gtcatagctg tttcctgtgt 6360
   gaaattgtta tccgctcaca attccacaca acatacgagc cggaagcata aagtgtaaag 6420
   cctggggtgc ctaatgagtg agctaactca cattaattgc gttgcgctca ctgcccgctt 6480
   tccagtcggg aaacctgtcg tgccagctgc attaatgaat cggccaacgc gcggggagag 6540
   gcggtttgcg tattgggcgc tcttccgctt cctcgctcac tgactcgctg cgctcggtcg 6600
   ttcggctgcg gcgagcggta tcagctcact caaaggcggt aatacggtta tccacagaat 6660
   caggggataa cgcaggaaag aacatgtgag caaaaggcca gcaaaaggcc aggaaccgta 6720
   aaaaggccgc gttgctggcg tttttccata ggctccgccc ccctgacgag catcacaaaa 6780
   atcgacgctc aagtcagagg tggcgaaacc cgacaggact ataaagatac caggcgtttc 6840
   cccctggaag ctccctcgtg cgctctcctg ttccgaccct gccgcttacc ggatacctgt 6900
   ccgcctttct cccttcggga agcgtggcgc tttctcatag ctcacgctgt aggtatctca 6960
   gttcggtgta ggtcgttcgc tccaagctgg gctgtgtgca cgaacccccc gttcagcccg 7020
   accgctgcgc cttatccggt aactatcgtc ttgagtccaa cccggtaaga cacgacttat 7080
   cgccactggc agcagccact ggtaacagga ttagcagagc gaggtatgta ggcggtgcta 7140
   cagagttctt gaagtggtgg cctaactacg gctacactag aaggacagta tttggtatct 7200
   gcgctctgct gaagccagtt accttcggaa aaagagttgg tagctcttga tccggcaaac 7260
   aaaccaccgc tggtagcggt ggtttttttg tttgcaagca gcagattacg cgcagaaaaa 7320
   aaggatctca agaagatcct ttgatctttt ctacggggtc tgacgctcag tggaacgaaa 7380
   actcacgtta agggattttg gtcatgagat tatcaaaaag gatcttcacc tagatccttt 7440
   taaattaaaa atgaagtttt aaatcaatct aaagtatata tgagtaaact tggtctgaca 7500
   gttaccaatg cttaatcagt gaggcaccta tctcagcgat ctgtctattt cgttcatcca 7560
   tagttgcctg actccccgtc gtgtagataa ctacgatacg ggagggctta ccatctggcc 7620
   ccagtgctgc aatgataccg cgagacccac gctcaccggc tccagattta tcagcaataa 7680
   accagccagc cggaagggcc gagcgcagaa gtggtcctgc aactttatcc gcctccatcc 7740
   agtctattaa ttgttgccgg gaagctagag taagtagttc gccagttaat agtttgcgca 7800
   acgttgttgc cattgctaca ggcatcgtgg tgtcacgctc gtcgtttggt atggcttcat 7860
   tcagctccgg ttcccaacga tcaaggcgag ttacatgatc ccccatgttg tgcaaaaaag 7920
   cggttagctc cttcggtcct ccgatcgttg tcagaagtaa gttggccgca gtgttatcac 7980
   tcatggttat ggcagcactg cataattctc ttactgtcat gccatccgta agatgctttt 8040
   ctgtgactgg tgagtactca accaagtcat tctgagaata gtgtatgcgg cgaccgagtt 8100
   gctcttgccc ggcgtcaata cgggataata ccgcgccaca tagcagaact ttaaaagtgc 8160
   tcatcattgg aaaacgttct tcggggcgaa aactctcaag gatcttaccg ctgttgagat 8220
   ccagttcgat gtaacccact cgtgcaccca actgatcttc agcatctttt actttcacca 8280
   gcgtttctgg gtgagcaaaa acaggaaggc aaaatgccgc aaaaaaggga ataagggcga 8340
   cacggaaatg ttgaatactc atactcttcc tttttcaata ttattgaagc atttatcagg 8400
   gttattgtct catgagcgga tacatatttg aatgtattta gaaaaataaa caaatagggg 8460
   ttccgcgcac atttccccga aaagtgccac ctgacgtcta agaaaccatt attatcatga 8520
   cattaaccta taaaaatagg cgtatcacga ggccctttcg tc 8562
<210> 19
   <211> 27
   <212> DNA
   <213> Artificial
<220>
   <223> Primer GS76
<400> 19
   cgatccatgg ctatagtgag tcgtatt 27
<210> 20
   <211> 28
   <212> DNA
   <213> Artificial
<220>
<223> Primer GS77
<400> 20
   cgatccatgg cgagcttggc gtaatcat 28
<210> 21
   <211> 29
   <212> DNA
   <213> Artificial
<220>
   <223> Primer GS78
<400> 21
   cgactgtaca ctcaactaga gtgtcggta 29
<210> 22
   <211> 29
   <212> DNA
   <213> Artificial
<220>
   <223> Primer GS79
<400> 22
   gtgttgtaca atgacaacgt cgccaatcg 29
<210> 23
   <211> 29
   <212> DNA
   <213> Artificial
<220>
   <223> Primer GS94
<400> 23
   cacctgtaca ggtttttgaa agggtttgt 29
<210> 24
   <211> 30
   <212> DNA
   <213> Artificial
<220>
   <223> Primer GS95
<400> 24
   acagccgcgg ttttcaatcg acgaattgca 30
<210> 25
   <211> 29
   <212> DNA
   <213> Artificial
<220>

   <223> Primer GS88
<400> 25
gatcgtcgac tcgaaccatc tagtagctg 29
<210> 26
   <211> 29
   <212> DNA
   <213> Artificial
<220>
   <223> Primer GS91
<400> 26
   agaggtcgac catctcttag gatcgacta 29
<210> 27
   <211> 30
   <212> DNA
   <213> Artificial
<220>
   <223> Primer GS89
<400> 27
   acgtgtcgac actgaattcg atcgagtctg 30
<210> 28
   <211> 30
   <212> DNA
   <213> Artificial
<220>
   <223> Primer GS90
<400> 28
   tctggtcgac gaagcagaat tcctgcagat 30
<210> 29
   <211> 28
   <212> DNA
   <213> Artificial
<220>
   <223> Primer GS96
<400> 29
   tataccgcgg cggccccagc ttgcatgc 28
<210> 30
   <211> 28
   <212> DNA
   <213> Artificial
<220>
   <223> Primer GS97
<400> 30
   ggaaccgcgg tcaccacatg ctgtcctt 28
<210> 31
   <211> 29
   <212> DNA
   <213> Artificial
<220>
   <223> Primer GS98
<400> 31
   tattccgcgg ttaaggaacg acatgccca 29
<210> 32
   <211> 28
   <212> DNA
   <213> Artificial
<220>
   <223> Primer GS99
<400> 32
   atatccgcgg tgcccagggg actctcca 28
<210> 33
   <211> 28
   <212> DNA
   <213> Artificial
<220>
   <223> Primer GS100
<400> 33
   gtgtccgcgg tctccactca caaaccct 28
<210> 34
   <211> 29
   <212> DNA
   <213> Artificial
<220>
   <223> Primer GS101
<400> 34
   atatccgcgg ccgctcggaa ggatacgca 29
<210> 35
   <211> 28
   <212> DNA
   <213> Artificial
<220>
   <223> Primer GS102
<400> 35
   atcaccgcgg cagaatgctt tatcgtgg 28
<210> 36
   <211> 28
   <212> DNA
<213> Artificial
<220>
   <223> Primer GS103
<400> 36
   tataccgcgg gctgccccgg gccgaaac 28
<210> 37
   <211> 28
   <212> DNA
   <213> Artificial
<220>
   <223> Primer GS104
<400> 37
   aacaccgcgg accggcattt tgggttga 28
<210> 38
   <211> 28
   <212> DNA
   <213> Artificial
<220>
   <223> Primer GS105
<400> 38
   aagaccgcgg catgctgtcc tttcctcc 28
<210> 39
   <211> 28
   <212> DNA
   <213> Artificial
<220>
   <223> Primer GS106
<400> 39
   tcttccgcgg aaggatacgc agaatgct 28
<210> 40
   <211> 29
   <212> DNA
   <213> Artificial
<220>
   <223> Primer GS107
<400> 40
   ataaccgcgg ttatcgtggg gctgccccg 29
<210> 41
   <211> 27
   <212> DNA
   <213> Artificial
<220>
<223> Primer GS108
<400> 41
   ttaaccgcgg ggccgaaacc ggcattt 27
<210> 42
   <211> 28
   <212> DNA
   <213> Artificial
<220>
   <223> Primer GS109
<400> 42
   acacccgcgg ttgggttgac tcttttaa 28